# EUROPEAN PATENT APPLICATION

(11) **EP 3 659 996 A1**
(43) Date of publication of application: **03.06.2020**
(21) Application number: 18839253.4
(22) Date of filing: 27.07.2018
(51) Int. Cl.: C07C 211/50, C07C 215/76, C07C 217/84, C07D 233/61, C08G 8/16, C08G 85/00

(54) **NOVEL (POLY)AMINE COMPOUND, RESIN, AND CURED PRODUCT**

(30) Priority: 28.07.2017 JP 2017147097
(71) Applicant: Mitsubishi Gas Chemical Company, Inc., Tokyo 100-8324 (JP)
(72) Inventor: ECHIGO, Masatoshi, Tokyo 100-8324 (JP); MAKINOSHIMA, Takashi, Hiratsuka-shi Kanagawa 254-0016 (JP); MATSUURA, Yutaka, Kurashiki-shi Okayama 712-8525 (JP); SHISHIMI, Toru, Kurashiki-shi Okayama 712-8525 (JP)
(74) Representative: Müller-Boré & Partner Patentanwälte PartG mbB
(86) International application number: PCT/JP2018/028340
(87) International publication number: WO 2019/022251

(57) **Abstract**

A (poly)amine compound represented by the following formula (0):
wherein
R^{X} is a 2n^{A}-valent group having 1 to 70 carbon atoms or a single bond;
each R^{1A} is independently any of an alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, a halogen atom, a nitro group, an amino group having 0 to 30 carbon atoms and optionally having a substituent, a carboxyl group, a thiol group and a hydroxy group, wherein when R^{1A} is any of the alkyl group, the aryl group, the crosslinking group and the alkoxy group, R^{1A} optionally contains at least one bond selected from the group consisting of an ether bond, a ketone bond and an ester bond, and at least one R^{1A} is an amino group having 0 to 30 carbon atoms and optionally having a substituent;
X is an oxygen atom or a sulfur atom, or X is optionally absent;
each R independently represents any of a benzene ring, a biphenyl ring, a naphthalene ring, an anthracene ring and a pyrene ring;
each m is independently an integer of 0 to 9, wherein at least one m is an integer of 1 to 9; and
n^{A} is an integer of 1 to 4.

## Description

### Technical Field

The present invention relates to a novel (poly)amine compound, resin and cured product.

### Background Art

(Poly)amine compounds have been used as a raw material for functional resins such as polyurea, polyamide, polyimide, polyamideimide, benzoxazine and maleimide, and as a curing agent for curing compounds such as epoxy, urethane, urea and cyanate (see, for example, Patent Literatures 1 to 7).

### Citation List

### Patent Literature

Patent Literature 1: International Publication No. WO 2015/152007
Patent Literature 2: International Publication No. WO 2015/120253
Patent Literature 3: International Publication No. WO 2015/118121
Patent Literature 4: Japanese Patent Laid-Open No. 2015-106629
Patent Literature 5: International Publication No. WO 2014/104557
Patent Literature 6: Japanese Patent Laid-Open No. 2010-235859
Patent Literature 7: Japanese Patent Laid-Open No. 2009-102252

### Summary of Invention

### Technical Problem

However, for development of novel materials, it has been demanded to further improve structure-forming ability (film-forming ability), heat resistance, transparency and refractive index in (poly)amine compounds described in these Patent Literatures.

As such, an object of the present invention is to provide a novel (poly)amine compound that can be used advantageously as a raw material for functional resins such as polyurea, polyamide, polyimide, polyamideimide, benzoxazine and maleimide, and as a curing agent for functional cured products such as epoxy, urethane, urea and cyanate.

### Solution to Problem

The inventors have, as a result of devoted examinations to solve the problems described above, found out that a novel (poly)amine compound having a specific structure is useful as a raw material for functional resins and as a curing agent for functional cured products, and reached the present invention.

More specifically, the present invention is as follows.
[1] A (poly)amine compound represented by the following formula (0): 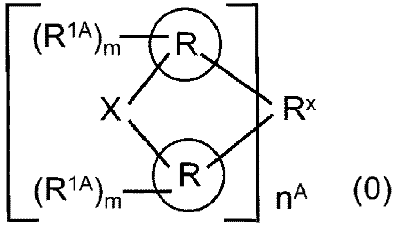 wherein
   R^{X} is a 2n^{A}-valent group having 1 to 70 carbon atoms or a single bond;
   each R^{1A} is independently any of an alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, a halogen atom, a nitro group, an amino group having 0 to 30 carbon atoms and optionally having a substituent, a carboxyl group, a thiol group and a hydroxy group, wherein when R^{1A} is any of the alkyl group, the aryl group, the crosslinking group and the alkoxy group, R^{1A} optionally contains at least one bond selected from the group consisting of an ether bond, a ketone bond and an ester bond, and at least one R^{1A} is an amino group having 0 to 30 carbon atoms and optionally having a substituent;
   X is an oxygen atom or a sulfur atom, or X is optionally absent;
   each R independently represents any of a benzene ring, a biphenyl ring, a naphthalene ring, an anthracene ring and a pyrene ring;
   each m is independently an integer of 0 to 9, wherein at least one m is an integer of 1 to 9; and
   n^{A} is an integer of 1 to 4.
[2] The (poly)amine compound according to [1], wherein the formula (0) is represented by the following formula (1): 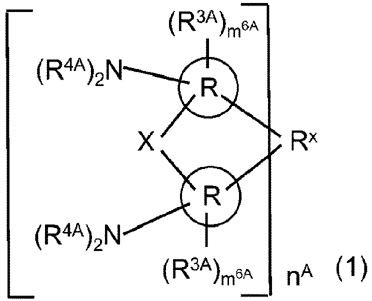 wherein
   each R^{3A} is independently any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, and a halogen atom;
   each R^{4A} is independently any of a hydrogen atom, a linear or branched, or cyclic alkyl group or acyl group having 1 to 30 carbon atoms and optionally having a substituent, an aryl group having 6 to 30 carbon atoms and optionally having a substituent, and a crosslinking group having 2 to 30 carbon atoms and optionally having a substituent, wherein when R^{4A} is any of the alkyl group or acyl group, the aryl group and the crosslinking group, R^{4A} optionally contains at least one bond selected from the group consisting of an ether bond, a ketone bond and an ester bond;
   each m^{6A} is independently an integer of 0 to 5; and
   R^{X}, X, R and n^{A} are as defined above.
[3] The (poly)amine compound according to [2], wherein the formula (1) is represented by the following formula (2) : 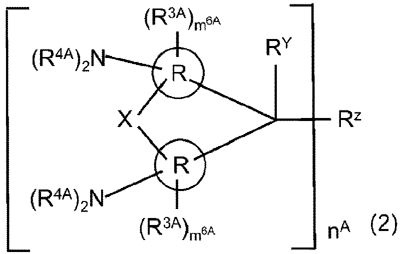 wherein
   R^{Y} is any of a hydrogen atom, a linear or branched, or cyclic alkyl group having 1 to 30 carbon atoms and optionally having a substituent and an aryl group having 6 to 30 carbon atoms and optionally having a substituent;
   R^{Z} is an n^{A}-valent group having 1 to 60 carbon atoms or a single bond, and the total number of carbon atoms in R^{Y} and R^{Z} is 69 or less; and
   X, R, n^{A}, R^{3A}, R^{4A} and m^{6A} are as defined above.
[4] The (poly)amine compound according to [3], wherein the formula (2) is represented by the following formula (3): 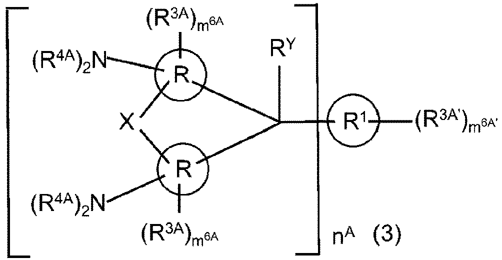
   R^{3A'} is any of a linear or branched, or cyclic alkyl group or alkoxy group having 1 to 30 carbon atoms and optionally having a substituent, an amino group optionally having a substituent, an aryl group having 6 to 30 carbon atoms and optionally having a substituent, a crosslinking group having 2 to 30 carbon atoms and optionally having a substituent, a hydroxy group and a cyano group;
   each R¹ independently represents any of a benzene ring, a biphenyl ring, a naphthalene ring, an anthracene ring, and a heterocyclic ring containing any of an oxygen atom, a sulfur atom and a nitrogen atom as a heteroatom;
   m^{6A'} is an integer of 0 to 5;
   X, R, n^{A}, R^{3A}, R^{4A}, m^{6A} and R^{Y} are as defined above; and
   when R¹ is a benzene ring, R^{3A'} is not a phenyl group.
[5] The (poly)amine compound according to [4], wherein R^{3A'} is any of a linear or branched, or cyclic alkyl group or alkoxy group having 1 to 30 carbon atoms and optionally having a substituent, an aryl group having 6 to 30 carbon atoms and optionally having a substituent, a crosslinking group having 2 to 30 carbon atoms and optionally having a substituent, a hydroxy group and a cyano group; and
   when R¹ is a benzene ring or a naphthalene ring, R^{3A'} is any of a linear or branched, or cyclic alkyl group or alkoxy group having 1 to 30 carbon atoms and optionally having a substituent, an aryl group having 6 to 30 carbon atoms and optionally having a substituent, a crosslinking group having 2 to 30 carbon atoms and optionally having a substituent, a hydroxy group and a cyano group.
[6] The (poly)amine compound according to [4] or [5], wherein the (poly)amine compound represented by the formula (3) is a compound represented by the following formula (0a): 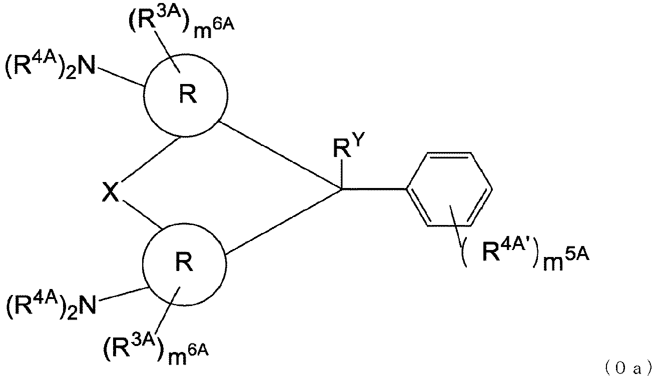 wherein X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom); and m^{5A} represents an integer of 1 to 5, or
   a compound represented by the following formula (0b): 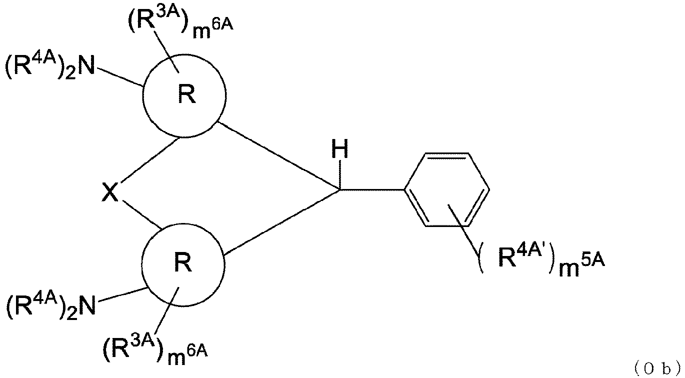 wherein X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom); and m^{5A} represents an integer of 1 to 5.
[7] The (poly)amine compound according to any of [4] to [6], wherein the (poly)amine compound represented by the formula (3) is a compound represented by the following formula (0a-1) : 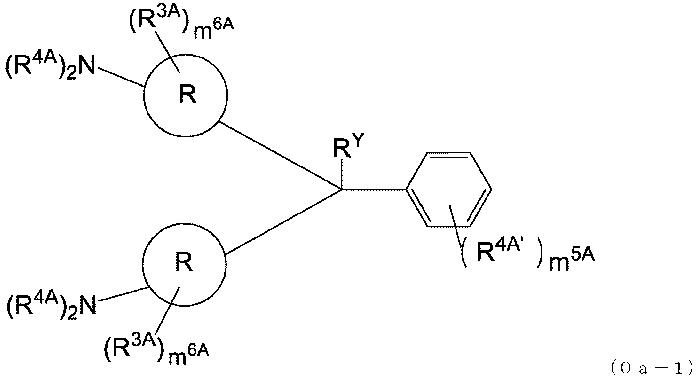 wherein R, R^{3A}, R^{4A}, m^{6A}, R^{4A'}, m^{5A} and R^{Y} are as defined above, or
   a compound represented by the following formula (0b-1): 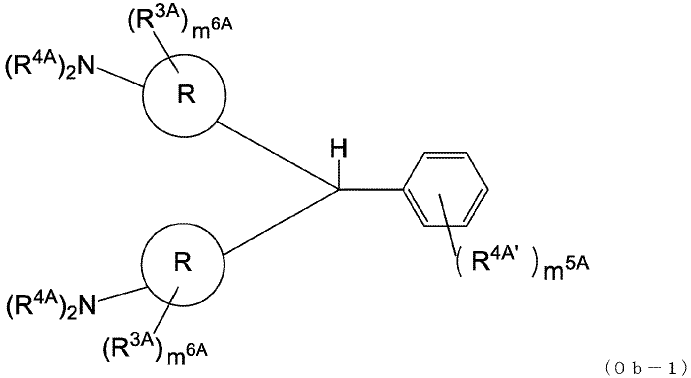 wherein R, R^{3A}, R^{4A}, m^{6A}, m^{5A} and R^{Y} are as defined above.
[8] The (poly)amine compound according to [7], wherein the (poly)amine compound represented by the formula (0b-1) is a compound represented by the following formula (0b-2) : 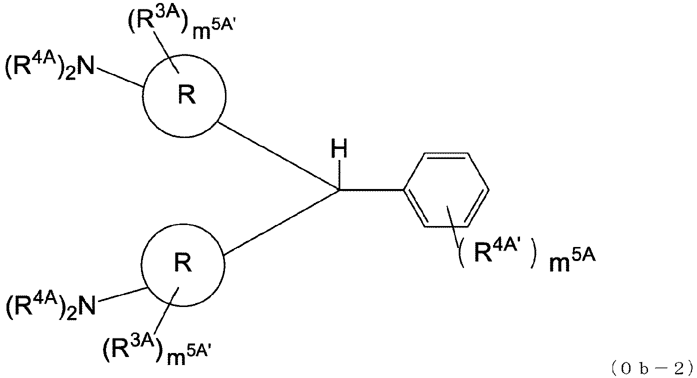 wherein R, R^{3A}, R^{4A}, R^{4A'}, m^{5A} and R^{Y} are as defined above, and each m^{5A'} is independently an integer of 1 to 5.
[10] The (poly)amine compound according to [8], wherein the (poly)amine compound represented by the formula (0a-1) is a compound represented by the following formula (0a-2) : 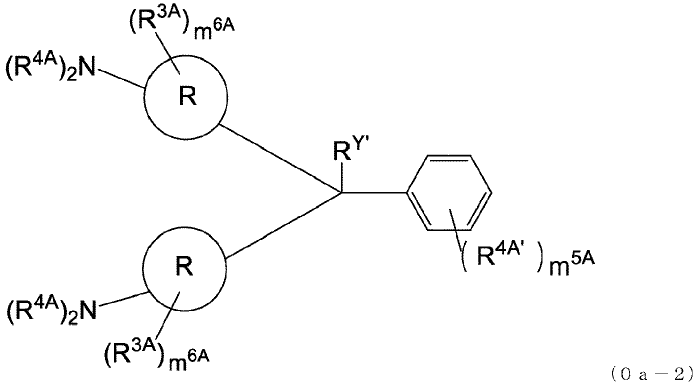 wherein R, R^{3A}, R^{4A}, m^{6A}, m^{5A} and R^{Y} are as defined above, and R^{Y'} is any of a linear or branched, or cyclic alkyl group having 1 to 30 carbon atoms and optionally having a substituent and an aryl group having 6 to 30 carbon atoms and optionally having a substituent.
[11] The (poly)amine compound according to [8], wherein the (poly)amine compound represented by the formula (0b-1) is a compound represented by the following formula (0b-3): 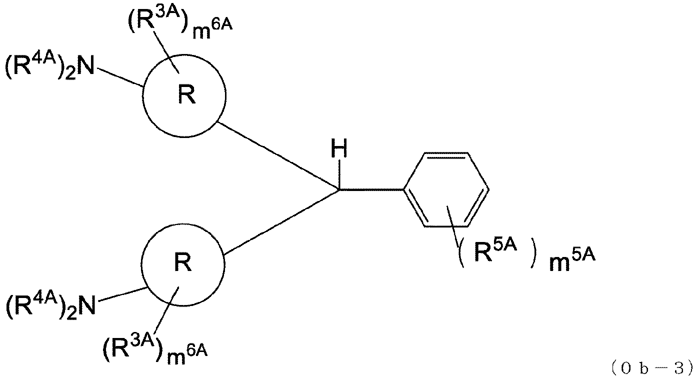 wherein R, R^{3A}, R^{4A}, R^{4A'} and m^{5A} are as defined above, and R^{5A} represents a linear or branched, or cyclic alkyl group having 5 or more carbon atoms and optionally having a substituent.
[12] The (poly)amine compound according to any of [4] to [6], wherein the (poly)amine compound represented by the formula (3) is a compound represented by the following formula (3a): 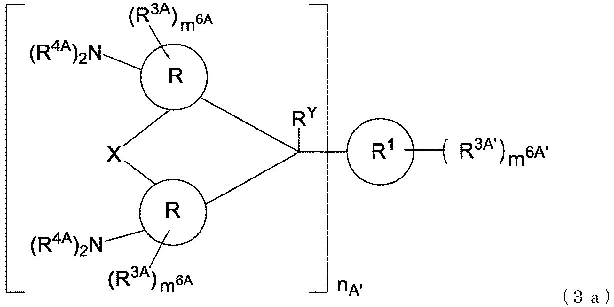 wherein R, X, R^{3A}, R^{4A}, m^{6A}, R^{Y}, R1 and m^{6A'} are as defined above, and n^{A'} is an integer of 2 to 4, or a compound represented by the following formula (3b): 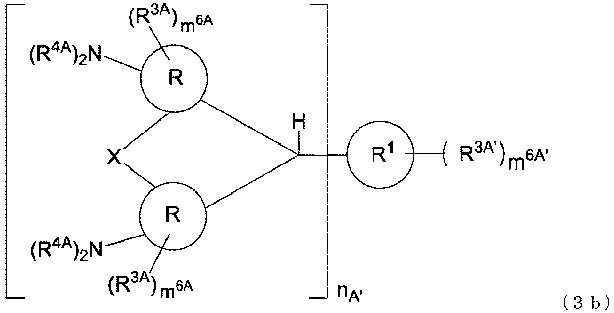 wherein R, X, R^{3A}, R^{4A}, m^{6A}, R1 and m^{6A'} are as defined above, and n^{A'} is an integer of 2 to 4.
[13] The (poly)amine compound according to any of [1] to [12], wherein X is an oxygen atom in the formulas (0), (1), (2) and (3).
[14] The (poly)amine compound according to any of [1] to [13], wherein the (poly)amine compound is any of compounds represented by the following formulas (BiA-1) to (BiA-35): 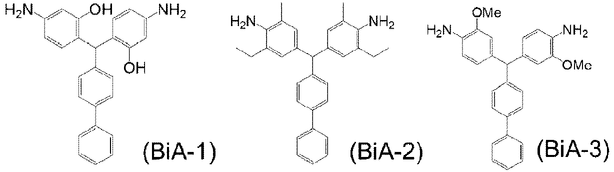 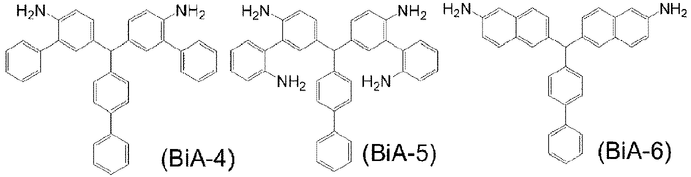 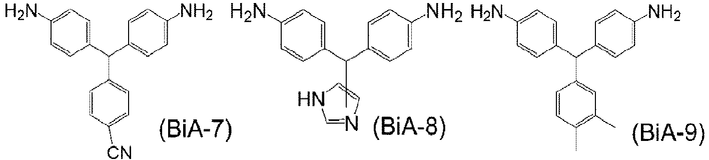 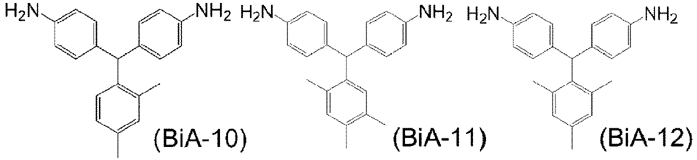 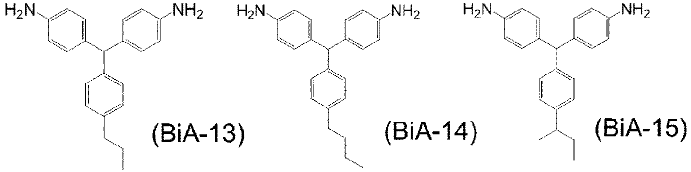 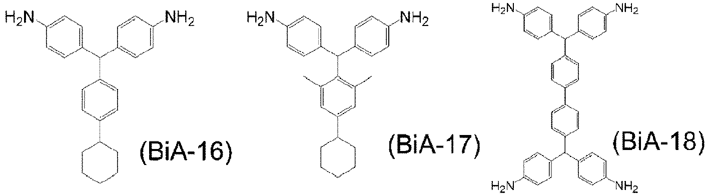 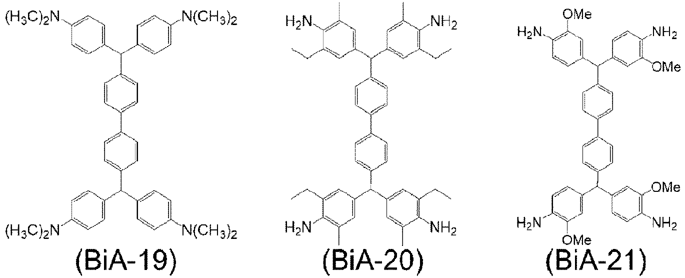 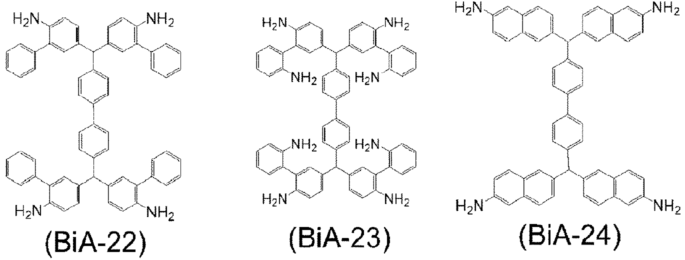 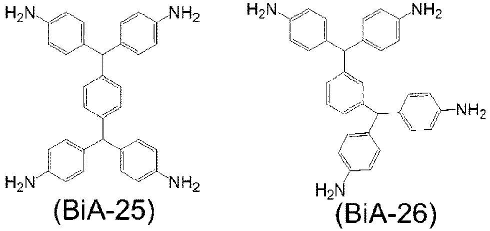 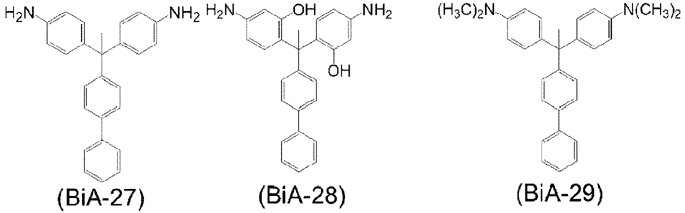 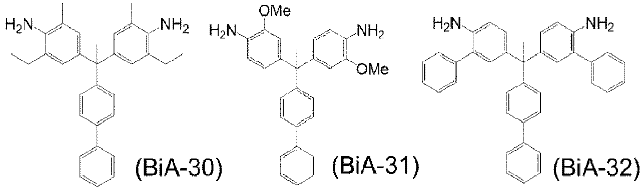 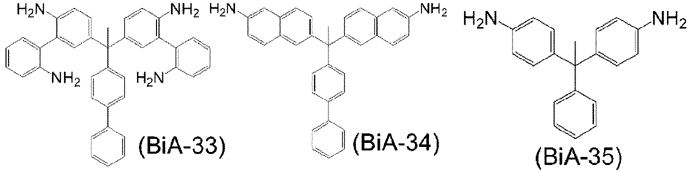
[15] A resin comprising a monomer unit containing a unit derived from the (poly)amine compound according to any of [1] to [14].
[16] The resin according to [15], wherein the monomer unit containing a unit derived from the (poly)amine compound is a unit represented by the following formula (4) : 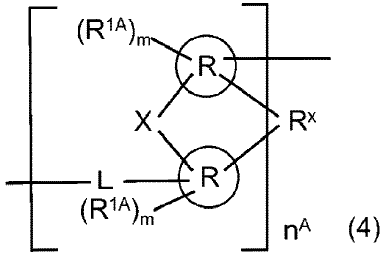 wherein
   L is a linear or branched linking group having 1 to 30 carbon atoms, or a single bond; and
   R^{X}, R^{1A}, X, R, m and n^{A} are as defined above.
[17] A cured product obtained by curing a curable compound with a curing agent,
   wherein the curing agent comprises the (poly)amine compound according to any of [1] to [14].

### Advantageous Effects of Invention

According to the present invention, it is possible to provide a novel (poly)amine compound that can be used advantageously as a raw material for functional resins such as polyurea, polyamide, polyimide, polyamideimide, benzoxazine and maleimide, and as a curing agent for functional cured products such as epoxy, urethane, urea and cyanate.

### Description of Embodiments

Hereinafter, embodiments of the present invention (hereinafter, simply referred to as the "present embodiment") will be described in detail. In the present embodiment, a "(poly)amine compound" refers to a monoamine compound or a polyamine compound.

### [(Poly)amine Compound Represented by Formula (0)]

A (poly)amine compound according to the present embodiment is represented by the following formula (0). The (poly)amine compound according to the present embodiment can be used advantageously as a raw material for functional resins such as polyurea, polyamide, polyimide, polyamideimide, benzoxazine and maleimide, and as a curing agent for functional cured products such as epoxy, urethane, urea and cyanate. Particularly, the (poly)amine compound according to the present embodiment has excellent structure-forming ability (for example, film-forming ability or the like), heat resistance, transparency and refractive index due to the fact that it has a high aromatic density and low crystallinity.

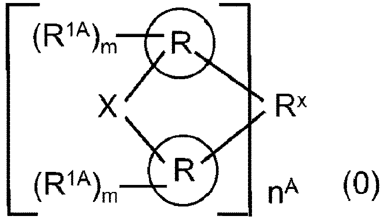

In the above formula (0), R^{X} is a 2n^{A}-valent group having 1 to 70 carbon atoms or a single bond; each R^{1A} is independently any of an alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, a halogen atom, a nitro group, an amino group having 0 to 30 carbon atoms and optionally having a substituent, a carboxyl group, a thiol group and a hydroxy group, wherein when R^{1A} is any of the alkyl group, the aryl group, the crosslinking group and the alkoxy group, R^{1A} optionally contains at least one bond selected from the group consisting of an ether bond, a ketone bond and an ester bond, and at least one R^{1A} is an amino group having 0 to 30 carbon atoms and optionally having a substituent; X is an oxygen atom or a sulfur atom, or X is optionally absent; each R independently represents any of a benzene ring, a biphenyl ring, a naphthalene ring, an anthracene ring and a pyrene ring; each m is independently an integer of 0 to 9, wherein at least one m is an integer of 1 to 9; and n^{A} is an integer of 1 to 4.

In the present specification, an "alkyl group" may be a linear or branched alkyl group, or may be a cyclic alkyl group. Also, an "alkoxy group" may be a linear or branched alkoxy group, or may be a cyclic alkoxy group.

### (1. R^{X})

In the above formula (0), R^{X} is a 2n^{A}-valent group or a single bond, and is preferably a 2n^{A}-valent group. The number of carbon atoms in the 2n^{A}-valent group is 1 to 70, preferably 3 to 50, and more preferably 6 to 30.

### (1-1. 2n^{A}-Valent Group)

Examples of the 2n^{A}-valent group include, but are not particularly limited to, hydrocarbon groups.
Examples of the hydrocarbon group include linear or branched hydrocarbon groups, alicyclic hydrocarbon groups, and a combination of linear or branched hydrocarbon groups and alicyclic hydrocarbon groups. The "alicyclic hydrocarbon group" in the present specification may be one of those having a bridged structure in the aliphatic ring, so-called polycyclic bridged alicyclic hydrocarbon groups. In addition, the hydrocarbon group may contain any of a double bond, a heteroatom and an aromatic group having 6 to 60 carbon atoms.

### (1-1-1. Group Represented by Formula (0a))

Examples of the 2n^{A}-valent group include a group represented by the following formula (0a).

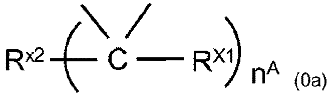

In the above formula (0a), each R^{x1} is independently any of a hydrogen atom, a linear or branched, or cyclic alkyl group having 1 to 30 carbon atoms and optionally having a substituent, and an aryl group (hydrocarbon group) having 6 to 30 carbon atoms and optionally having a substituent, wherein when R^{X1} is the alkyl group or the aryl group, R^{X1} optionally contains at least one bond selected from the group consisting of an ether bond, a ketone bond and an ester bond; R^{X2} is any of a hydrogen atom and an n^{A}-valent group having 1 to 60 carbon atoms, and the total number of carbon atoms in R^{X1} and R^{X2} is 69 or less; and n^{A} represents an integer of 1 to 4.

### (1-1-1a. R^{X1})

Examples of the linear or branched alkyl group for R^{x1} may include linear or branched alkyl groups having 1 to 30 carbon atoms, such as a methyl group, an ethyl group, a n-propyl group, an isopropyl group, a n-butyl group, an isobutyl group, a tert-butyl group, a n-pentyl group, a neopentyl group, a tert-pentyl group, a n-hexyl group, a n-heptyl group, a 2,2,4-trimethylpentyl group, a n-octyl group, an isooctyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group, a n-heneicosyl group, a n-tricosyl group, a n-pentacocyl group, a n-heptacocyl group and a n-nonacocyl group, and it is preferably a linear or branched alkyl group having 1 to 20 carbon atoms and more preferably a linear or branched alkyl group having 1 to 10 carbon atoms from the viewpoint of exhibiting effects of the present invention more effectively and certainly. Examples of the cyclic alkyl group include monocyclic groups (monocyclic cycloalkyl groups) and polycyclic groups (polycyclic cycloalkyl groups). Examples of the monocyclic group include monocyclic groups having 3 to 30 carbon atoms, such as a cyclopropyl group, a cyclobutyl group, a cyclopentyl group, a cyclohexyl group, a cyclopentyl group, a cycloheptyl group, a cyclooctyl group, a cyclodecyl group and a cycloicosyl group. Examples of the polycyclic groups include polycyclic groups having 7 to 30 carbon atoms, such as a dicyclopentyl group, a dicyclohexyl group, a norbornyl group, an adamantyl group, a tricyclodecyl group and a tetracyclododecyl group.

The alkyl group may have a substituent. Examples of the substituent include a halogen atom (for example, a fluorine atom, a chlorine atom and a bromine atom), a nitro group, an amino group optionally having a substituent, a carboxyl group, a thiol group and a hydroxy group. The number of the substituent is not particularly limited, and it may be one or may be multiple.

Examples of the aryl group include, but are not particularly limited to, aryl groups having 6 to 30 carbon atoms such as a phenyl group, a naphthyl group (for example, a 1-naphthyl group and a 2-naphthyl group), an anthryl group (for example, a 1-anthryl group) and a phenanthryl group (for example, a 1-phenanthryl group), and it is preferably an aryl group having 6 to 10 carbon atoms such as a phenyl group and a naphthyl group, and is more preferably a phenyl group from the viewpoint of exhibiting effects of the present invention more effectively and certainly.

The aryl group may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple.

### (1-1-1b. R^{X2})

Examples of the n^{A}-valent group having 1 to 60 carbon atoms include linear or branched alkyl groups having 1 to 60 carbon atoms and optionally having a substituent (n^{A} = 1), linear or branched alkylene groups having 1 to 60 carbon atoms and optionally having a substituent (n^{A} = 2), linear or branched alkyltriyl groups having 1 to 60 carbon atoms and optionally having a substituent (n^{A} = 3), linear or branched alkyltetrayl groups having 1 to 60 carbon atoms and optionally having a substituent (n^{A} = 4), n^{A}-valent aromatic hydrocarbon rings having 6 to 60 carbon atoms and optionally having a substituent, and n^{A}-valent alicyclic hydrocarbon rings having 3 to 60 carbon atoms and optionally having a substituent.

Examples of the linear or branched alkyl group having 1 to 60 carbon atoms include those exemplified as the alkyl group in (1-1-1a. R^{X1}), and examples of the alkyl group that is preferable from the viewpoint of exhibiting effects of the present invention more effectively and certainly also include those exemplified as the preferable alkyl group in (1-1-1a. R^{X1}). The alkyl group may have a substituent, and examples of the substituent include those exemplified as the substituent for the alkyl group in (1-1-1a. R^{X1}).

Examples of the linear or branched alkylene group having 1 to 60 carbon atoms include groups obtained by removing one hydrogen atom from alkyl groups exemplified as the linear or branched alkyl group having 1 to 60 carbon atoms. The alkylene group may have a substituent, and examples of the substituent include those exemplified as the substituent for the alkyl group in (1-1-1a. R^{X1}).

Examples of the linear or branched alkyltriyl group having 1 to 60 carbon atoms include groups obtained by removing two hydrogen atoms from alkyl groups exemplified as the linear or branched alkyl group having 1 to 60 carbon atoms. The alkyltriyl group may have a substituent, and examples of the substituent include those exemplified as the substituent for the alkyl group in (1-1-1a. R^{X1}).

Examples of the linear or branched alkyltetrayl group having 1 to 60 carbon atoms include groups obtained by removing three hydrogen atoms from alkyl groups exemplified as the linear or branched alkyl group having 1 to 60 carbon atoms. The alkyltetrayl group may have a substituent, and examples of the substituent include those exemplified as the substituent for the alkyl group in (1-1-1a. R^{X1}).

Examples of the aromatic hydrocarbon ring include monocyclic aromatic hydrocarbon rings, condensed cyclic aromatic hydrocarbon rings, and rings (ring-aggregated rings) in which multiple aromatic rings are bonded to each other directly, or via a linking group.

The aromatic hydrocarbon ring may contain a heteroatom (for example, an oxygen atom, a nitrogen atom and a sulfur atom), and may contain at least one bond selected from the group consisting of an ether bond, a ketone bond and an ester bond.

Examples of the monocyclic aromatic hydrocarbon ring include a benzene ring, and five-membered or six-membered, aromatic heterocyclic rings containing a heteroatom. Examples of the five-membered or six-membered, aromatic heterocyclic ring containing a heteroatom include a thiophene ring, a pyridine ring, a furan ring, a thiazole ring, an oxazole ring, a pyrazole ring, a pyrazine ring, a pyrimidine ring, a pyrrole ring, an imidazole ring, a pyridazine ring, an isothiazole ring and an isoxazole ring. Among these monocyclic aromatic hydrocarbon rings, a benzene ring is preferable from the viewpoint of exhibiting effects of the present invention more effectively and certainly. These monocyclic aromatic hydrocarbon rings may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the monocyclic aromatic hydrocarbon ring is substituted with the substituent is not particularly limited, either.

For the monocyclic aromatic hydrocarbon ring, a benzene ring is preferable from the viewpoint of exhibiting effects of the present invention more effectively and certainly. When a benzene ring has a substituent, it is preferable that the benzene ring has a substituent at position 4.

More specifically, examples of the monocyclic aromatic hydrocarbon ring having a substituent include (i) hydroxyphenyl groups, (ii) monoalkylphenyl groups, (iii) dialkylphenyl groups, (iv) trialkylphenyl groups and (v) cyclohexylphenyl groups when n^{A} is 1.
(i) Examples of the hydroxyphenyl group include:
   (i-a) monohydroxyphenyl groups (for example, a 2-hydroxyphenyl group, a 3-hydroxyphenyl group and a 4-hydroxyphenyl group);
   (i-b) dihydroxyphenyl groups (for example, a 2,3-dihydroxyphenyl group, a 2,4-dihydroxyphenyl group, a 2,5-dihydroxyphenyl group, a 2,6-dihydroxyphenyl group, a 3,4-dihydroxyphenyl group and a 3,5-dihydroxyphenyl group); and
   (i-c) trihydroxyphenyl groups (for example, a 2,3,4-trihydroxyphenyl group, a 2,3,5-trihydroxyphenyl group, a 2,3,6-trihydroxyphenyl group, a 2,4,5-trihydroxyphenyl group and a 2,4,6-trihydroxyphenyl group).
(ii) Examples of the monoalkylphenyl group include: a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-butylphenyl group, a 3-butylphenyl group and a 4-butylphenyl group.
(iii) Examples of the dialkylphenyl group include:
   (iii-a) 2,3-dialkylphenyl groups (for example, a 2,3-dimethylphenyl group, a 2,3-diethylphenyl group, a 2,3-dipropylphenyl group, a 2,3-diisopropylphenyl group, a 2-ethyl-3-methylphenyl group and a 3-ethyl-2-methylphenyl group);
   (iii-b) 2,4-dialkylphenyl groups (for example, a 2,4-dimethylphenyl group, a 2,4-diethylphenyl group, a 2.4-dipropylphenyl group, a 2,4-diisopropylphenyl group, a 2-ethyl-4-methylphenyl group and a 4-ethyl-2-methylphenyl group); (iii-c) 2,5-dialkylphenyl groups (for example, a 2,5-dimethylphenyl group, a 2,5-diethylphenyl group, a 2,5-dipropylphenyl group, a 2,5-diisopropylphenyl group, a 2-ethyl-5-methylphenyl group and a 5-ethyl-2-methylphenyl group);
   (iii-d) 2,6-dialkylphenyl groups (for example, a 2,6-dimethylphenyl group, a 2,6-diethylphenyl group, a 2,6-dipropylphenyl group, a 2,6-diisopropylphenyl group, a 2-ethyl-6-methylphenyl group and a 6-ethyl-2-methylphenyl group);
   (iii-e) 3,4-dialkylphenyl groups (for example, a 3,4-dimethylphenyl group, a 3,4-diethylphenyl group, a 3,4-dipropylphenyl group, a 3,4-diisopropylphenyl group, a 3-ethyl-4-methylphenyl group and a 4-ethyl-3-methylphenyl group); and
   (iii-f) 3,5-dialkylphenyl groups (for example, a 3,5-dimethylphenyl group, a 3,5-diethylphenyl group, a 3,5-dipropylphenyl group, a 3,5-diisopropylphenyl group, a 3-ethyl-5-methylphenyl group and a 5-ethyl-3-methylphenyl group).
(iv) Examples of the trialkylphenyl group include:
   (iv-a) trimethylphenyl groups (for example, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group and a 3,4,5-trimethylphenyl group);
   (iv-b) ethyldimethylphenyl groups (for example, a group obtained by substituting one methyl group in the above trimethylphenyl group with one ethyl group);
   (iv-c) diethylmethylphenyl groups (for example, a group obtained by substituting each of two methyl groups in the above trimethylphenyl group with an ethyl group); and (iv-d) triethylphenyl groups (for example, a group obtained by substituting each of three methyl groups in the above trimethylphenyl group with an ethyl group).
(v) Examples of the cycloalkylphenyl group include cyclohexylphenyl groups such as a 2-cyclohexylphenyl group, a 3-cyclohexylphenyl group and a 4-cyclohexylphenyl group.

Examples of the monocyclic aromatic hydrocarbon ring having a substituent include (i) hydroxyphenylene groups obtained by removing one hydrogen atom from phenyl groups of hydroxyphenyl groups exemplified as the hydroxyphenyl group, (ii) monoalkylphenylene groups obtained by removing one hydrogen atom from phenyl groups of monoalkylphenyl groups exemplified as the monoalkylphenyl group, (iii) dialkylphenylene groups obtained by removing one hydrogen atom from phenyl groups of dialkylphenyl groups exemplified as the dialkylphenyl group, (iv) trialkylphenylene groups obtained by removing one hydrogen atom from phenyl groups of trialkylphenyl groups exemplified as the trialkylphenyl group, and (v) cyclohexylphenylene groups obtained by removing one hydrogen atom from cyclohexylphenyl groups exemplified as the cyclohexylphenyl group when n^{A} is 2.

Examples of the monocyclic aromatic hydrocarbon ring having a substituent include (i) hydroxyphenyltriyl groups obtained by removing two hydrogen atoms from phenyl groups of hydroxyphenyl groups exemplified as the hydroxyphenyl group, (ii) monoalkylphenyltriyl groups obtained by removing two hydrogen atoms from phenyl groups of monoalkylphenyl groups exemplified as the monoalkylphenyl group, (iii) dialkylphenyltriyl groups obtained by removing two hydrogen atoms from phenyl groups of dialkylphenyl groups exemplified as the dialkylphenyl group, (iv) trialkylphenyltriyl groups obtained by removing two hydrogen atoms from phenyl groups of trialkylphenyl groups exemplified as the trialkylphenyl group, and (v) cyclohexylphenyltriyl groups obtained by removing two hydrogen atoms from cyclohexylphenyl groups exemplified as the cyclohexylphenyl group when n^{A} is 3.

Examples of the monocyclic aromatic hydrocarbon ring having a substituent include (i) hydroxyphenyltetrayl groups obtained by removing three hydrogen atoms from phenyl groups of hydroxyphenyl groups exemplified as the hydroxyphenyl group, (ii) monoalkylphenyltetrayl groups obtained by removing three hydrogen atoms from phenyl groups of monoalkylphenyl groups exemplified as the monoalkylphenyl group, (iii) dialkylphenyltetrayl groups obtained by removing three hydrogen atoms from phenyl groups of dialkylphenyl groups exemplified as the dialkylphenyl group, (iv) trialkylphenyltetrayl groups obtained by removing three hydrogen atoms from phenyl groups of trialkylphenyl groups exemplified as the trialkylphenyl group, and (v) cyclohexylphenyltetrayl groups obtained by removing three hydrogen atoms from cyclohexylphenyl groups exemplified as the cyclohexylphenyl group when n^{A} is 4.

Examples of the condensed cyclic aromatic hydrocarbon ring include condensed bicyclic aromatic hydrocarbon rings (for example, a naphthalene ring and an indene ring), condensed tricyclic aromatic hydrocarbon rings (for example, an anthracene ring and a phenanthrene ring), condensed tetracyclic aromatic hydrocarbon rings (for example, a tetracene ring and a pyrene ring), rings having an acenaphthene skeleton (for example, an acenaphthene ring and a 1-acenaphthenone ring), and rings having a fluorene skeleton (for example, a fluorene ring, benzofluorene rings such as a 2,3-benzofluorene ring, dibenzofluorene rings such as a 2,3:6,7-dibenzofluorene ring).

These condensed cyclic aromatic hydrocarbon rings may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the condensed cyclic aromatic hydrocarbon ring is substituted with the substituent is not particularly limited, either.

Examples of the ring (biarene ring) in which multiple aromatic rings are bonded to each other directly include rings in which two benzene rings are bonded to each other directly (biphenyl rings), rings in which two naphthalene rings are bonded to each other directly (binaphtyl rings), and rings in which a benzene ring and a naphthalene ring are bonded to each other directly (binaphtylphenyl rings).

The biarene ring may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the biarene ring is substituted with the substituent is not particularly limited, either.

Examples of the ring in which multiple aromatic hydrocarbon rings are bonded to each other via a linking group (the ring-aggregated ring in which rings are bonded to each other via a linking group) include rings represented by the formula: R^{b1}-L^{a}-R^{b2}, wherein R^{b1} and R^{b2} represent either a benzene ring or a naphthalene ring and L^{a} represents a linking group. In the above formula, examples of L^{a} include groups containing an oxygen atom (for example, an ether group (-O-), a carbonyl group (-CO-) and an oxycarbonyl group (-OCO-)); groups containing a sulfur atom (for example, a thio group (-S-)); and alkylene groups (for example, linear or branched alkylene groups such as a methylene group and an ethylene group, and cycloalkylene groups such as a cyclohexylidene group), and it is preferably a group containing an oxygen atom, and is more preferably an ether group from the viewpoint of exhibiting effects of the present invention more effectively and certainly.

These ring-aggregated rings in which rings are bonded to each other via a linking group may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the ring-aggregated ring in which rings are bonded to each other via a linking group is substituted with the substituent is not particularly limited, either.

Examples of the alicyclic hydrocarbon ring include monocyclic aliphatic hydrocarbon rings and polycyclic aliphatic hydrocarbon rings.

Examples of the monocyclic aliphatic hydrocarbon ring include cycloalkane rings such as a cyclobutane ring, a cyclopentane ring, a cyclohexane ring and a cycloheptane ring. The monocyclic aliphatic hydrocarbon ring may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the monocyclic aliphatic hydrocarbon ring is substituted with the substituent is not particularly limited, either.

Examples of the polycyclic aliphatic hydrocarbon ring include a decalin ring, an adamantane ring, a dicyclopentane ring, a norbornane ring, a tricyclodecane ring, and rings having at least one double bond in these rings (for example, a dicyclopentadiene ring and the like). The polycyclic aliphatic hydrocarbon ring may have a substituent. Examples of the substituent include those exemplified as the substituent for the alkyl group in (1-1-1a. R^{X1}), and alkyl groups exemplified as the alkyl group in (1-1-1a. R^{X1}). The number of the substituent is not particularly limited, and it may be one or may be multiple. The substitution position where the polycyclic aliphatic hydrocarbon ring is substituted with the substituent is not particularly limited, either.

Examples of the representative R^{X2} include those obtained by removing one hydrogen atom from the rings (x1) to (x21) shown below. In the following formulas (x1), (x2) and (x3), R* represents any of a hydrogen atom, a methyl group, an ethyl group, a n-propyl group, a n-butyl group, a cyclohexyl group and a hydroxy group.

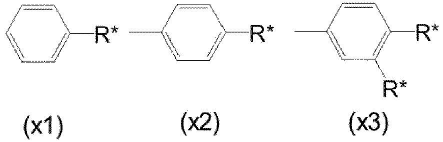

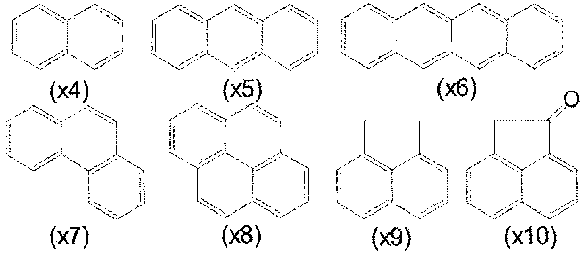

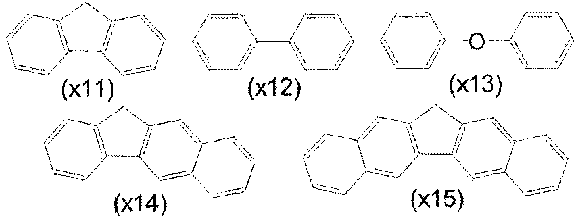

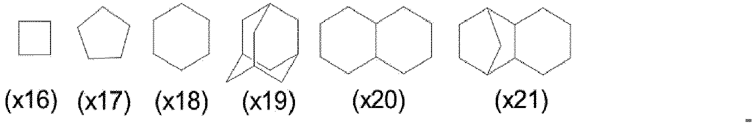

Examples of the representative combination of R^{X1}, R^{X2} and n^{A} in the formula (0a) include a combination in which R^{X1} is any of a hydrogen atom, a methyl group and a phenyl group, R^{X2} is any of groups (x1) to (x21), and n^{A} is 1.

In addition, the 2n^{A}-valent group may be a group represented by the following formula (Cy1) when n^{A} = 1, may be a group represented by the following formula (Cy2) when n^{A} = 2, may be a group represented by the following formula (Cy3) when n^{A} = 3 and represents a cycloalkanetetrayl group having 3 to 70 carbon atoms, and may be a group represented by the following formula (Cy4) when n^{A} = 4.

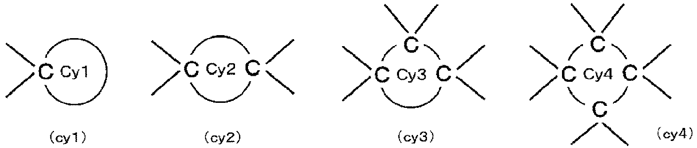

In the above formulas (cy1), (cy2), (cy3) and (cy4), cy1 is a divalent group obtained by removing one hydrogen atom from cyclic alkyl groups or aryl groups exemplified as R^{x2}, cy2 is a tetravalent group obtained by removing three hydrogen atoms from cyclic alkyl groups or aryl groups exemplified as R^{x2}, cy3 is a hexavalent group obtained by removing one hydrogen atom from cyclic alkyl groups or aryl groups exemplified as R^{x2}, and cy4 is an octavalent group obtained by removing one hydrogen atom from cyclic alkyl groups or aryl groups exemplified as R^{x2}.

More specifically, examples of the group represented by the formulas (cy1) to (cy4) include those obtained by removing 2n^{A} hydrogen atoms (n^{A} = 1, 2, 3, 4) from (x9), (x10), (x11) and (x14) to (x21).

### (2. R^{1A})

In the formula (0), each R^{1A} independently represents any of an alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, a halogen atom (for example, a fluorine atom, a chlorine atom and a bromine atom), a nitro group, an amino group having 0 to 30 carbon atoms and optionally having a substituent, a carboxyl group, a thiol group and a hydroxy group. When R^{1A} is any of the alkyl group, the aryl group, the alkenyl group, the alkynyl group and the alkoxy group, R^{1A} optionally contains at least one selected from the group consisting of an ether bond, a ketone bond and an ester bond. However, at least one R^{1A} represents an amino group having 1 to 30 carbon atoms and optionally having a substituent.

Examples of the alkyl group having 1 to 30 carbon atoms and optionally having a substituent include those exemplified as the alkyl group in (1-1-1a. R^{X1}). Among them, from the viewpoint of exhibiting effects of the present invention more effectively and certainly, it is preferably a linear or branched alkyl group having 1 to 6 carbon atoms such as a methyl group, an ethyl group, a n-propyl group, an isopropyl group, a n-butyl group, an isobutyl group and a tert-butyl group, is more preferably a linear or branched alkyl group having 1 to 3 carbon atoms, and is further preferably a methyl group or an ethyl group. In addition, when these alkyl groups have a substituent, the substituent is preferably a halogen atom (a fluorine atom, a chlorine atom and a bromine atom) from the viewpoint of exhibiting effects of the present invention more effectively and certainly. An alkyl group substituted with a halogen atom is preferably a haloalkyl group having 1 to 6 carbon atoms, such as a trifluoromethyl group, a trichloromethyl group and a tetrafluoroethyl group.

Examples of the aryl group having 6 to 30 carbon atoms and optionally having a substituent include those exemplified as the aryl group in (1-1-1a. R^{X1}). Among them, from the viewpoint of exhibiting effects of the present invention more effectively and certainly, it is preferably a phenyl group or a naphthyl group (a 1-naphthyl group and a 2-naphthyl group), and is more preferably a phenyl group. In addition, when these aryl groups have a substituent, the substituent is preferably an amino group, and the substitution position where the aryl group (particularly, a phenyl group) is substituted with the amino group is preferably position 2 or position 4.

The crosslinking group having 2 to 30 carbon atoms and optionally having a substituent is, for example, a group that is capable of producing a new bond with another compound due to specific conditions (for example, acid or base catalyst existence conditions, heating conditions, conditions subjected to radical reaction, irradiation conditions for visible light or invisible light (for example, ultraviolet light, infrared light and the like), and the like), and examples thereof include an allyl group, a (meth)acryloyl group, a vinyl group, an epoxy group, an alkoxymethyl group and a cyanato group.

Examples of the alkoxy group having 1 to 30 carbon atoms and optionally having a substituent include groups represented by -O-R^{a1}, wherein R^{a1} represents an alkyl group exemplified as the alkyl group in (1-1-1a. R^{x1}). Among them, from the viewpoint of exhibiting effects of the present invention more effectively and certainly, it is preferably a linear or branched alkoxy group having 1 to 6 carbon atoms such as a methoxy group, an ethoxy group, a n-propoxy group, an isopropoxy group, a n-butoxy group, an isobutoxy group and a tert-butoxy group, is more preferably a linear or branched alkoxy group having 1 to 3 carbon atoms, and is further preferably a methoxy group or an ethoxy group.

Examples of the amino group having 0 to 30 carbon atoms and optionally having a substituent include groups represented by (R⁴)₂N-, wherein each R⁴ independently represents any of a hydrogen atom, a linear or branched, or cyclic alkyl group having 1 to 30 carbon atoms and optionally having a substituent, an aryl group having 6 to 30 carbon atoms and optionally having a substituent, and a crosslinking group having 2 to 30 carbon atoms and optionally having a substituent, and when R⁴ is any of the alkyl group, the aryl group and the crosslinking group, R⁴ optionally contains at least one bond selected from the group consisting of an ether bond, a ketone bond and an ester bond. Examples of the linear or branched, or cyclic alkyl group having 1 to 30 carbon atoms may include those exemplified as the alkyl group in (1-1-1a. R^{X1}). Examples of the aryl group having 6 to 30 carbon atoms and optionally having a substituent may include those exemplified as the aryl group in (1-1-1a. R^{X1}). Examples of the crosslinking group having 2 to 30 carbon atoms and optionally having a substituent may include those exemplified as the crosslinking group in (2. R^{1A}).

From the viewpoint of exhibiting effects of the present invention more effectively and certainly, the group represented by the above formula (R⁴)₂N- is preferably an amino group; a monosubstituted amino group in which one R⁴ is a hydrogen atom and the other R⁴ is an alkyl group (for example, a linear or branched alkyl group having 1 to 4 carbon atoms such as a methyl group, an ethyl group, a n-propyl group and a n-butyl group) or an alkylcarbonyl group (for example, a linear or branched alkylcarbonyl group having 2 to 5 carbon atoms such as a methylcarbonyl group, an ethyl carbonyl group, a n-propylcarbonyl group and a n-butylcarbonyl group); or a disubstituted amino group in which each of two R⁴s is an alkyl group (for example, a linear or branched alkyl group having 1 to 4 carbon atoms such as a methyl group, an ethyl group, a n-propyl group and a n-butyl group) or an alkylcarbonyl group (for example, a linear or branched alkylcarbonyl group having 2 to 5 carbon atoms such as a methylcarbonyl group, an ethyl carbonyl group, a n-propylcarbonyl group and a n-butylcarbonyl group), and is more preferably an amino group; a monosubstituted amino group in which one R⁴ is a hydrogen atom and the other R⁴ is a methyl group or a methylcarbonyl group; or a disubstituted amino group in which each of two R⁴s is a methyl group or a methylcarbonyl group.

At least one R^{1A} represents an amino group having 1 to 30 carbon atoms and optionally having a substituent. The number of the amino group is not particularly limited as long as it is 1 or more, but from the viewpoint of exhibiting effects of the present invention more effectively and certainly, it is preferably 1 to 3, and is more preferably 1 or 2. When multiple amino groups are present, for example, each amino group may substitute the same aromatic ring or may substitute different aromatic rings.

### (3. X)

In the formula (0), X represents an oxygen atom or a sulfur atom, or X is optionally absent, but from the viewpoint of further excellent structure-forming ability, it is preferably an oxygen atom or a sulfur atom, and is more preferably an oxygen atom. Compounds represented by the formula (0) in which X represents an oxygen atom are represented by the following formula (0-1). 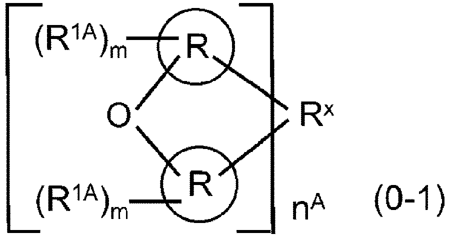 In the above formula (0-1), R^{X}, R^{1A}, R, m and n^{A} are as defined above.

### (4. m)

Each m is independently an integer of 0 to 9. However, at least one m is an integer of 1 to 9. From the viewpoint of exhibiting effects of the present invention more effectively and certainly, m is preferably an integer of 1 to 5, is more preferably an integer of 1 to 3, and is further preferably an integer of 1 or 2.

### (5. n^{A})

n^{A} is an integer of 1 to 4, and from the viewpoint of exhibiting effects of the present invention more effectively and certainly, it is preferably an integer of 1 or 2, and is more preferably 1.

### (6. R)

Each R independently represents any of a benzene ring, a naphthalene ring, an anthracene ring and a pyrene ring. In the above formula (0), when R is a benzene ring, it is shown that the benzene ring is substituted with one or more R^{1A}s, and the substitution position for each R^{1A} to the benzene ring is not particularly limited, and the site of bonding for X to the benzene ring is not particularly limited, either. In the above formula (0), when R is a naphthalene ring, it is shown that the naphthalene ring is substituted with one or more R^{1A}s, and the substitution position for each R^{1A} to the naphthalene ring is not particularly limited, and the site of bonding for X to the naphthalene ring is not particularly limited, either. In the above formula (0), when R is an anthracene ring, it is shown that the anthracene ring is substituted with one or more R^{1A}s, and the substitution position for each R^{1A} to the anthracene ring is not particularly limited, and the site of bonding for X to the anthracene ring is not particularly limited, either. From the viewpoint of exhibiting effects of the present invention more effectively and certainly, R is preferably a benzene ring or a naphthalene ring, and is more preferably a benzene ring.

The (poly)amine compound represented by the above formula (0) is, as mentioned above, a (poly)amine compound that has a high aromatic density and low crystallinity, and has excellent structure-forming ability (for example, film-forming ability), heat resistance, transparency and refractive index. As such, the compound according to the present embodiment can be used advantageously as a raw material for functional resins such as polyurea, polyamide, polyimide, polyamideimide, benzoxazine and maleimide, and as a curing agent for functional cured products such as epoxy, urethane, urea and cyanate. Functional resins and functional cured products are used advantageously in electrical insulating materials, resins for solder resists, encapsulation resins for semiconductors, adhesives for printed circuit boards, electrical laminates mounted in electric equipment, electronic equipment, industrial equipment, and the like, matrix resins of prepregs mounted in electric equipment, electronic equipment, industrial equipment, and the like, buildup laminate materials, resins for fiber-reinforced plastics, resins for encapsulation of liquid crystal display panels, coating materials, various coating agents, adhesives, coating agents for semiconductors, resins for resists for semiconductor lithography, resins for underlayer film formation, and additive agents, for which properties such as high heat resistance and high refractive index are required. Functional resins and functional cured products are used in the form of a film or a sheet when they are used for the above materials. In addition, functional resins and functional cured products are also used advantageously as a component or the like in a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, a photosensitive optical waveguide or the like.

### [(Poly)amine Compound Represented by Formula (1)]

The (poly)amine compound represented by the formula (0) is preferably a compound represented by the following formula (1) from the viewpoint of further excellent structure-forming ability.

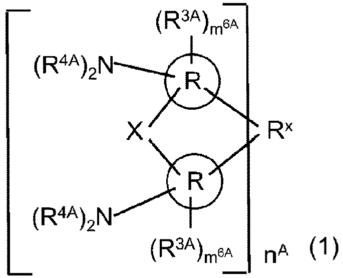

In the formula (1), each R^{3A} is independently any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, and a halogen atom (for example, a fluorine atom, a chlorine atom and a bromine atom); each R^{4A} is independently any of a hydrogen atom, a linear or branched, or cyclic alkyl group or acyl group having 1 to 30 carbon atoms and optionally having a substituent, an aryl group having 6 to 30 carbon atoms and optionally having a substituent, and a crosslinking group having 2 to 30 carbon atoms and optionally having a substituent, wherein when R^{4A} is any of the alkyl group or acyl group, the aryl group and the crosslinking group, R^{4A} optionally contains at least one bond selected from the group consisting of an ether bond, a ketone bond and an ester bond; each m^{6A} is independently an integer of 0 to 5; and R^{X}, X, R and n^{A} are as defined above.

### (1. R^{3A})

Each R^{3A} independently represents any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (for example, a fluorine atom, a chlorine atom and a bromine atom). Examples of the linear or branched, or cyclic alkyl group having 1 to 30 carbon atoms and optionally having a substituent include those exemplified as the alkyl group in (1-1-1a. R^{X1}). Examples of the aryl group having 6 to 30 carbon atoms and optionally having a substituent include those exemplified as the aryl group in (1-1-1a. R^{X1}). Examples of the crosslinking group having 2 to 30 carbon atoms and optionally having a substituent include those exemplified as the crosslinking group in (2. R^{1A}).

Among them, from the viewpoint of exhibiting effects of the present invention more effectively and certainly, R^{3A} is preferably a linear or branched alkyl group having 1 to 30 carbon atoms and optionally having a substituent and an aryl group having 6 to 30 carbon atoms and optionally having a substituent. The linear or branched alkyl group having 1 to 30 carbon atoms and optionally having a substituent is preferably an alkyl group exemplified as the preferable alkyl group optionally having a substituent in (2. R^{1A}) of [(Poly)amine Compound Represented by Formula (0)]. The aryl group having 6 to 30 carbon atoms and optionally having a substituent is preferably an aryl group exemplified as the preferable aryl group optionally having a substituent in (2. R^{1A}).

### (2. R^{4A})

Each R^{4A} independently represents any of a hydrogen atom, a linear or branched, or cyclic alkyl group or acyl group having 1 to 30 carbon atoms and optionally having a substituent, an aryl group having 6 to 30 carbon atoms and optionally having a substituent, and a crosslinking group having 2 to 30 carbon atoms and optionally having a substituent, wherein when R^{4A} is any of the alkyl group or acyl group, the aryl group and the crosslinking group, R^{4A} optionally contains at least one bond selected from the group consisting of an ether bond, a ketone bond and an ester bond. Examples of the linear or branched, or cyclic alkyl group having 1 to 30 carbon atoms and optionally having a substituent include those exemplified as the alkyl group in (1-1-1a. R^{X1}). Examples of the linear or branched, or cyclic acyl group having 1 to 30 carbon atoms and optionally having a substituent include groups represented by Rp-CO-, wherein Rp is an alkyl group exemplified as the alkyl group in (1-1-1a. R^{X1}). Examples of the aryl group having 6 to 30 carbon atoms and optionally having a substituent include those exemplified as the aryl group in (1-1-1a. R^{X1}). Examples of the crosslinking group having 2 to 30 carbon atoms and optionally having a substituent include those exemplified as the crosslinking group in (2. R^{1A}).

Among them, from the viewpoint of exhibiting effects of the present invention more effectively and certainly, it is preferably a hydrogen atom, a linear or branched alkyl group having 1 to 30 carbon atoms and optionally having a substituent, or the alkyl group containing a ketone bond (for example, an alkylcarbonyl group).

### (3. m^{6A})

Each m^{6A} is independently an integer of 0 to 5, and from the viewpoint of exhibiting effects of the present invention more effectively and certainly, it is preferably an integer of 1 to 3, and is more preferably an integer of 1 or 2.

### (4. X)

In the (poly)amine compound represented by the formula (1), it is preferable that X be an oxygen atom from the viewpoint of further excellent structure-forming ability. (Poly)amine compounds represented by the formula (1) in which X is an oxygen atom are represented by the following formula (0-2).

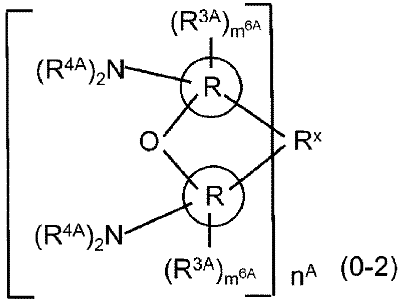

In the above formula (0-2), R^{X}, R, n^{A}, R^{3A}, R^{4A} and m^{6A} are as defined above.

### [(Poly)amine Compound Represented by Formula (2)]

The (poly)amine compound represented by the above formula (1) is more preferably a (poly)amine compound represented by the following formula (2) from the viewpoint of further improving solubility in an organic solvent.

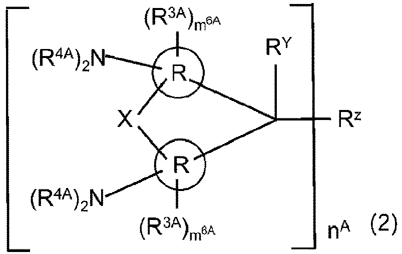

In the above formula (2), R^{Y} is any of a hydrogen atom, a linear or branched, or cyclic alkyl group having 1 to 30 carbon atoms and optionally having a substituent and an aryl group having 6 to 30 carbon atoms and optionally having a substituent; R^{Z} is an n^{A}-valent group having 1 to 60 carbon atoms or a single bond, and the total number of carbon atoms in R^{Y} and R^{Z} is 69 or less; and X, R, n^{A}, R^{3A}, R^{4A} and m^{6A} are as defined above.

### (1. R^{Y})

R^{Y} represents any of a hydrogen atom, a linear or branched, or cyclic alkyl group having 1 to 30 carbon atoms and optionally having a substituent and an aryl group having 6 to 30 carbon atoms and optionally having a substituent. Examples of the linear or branched, or cyclic alkyl group having 1 to 30 carbon atoms and optionally having a substituent include those exemplified as the alkyl group in (1-1-1a. R^{X1}), and examples of the alkyl group that is preferable from the viewpoint of exhibiting effects of the present invention more effectively and certainly include those exemplified as the preferable alkyl group in (1-1-1a. R^{X1}). Examples of the aryl group having 6 to 30 carbon atoms and optionally having a substituent include those exemplified as the aryl group in (1-1-1a. R^{X1}), and examples of the aryl group that is preferable from the viewpoint of exhibiting effects of the present invention more effectively and certainly include those exemplified as the preferable aryl group in (1-1-1a. R^{X1}). Among them, R^{Y} is preferably a hydrogen atom or an aryl group having 6 to 30 carbon atoms and optionally having a substituent, and is more preferably a hydrogen atom or a phenyl group.

### (2. R^{Z})

From the viewpoint of exhibiting effects of the present invention more effectively and certainly, R^{Z} is preferably an n^{A}-valent group having 1 to 60 carbon atoms. Examples of the n^{A}-valent group having 1 to 60 carbon atoms include n^{A}-valent groups exemplified in (1-1-1b. R^{X2}), and examples of the preferable group include n^{A}-valent groups exemplified as the preferable n^{A}-valent group in (1-1-1b. R^{X2}).

### (3. X)

In the (poly)amine compound represented by the formula (2), it is preferable that X be an oxygen atom from the viewpoint of further excellent solubility in an organic solvent. (Poly)amine compounds represented by the formula (2) in which X is an oxygen atom are represented by the following formula (0-3).

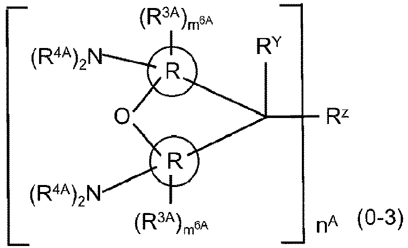

In the above formula (0-3), R, n^{A}, R^{3A}, R^{4A}, m^{6A}, R^{Y} and R^{Z} are as defined above.

### [(Poly)amine Compound Represented by Formula (3)]

The (poly)amine compound represented by the formula (2) is further preferably a (poly)amine compound represented by the following formula (3) from the viewpoint of further improving solubility in an organic solvent and further improving heat resistance and refractive index.

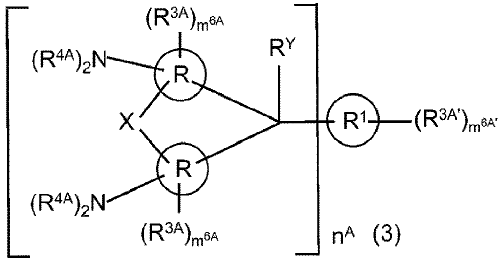

In the above formula (3), R^{3A'} is any of a linear or branched, or cyclic alkyl group or alkoxy group having 1 to 30 carbon atoms and optionally having a substituent, an amino group optionally having a substituent, an aryl group having 6 to 30 carbon atoms and optionally having a substituent, a crosslinking group having 2 to 30 carbon atoms and optionally having a substituent, a hydroxy group and a cyano group; each R¹ independently represents any of a benzene ring, a biphenyl ring, a naphthalene ring, an anthracene ring, and a heterocyclic ring containing any of an oxygen atom, a sulfur atom and a nitrogen atom as a heteroatom (for example, a furan ring, an imidazole ring, a carbazole ring, a chromene ring, a pyrrole ring, a thiophene ring, an oxazole ring, a thiazole ring, a pyridine ring, a pyrazine ring, a pyrimidine ring, a pyridazine ring, an indolizine ring, an indole ring, a benzofuran ring, a benzothiophene ring, an isobenzofuran ring, a quinolizine ring, a quinoline ring, a phthalazine ring, a naphthyridine ring, a quinoxaline ring, a quinoxazoline ring, an isoquinoline ring, a phenanthridine ring, an acridine ring, a phenanthroline ring, a thianthrene ring, a xanthene ring, a phenoxathiin ring, a phenothiazine ring and a phenazine ring) ; m^{6A'} is an integer of 0 to 5; X, R, n^{A}, R^{3A}, R^{4A}, m^{6A} and R^{Y} are as defined above; and when R¹ is a benzene ring, R^{3A'} is not a phenyl ring.

R^{3A'} is any of a linear or branched, or cyclic alkyl group or alkoxy group having 1 to 30 carbon atoms and optionally having a substituent, an aryl group having 6 to 30 carbon atoms and optionally having a substituent, a crosslinking group having 2 to 30 carbon atoms and optionally having a substituent, a hydroxy group and a cyano group, and when R¹ is a benzene ring or a naphthalene ring, R^{3A'} is any of a linear or branched, or cyclic alkyl group or alkoxy group having 1 to 30 carbon atoms and optionally having a substituent, an aryl group having 6 to 30 carbon atoms and optionally having a substituent, a crosslinking group having 2 to 30 carbon atoms and optionally having a substituent, a hydroxy group and a cyano group.

### (1. R^{3A'})

Each R^{3A'} independently represents any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (for example, a fluorine atom, a chlorine atom and a bromine atom). Examples of the linear or branched, or cyclic alkyl group having 1 to 30 carbon atoms and optionally having a substituent include those exemplified as the alkyl group in (1-1-1a. R^{X1}). Examples of the aryl group having 6 to 30 carbon atoms and optionally having a substituent include those exemplified as the aryl group in (1-1-1a. R^{X1}). Examples of the crosslinking group having 2 to 30 carbon atoms and optionally having a substituent include those exemplified as the crosslinking group in (2. R^{1A}).

Among them, from the viewpoint of exhibiting effects of the present invention more effectively and certainly, R^{3A} is preferably a linear or branched alkyl group having 1 to 30 carbon atoms and optionally having a substituent and an aryl group having 6 to 30 carbon atoms and optionally having a substituent. The linear or branched alkyl group having 1 to 30 carbon atoms and optionally having a substituent is preferably an alkyl group exemplified as the preferable alkyl group optionally having a substituent in (2. R^{1A}) of [(Poly)amine Compound Represented by Formula (0)]. The aryl group having 6 to 30 carbon atoms and optionally having a substituent is preferably an aryl group exemplified as the preferable aryl group optionally having a substituent in (2. R^{1A}).

### (2. m^{6A'})

m^{6A'} is an integer of 0 to 5, and from the viewpoint of exhibiting effects of the present invention more effectively and certainly, it is preferably an integer of 1 to 3, and is more preferably an integer of 1 or 2.

### (3. R¹)

Each R¹ independently represents any of a benzene ring, a naphthalene ring and an anthracene ring. In the above formula (0), when R¹ is a benzene ring, it is shown that the benzene ring is substituted with one or more R^{3A'}s, and the substitution position for each R^{3A'} to the benzene ring is not particularly limited. In the above formula (0), when R¹ is a naphthalene ring, it is shown that the naphthalene ring is substituted with one or more R^{3A'}s, and the substitution position for each R^{3A'} to the naphthalene ring is not particularly limited. In the above formula (0), when R is an anthracene ring, it is shown that the anthracene ring is substituted with one or more R^{3A'}s, and the substitution position for each R^{3A'} to the anthracene ring is not particularly limited. From the viewpoint of exhibiting effects of the present invention more effectively and certainly, R¹ is preferably a benzene ring or a naphthalene ring, and is more preferably a benzene ring.

In the (poly)amine compound represented by the formula (3), from the viewpoint of exhibiting effects of the present invention more effectively and certainly, it is preferable that R^{3A}, R^{4A}, m^{6A}, R, X, R^{Y}, R^{3A'}, m^{6A'} and R¹ all satisfy the following conditions (i) to (ix) simultaneously:
(i) each R^{3A} is independently any of an alkyl group (particularly, a methyl group and an ethyl group), a haloalkyl group (particularly, a trichloromethyl group and a trifluoromethyl group), a hydroxy group, a phenyl group and a phenyl group substituted with an amino group (particularly, an aminophenyl group);
(ii) each R^{4A} independently represents any of a hydrogen atom, an alkyl group and an alkylcarbonyl group;
(iii) m^{6A} is 0 or 1;
(iv) R is a benzene ring or a naphthalene ring;
(v) X represents an oxygen atom, or is absent;
(vi) R^{Y} represents a methyl group or a phenyl group;
(vii) R^{3A'} represents any of a methyl group, an ethyl group, a n-propyl group, a n-butyl group, a cyclohexyl group and a hydroxy group;
(viii) m^{6A'} is 0 or 1; and
(ix) R¹ is a benzene ring.

### [Compound Represented by Formula (0a) or (0b)]

The (poly)amine compound represented by the formula (3) is preferably a compound represented by the following formula (0a), and is more preferably a compound represented by the following formula (0b) from the viewpoint of being capable of further improving solubility and heat resistance.

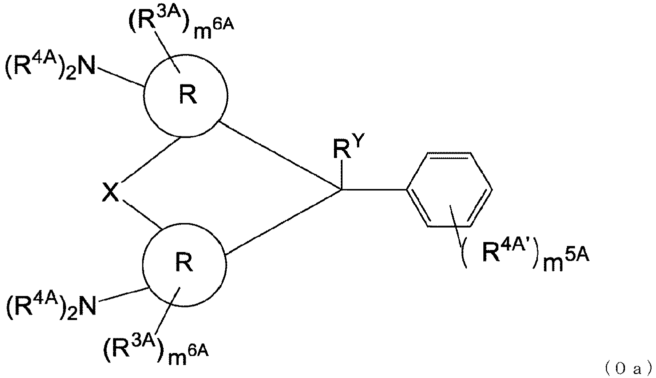

In the above formula (0a), X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom). m^{5A} represents an integer of 1 to 5.

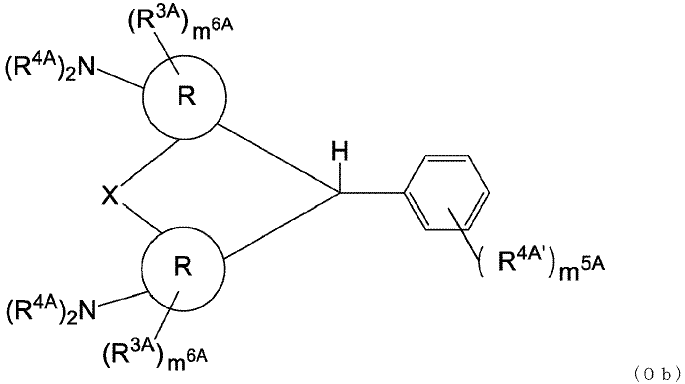

In the above formula (0a), X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom). m^{5A} represents an integer of 1 to 5.

### [Compound Represented by Formula (0a-1) or (0b-1)]

The (poly)amine compound represented by the formula (3) is preferably a compound represented by the following formula (0a-1), and is more preferably a compound represented by the following formula (0b-1) from the viewpoint of being capable of further improving solubility.

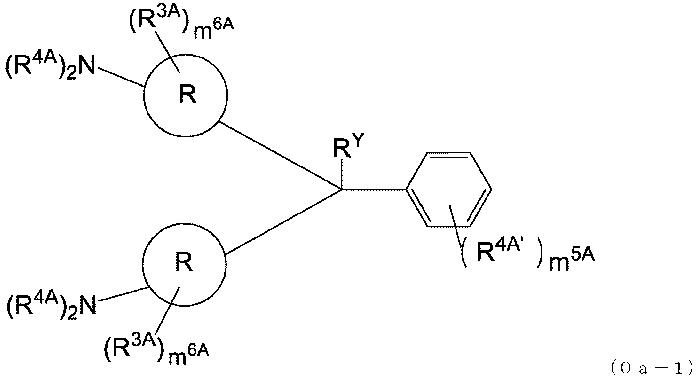

In the above formula (0a-1), R, R^{3A}, R^{4A}, m^{6A}, R^{4A'}, m^{5A} and R^{Y} are as defined above.

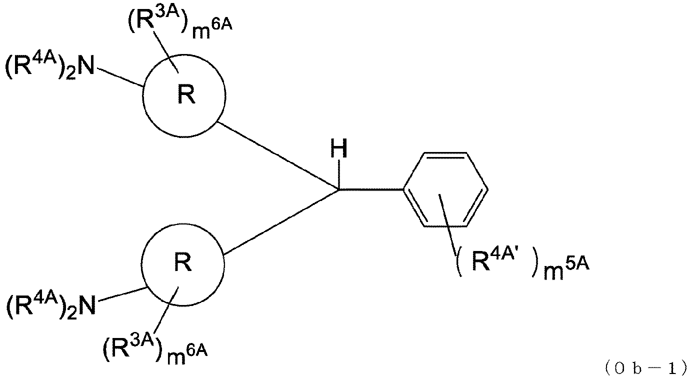

In the above formula (0b-1), R, R^{3A}, R^{4A}, m^{6A}, m^{5A} and R^{Y} are as defined above.

### [Compound Represented by Formula (0b-2)]

The (poly)amine compound represented by the formula (0b-1) is further preferably a compound represented by the following formula (0b-2) from the viewpoint of being capable of further improving flowability upon formation of a film.

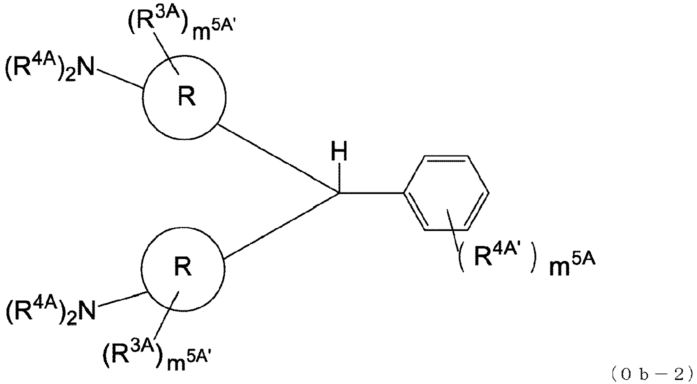

In the above formula (0b-2), R, R^{3A}, R^{4A}, R^{4A'}, m^{5A} and R^{Y} are as defined above, and each m^{5A'} is independently an integer of 1 to 5.

### [Compound Represented by Formula (0a-2)]

The (poly)amine compound represented by the formula (0a-1) is further preferably a compound represented by the following formula (0a-2) from the viewpoint of being capable of further improving solubility.

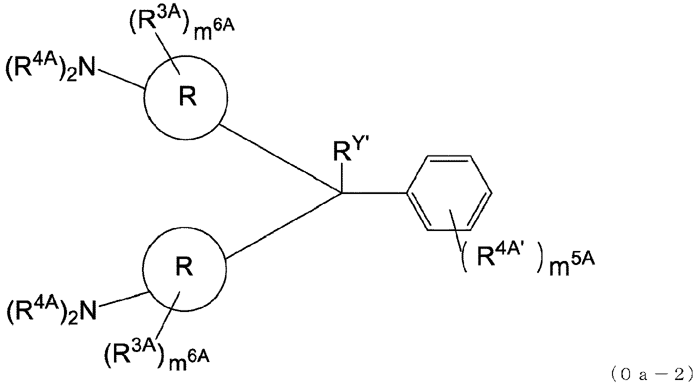

In the above formula (0b-1), R, R^{3A}, R^{4A}, m^{6A}, m^{5A} and R^{Y} are as defined above, and R^{Y'} is any of a linear or branched, or cyclic alkyl group having 1 to 30 carbon atoms and optionally having a substituent and an aryl group having 6 to 30 carbon atoms and optionally having a substituent.

### [Compound Represented by Formula (0b-3)]

The (poly)amine compound represented by the formula (0b-1) is further preferably a compound represented by the following formula (0b-3) from the viewpoint of being capable of further improving flowability upon formation of a film.

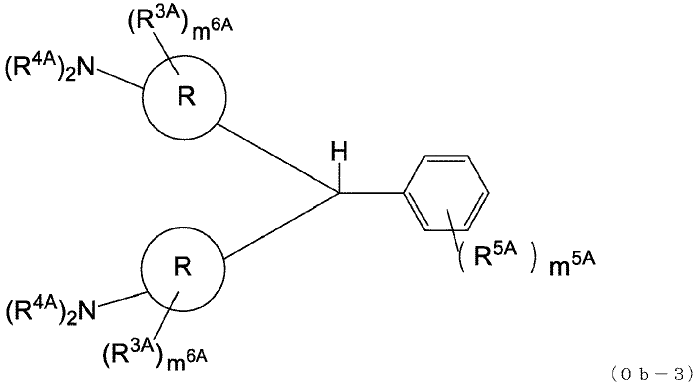

In the above formula (0b-3), R, R^{3A}, R^{4A}, R^{4A'} and m^{5A} are as defined above, and R^{5A} represents a linear or branched, or cyclic alkyl group having 5 or more carbon atoms and optionally having a substituent.

### [Compound Represented by Formula (1a) or (1b)]

The (poly)amine compound represented by the formula (3) is preferably a compound represented by the following formula (1a), and is more preferably a compound represented by the following formula (1b) from the viewpoint of further excellent supply of raw materials and being capable of further improving solubility and heat resistance.

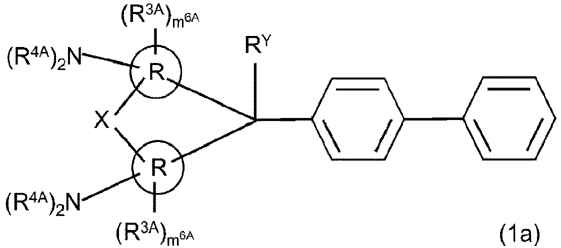

In the above formula (1a), X, R, R^{3A}, R^{4A}, m^{6A} and R^{Y} are as defined above.

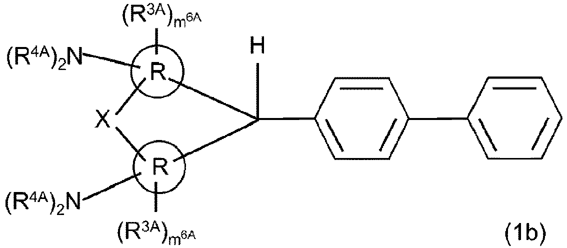

In the above formula (1b), X, R, R^{3A}, R^{4A} and m^{6A} are as defined above.

### [Compound Represented by Formula (3a) or (3b)]

The (poly)amine compound represented by the formula (3) is preferably a compound represented by the following formula (3a), and is more preferably a compound represented by the following formula (3b) from the viewpoint of being capable of further improving heat resistance. 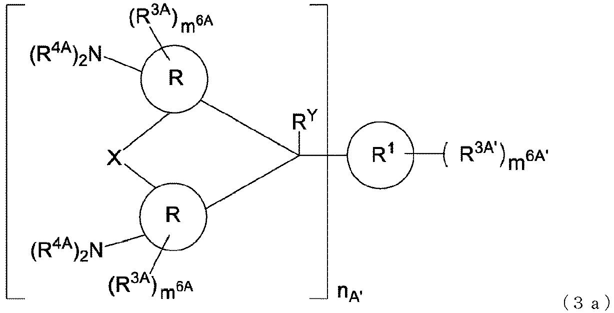 In the above formula (3a), R, X, R^{3A}, R^{4A}, m^{6A}, R^{Y}, R1 and m^{6A'} are as defined above, and n^{A'} is an integer of 2 to 4. 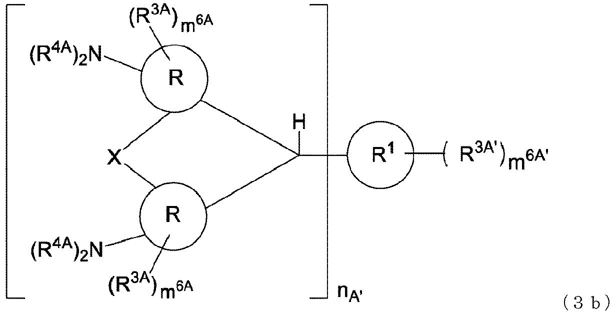 In the above formula (3b), R, X, R^{3A}, R^{4A}, m^{6A}, R1 and m^{6A'} are as defined above, and n^{A'} is an integer of 2 to 4.

In the compound represented by the above formula (3), it is preferable that X be an oxygen atom from the viewpoint of further improving solubility in an organic solvent and further improving heat resistance and refractive index. (Poly)amine compounds represented by the formula (3) in which X is an oxygen atom are represented by compounds represented by the following formula (0-4).

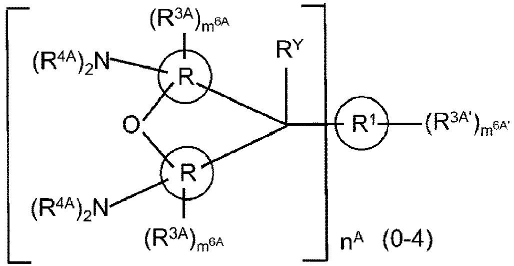

In the above formula (0-4), R, n^{A}, R^{3A}, R^{4A}, m^{6A}, R^{Y}, R1 and m^{6A'} are as defined above.

In addition, the compound represented by the above formula (0-4) is preferably a compound represented by the following formula (1a-1), and is more preferably a compound represented by the following formula (1b-1) from the viewpoint of excellent supply of raw materials and being capable of further improving solubility and heat resistance.

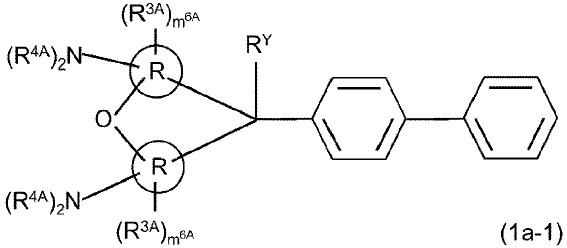

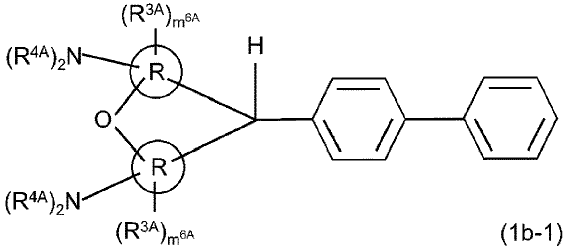

In the above formula (1a-1), R, R^{3A}, R^{4A}, m^{6A} and R^{Y} are as defined above.

In the above formula (1b-1), R, R^{3A}, R^{4A} and m^{6A} are as defined above.

In the representative (poly)amine compound according to the present embodiment, it is preferable that R^{4A} in the above formulas be a hydrogen atom from the viewpoint of improving curing performance due to a reactive functional group.

Examples of the representative (poly)amine compound according to the present embodiment include (poly)amine compounds represented by formulas (p1) to (p55) and (p61) to (p509).

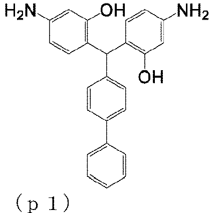

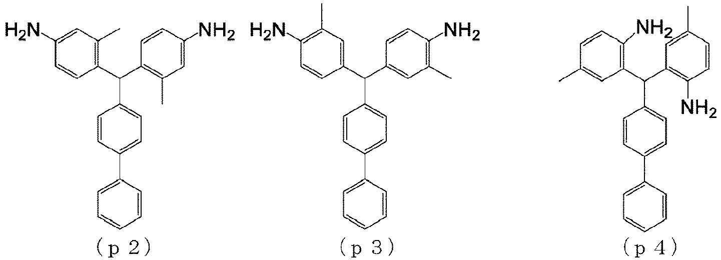

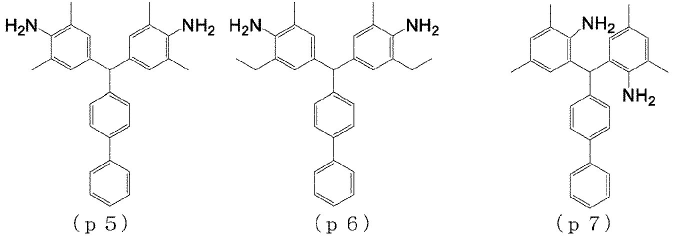

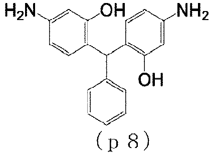

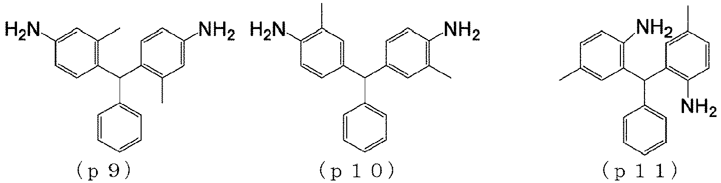

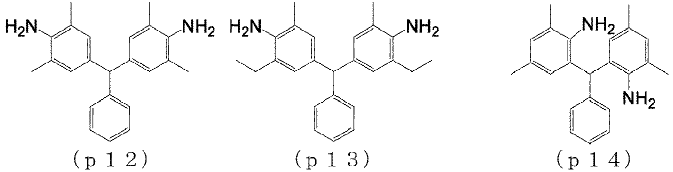

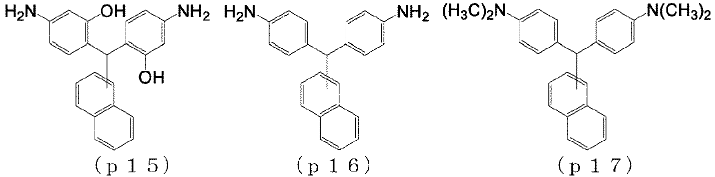

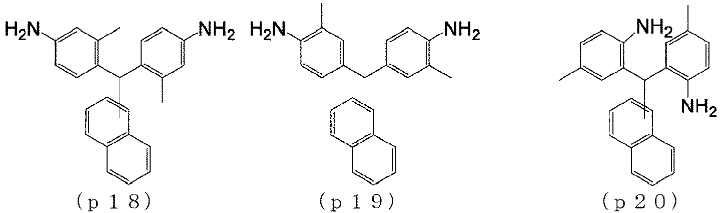

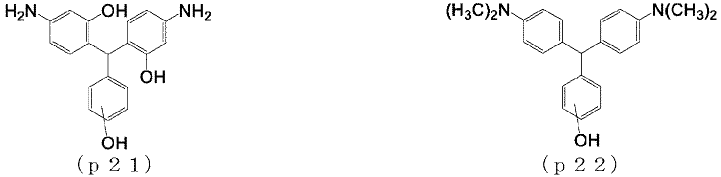

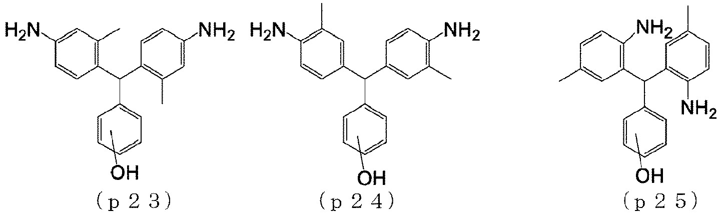

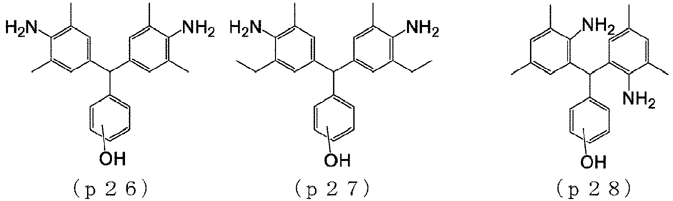

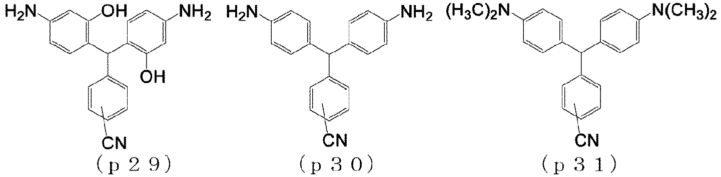

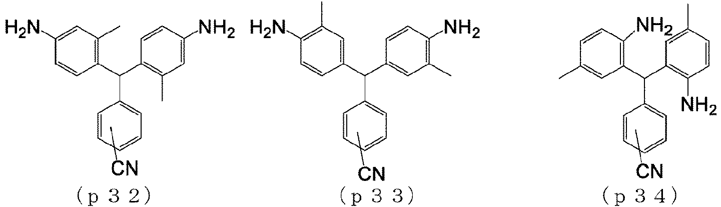

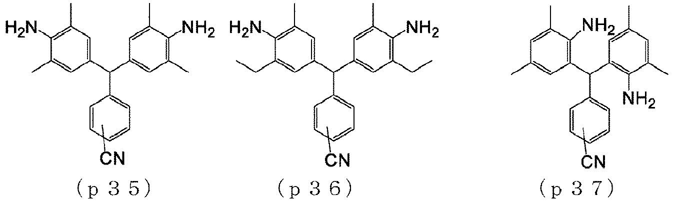

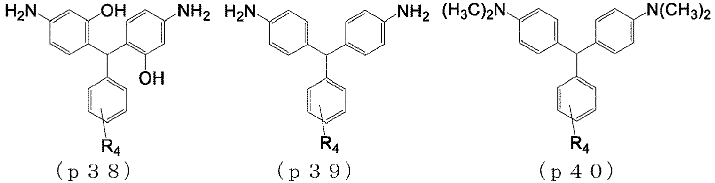

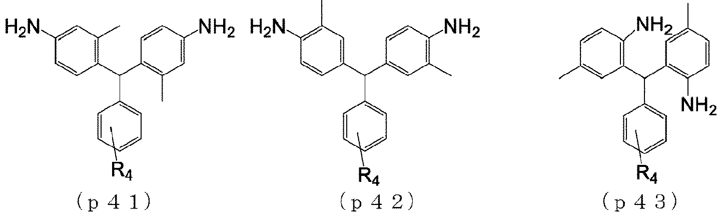

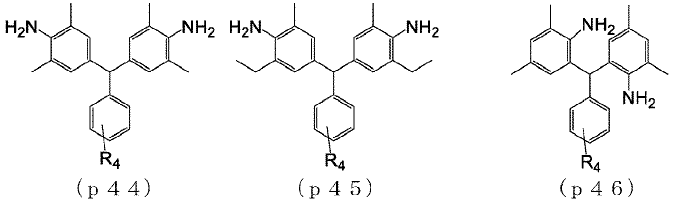

Examples of R₄ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₄ may be 1 or more.

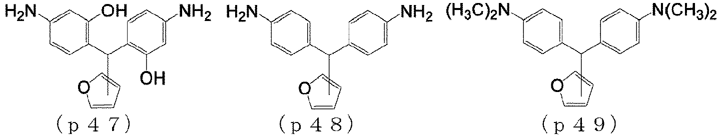

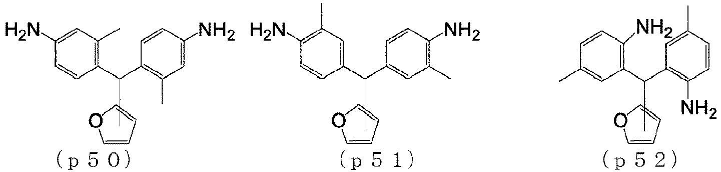

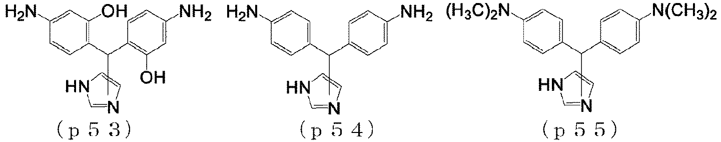

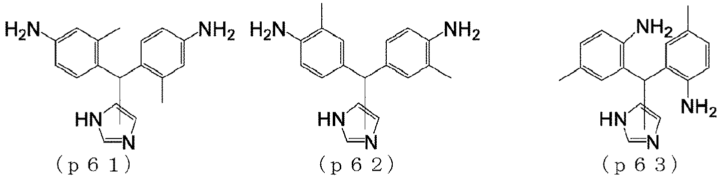

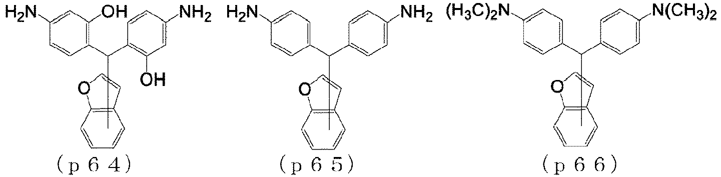

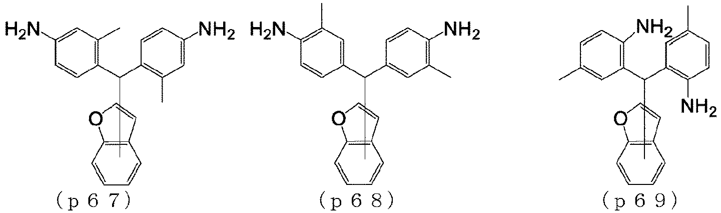

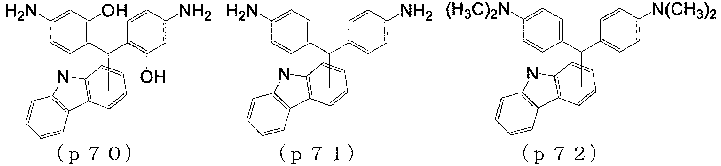

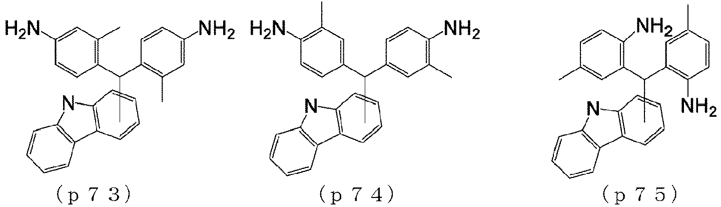

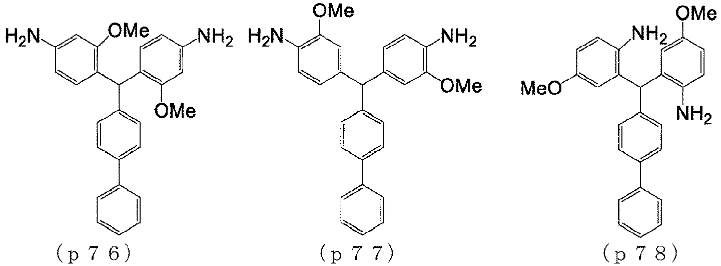

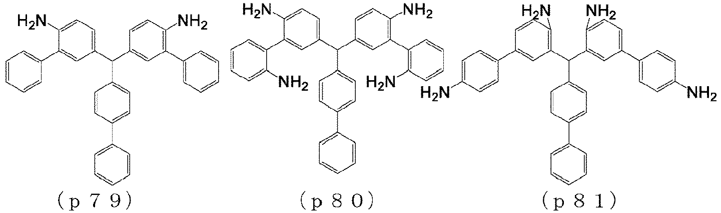

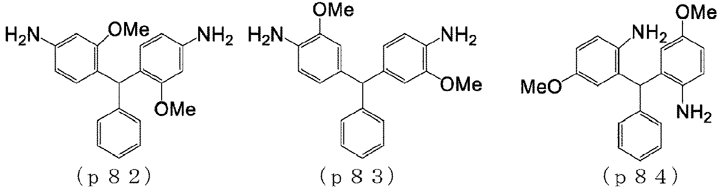

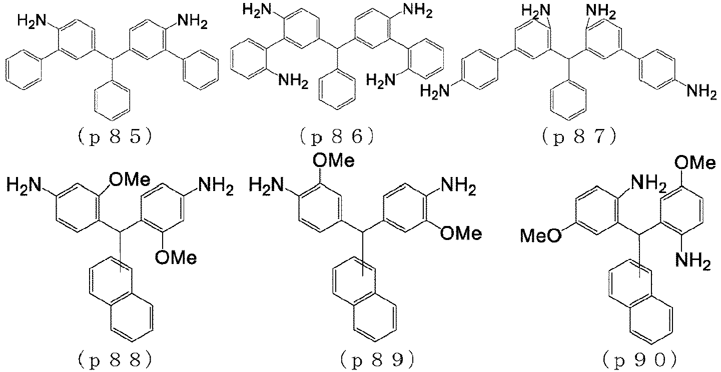

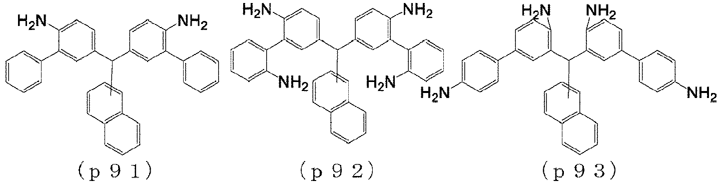

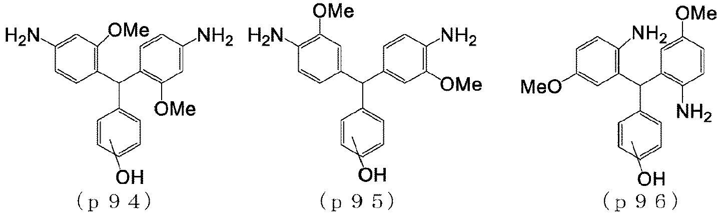

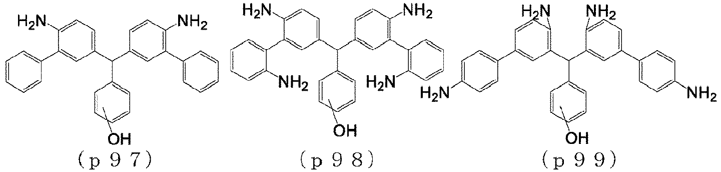

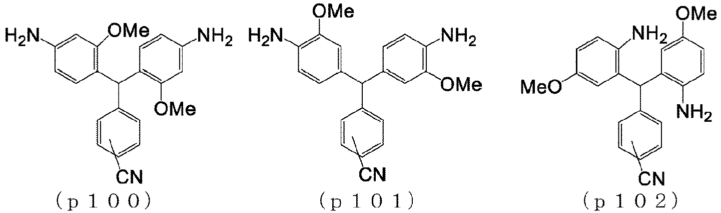

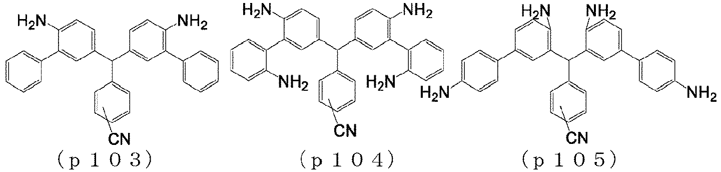

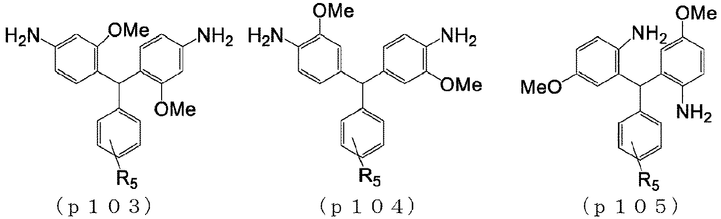

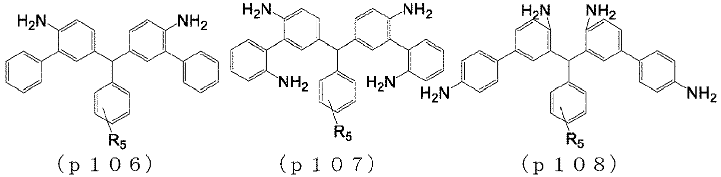

Examples of R₅ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₅ may be 1 or more.

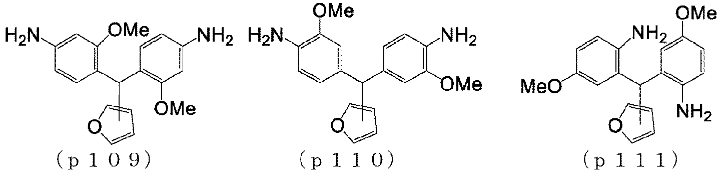

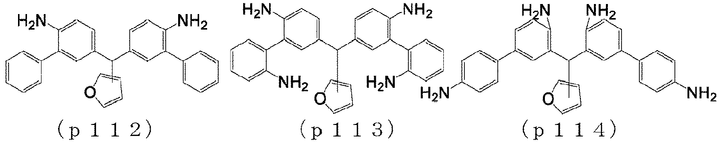

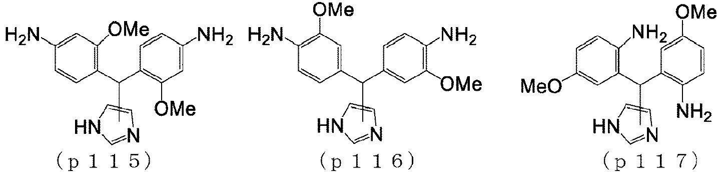

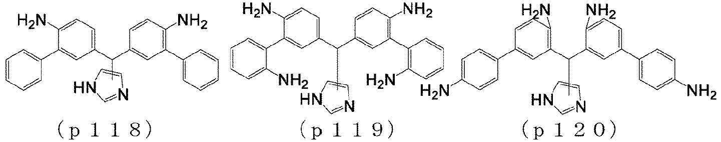

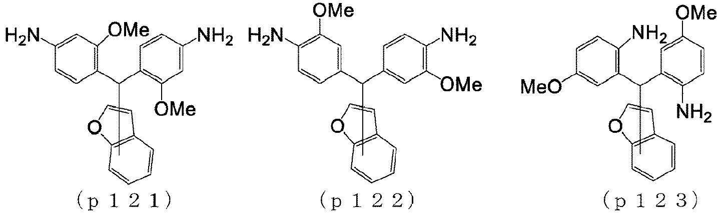

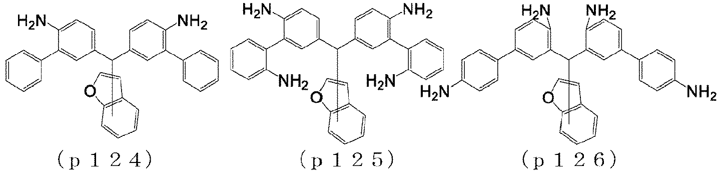

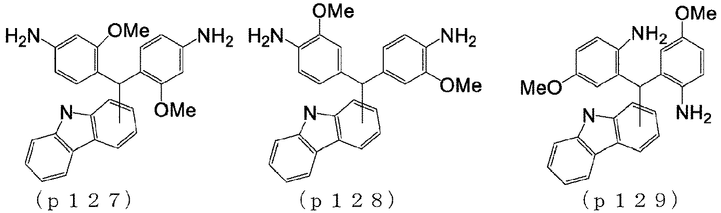

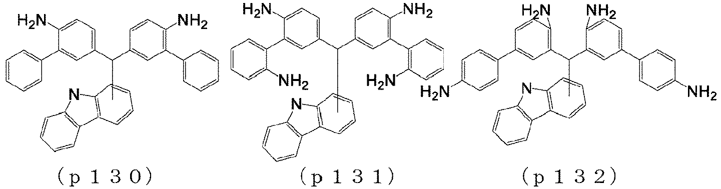

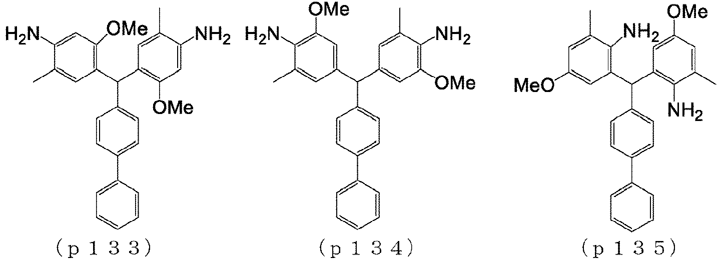

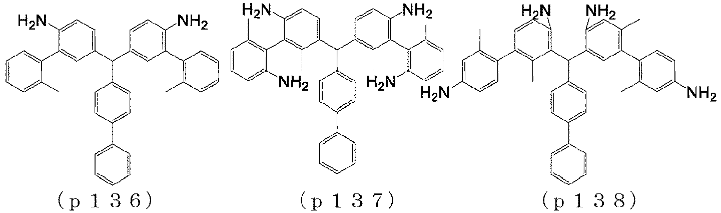

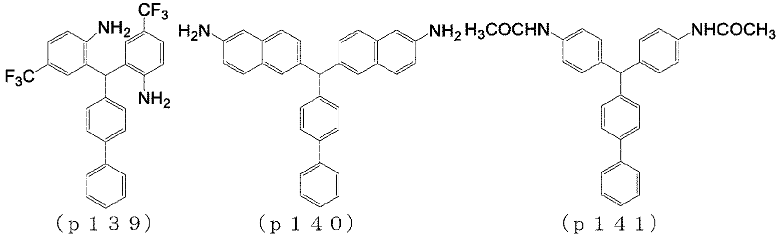

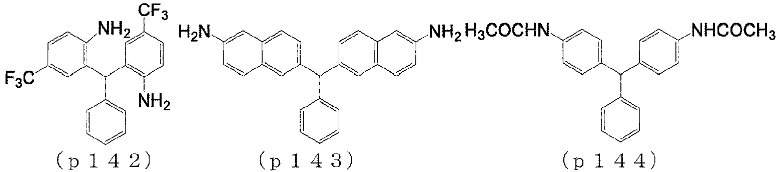

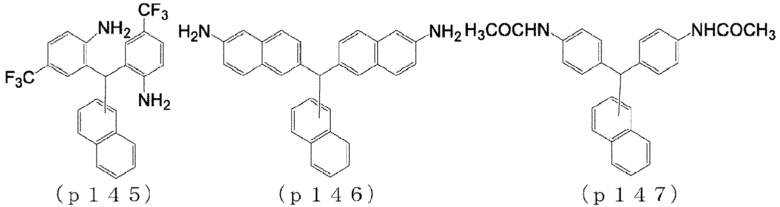

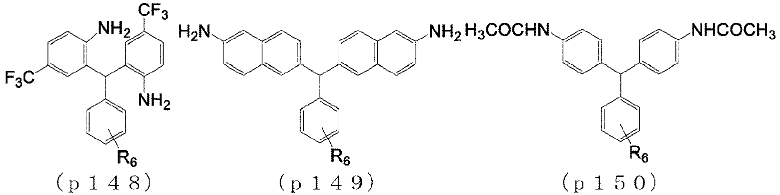

Examples of R₆ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₆ may be 1 or more.

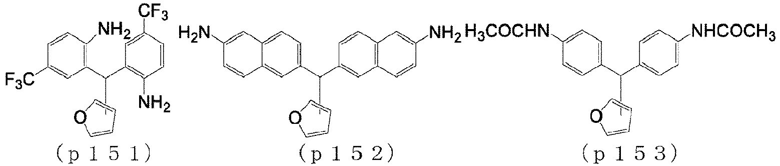

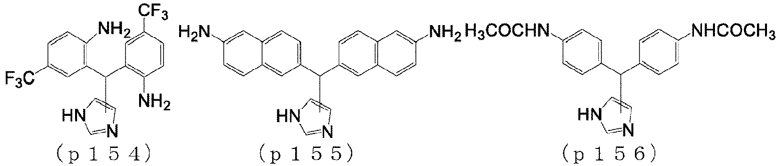

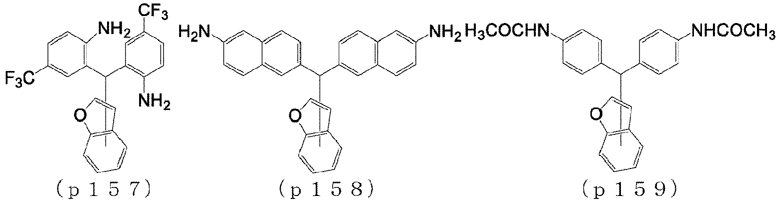

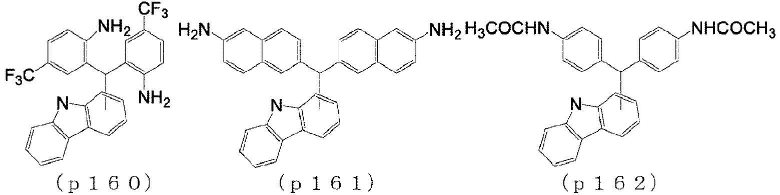

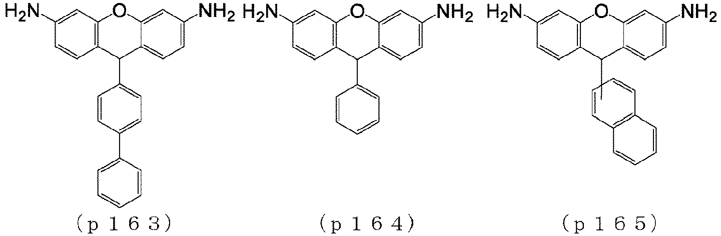

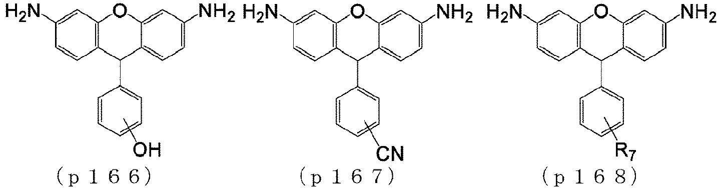

Examples of R₇ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₇ may be 1 or more.

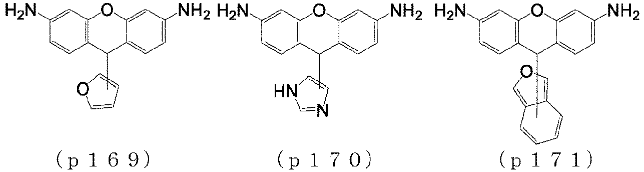

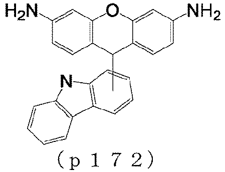

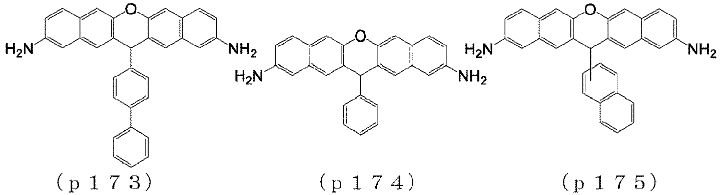

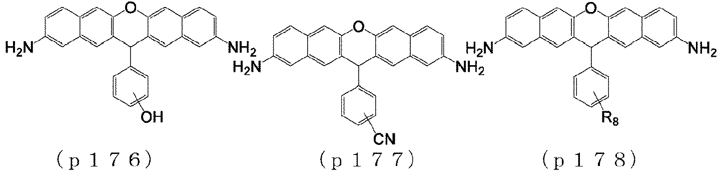

Examples of R₈ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₈ may be 1 or more.

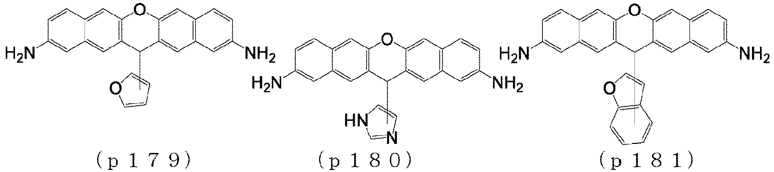

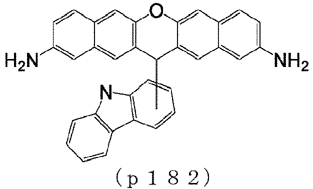

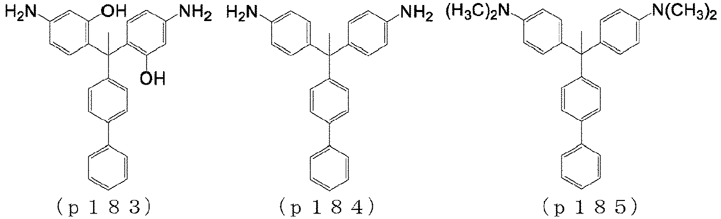

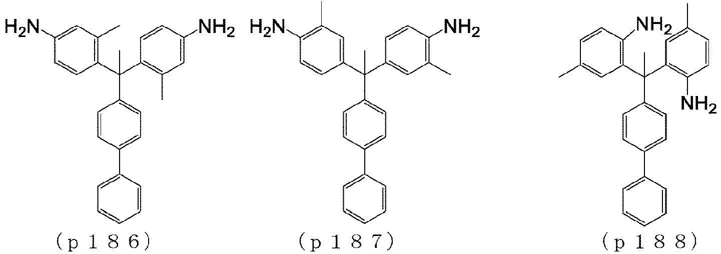

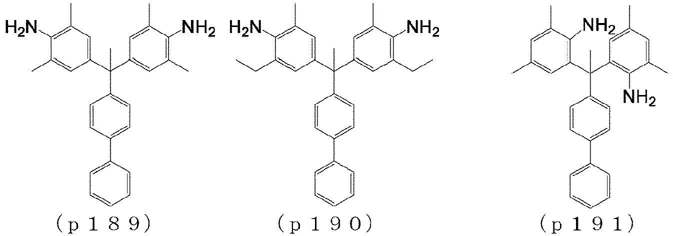

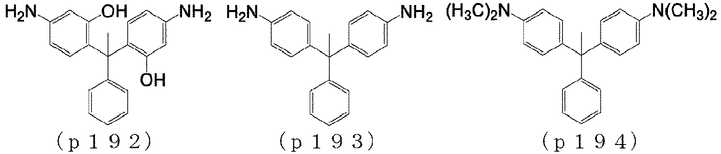

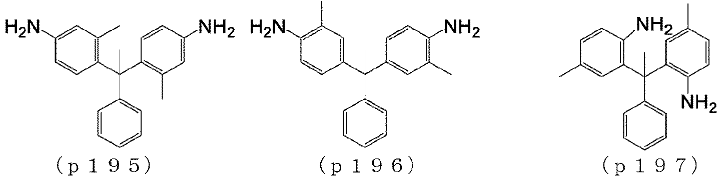

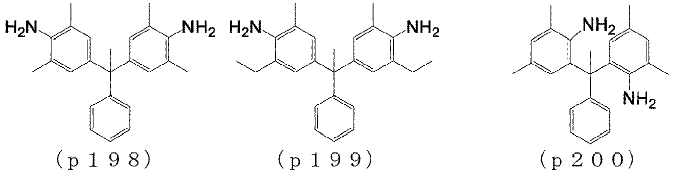

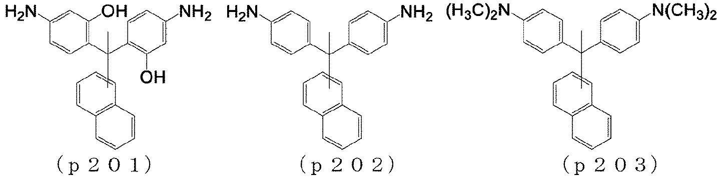

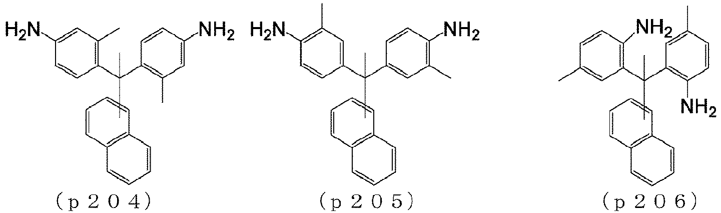

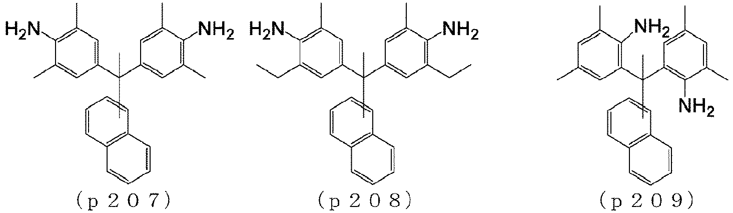

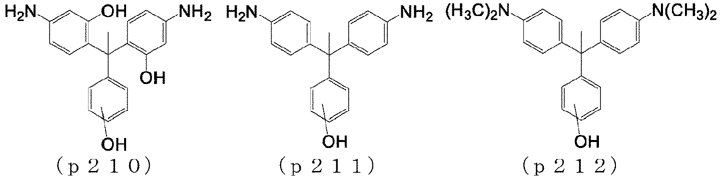

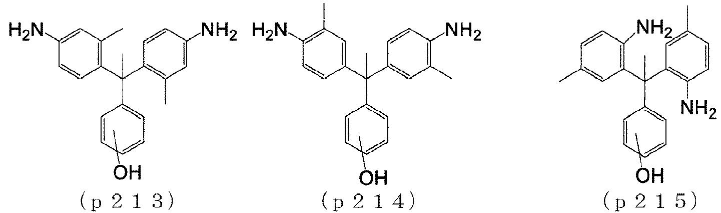

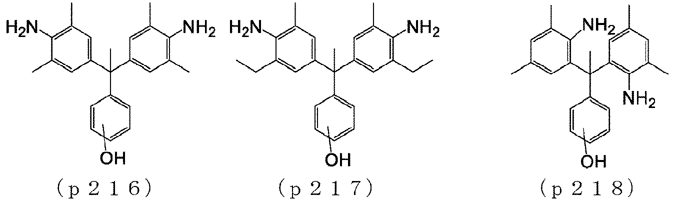

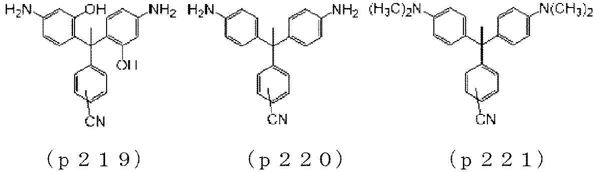

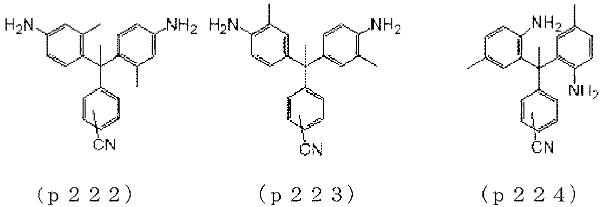

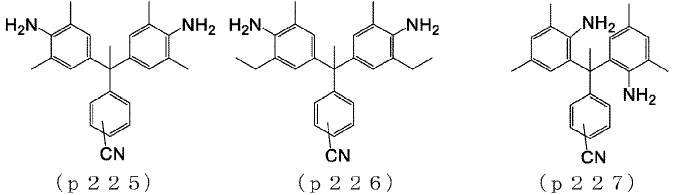

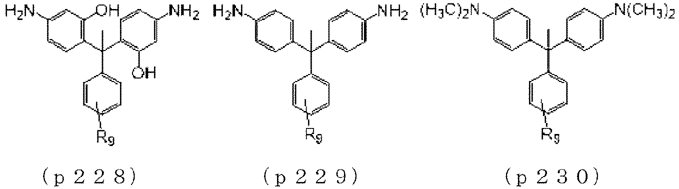

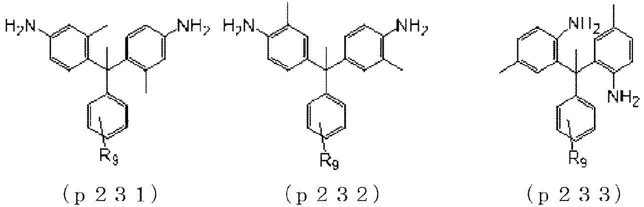

Examples of R₉ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₉ may be 1 or more.

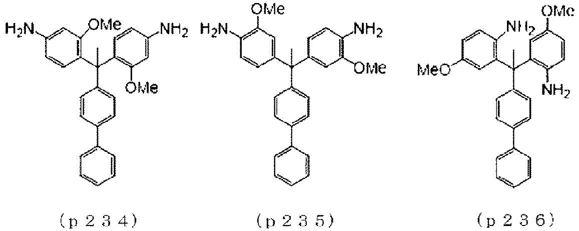

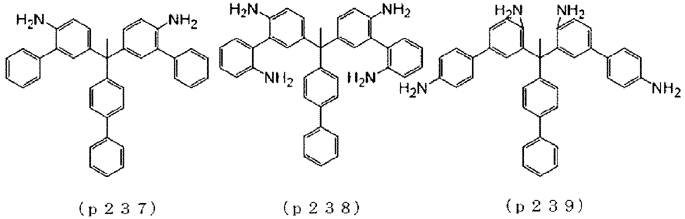

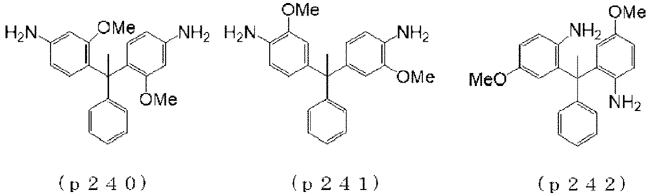

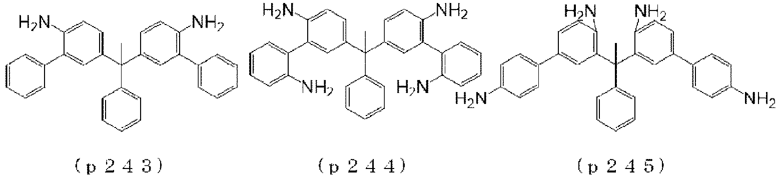

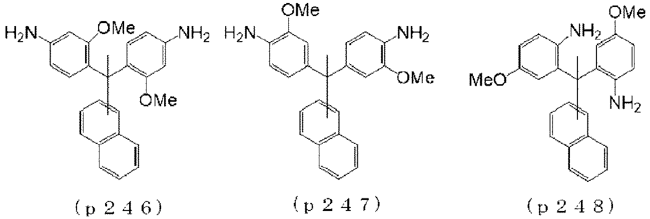

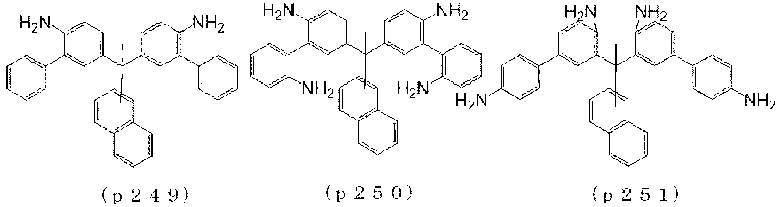

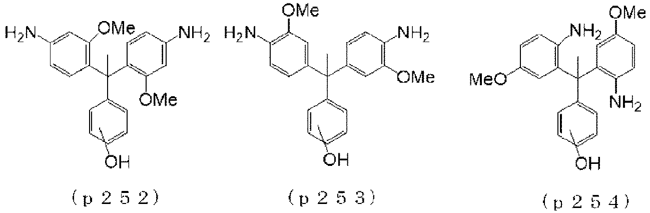

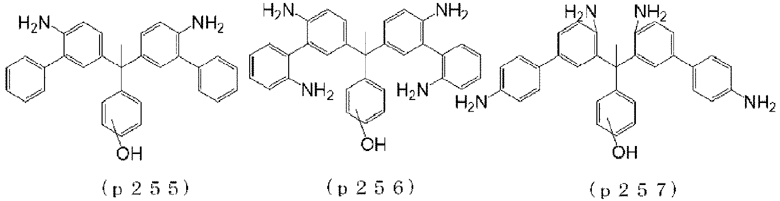

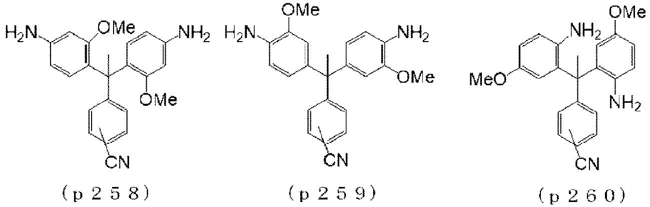

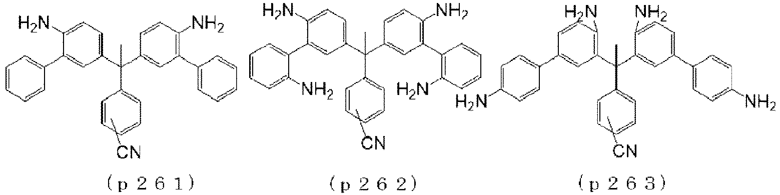

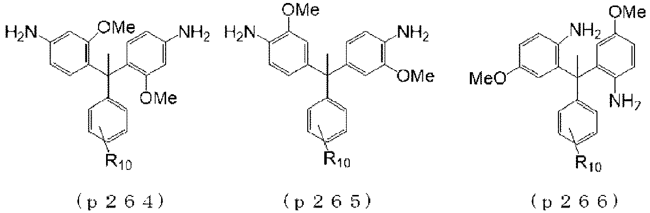

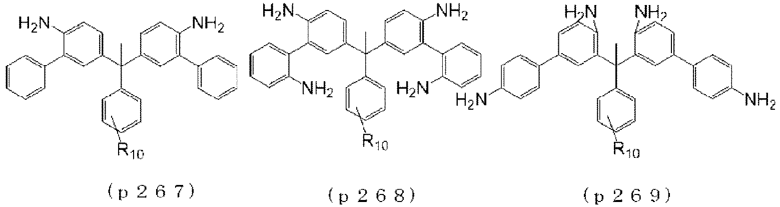

Examples of R₁₀ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₀ may be 1 or more.

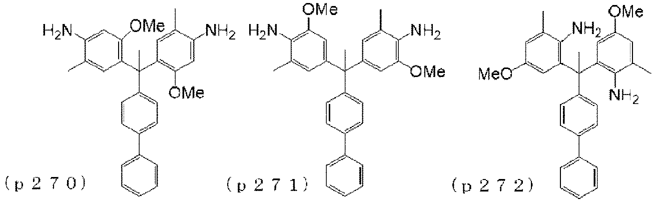

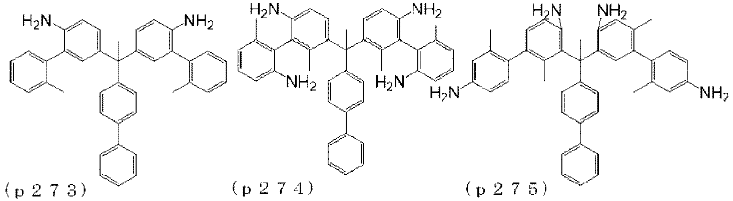

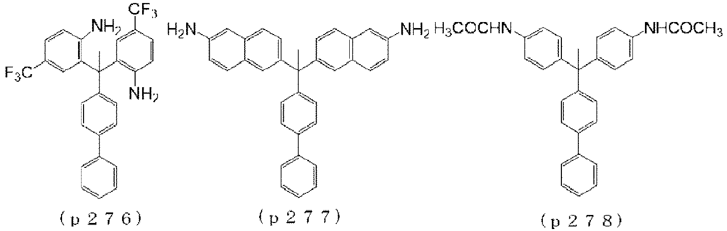

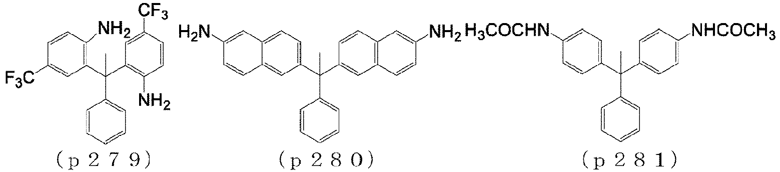

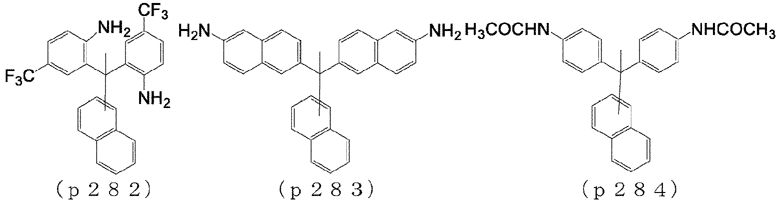

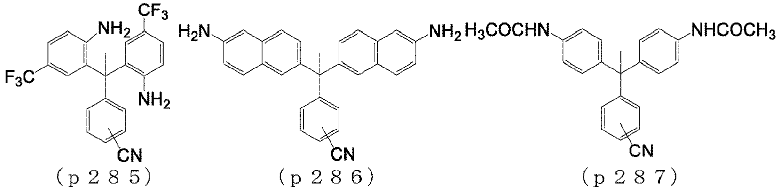

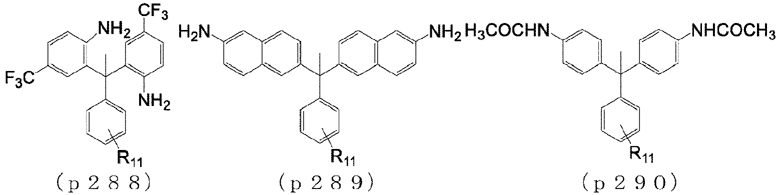

Examples of R₁₁ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₁ may be 1 or more.

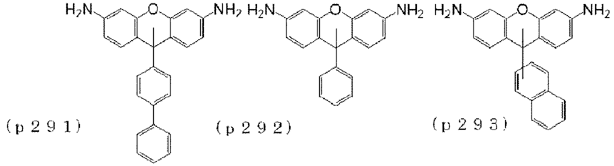

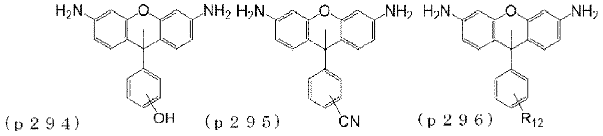

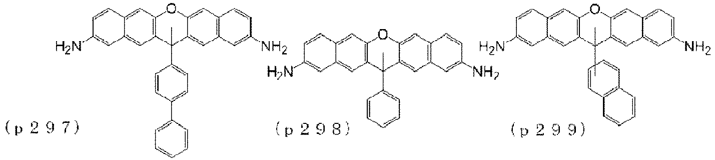

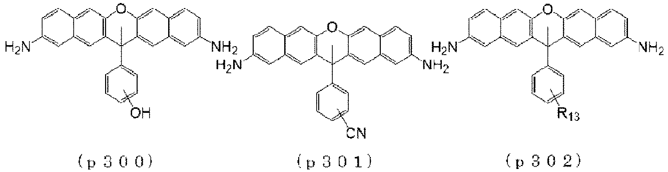

Examples of R₁₂ or R₁₃ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₂ or R₁₃ may be 1 or more.

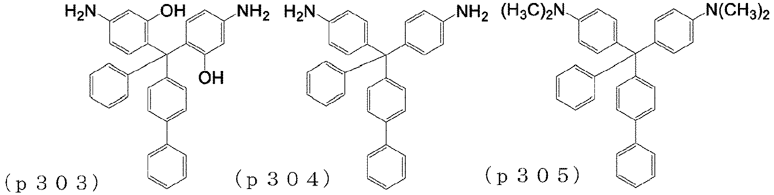

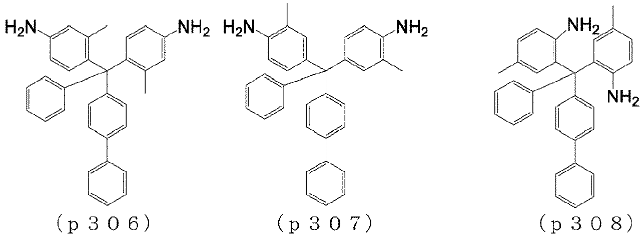

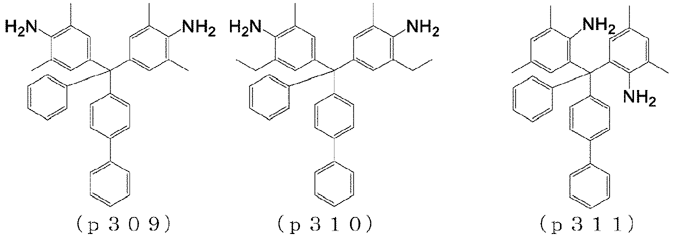

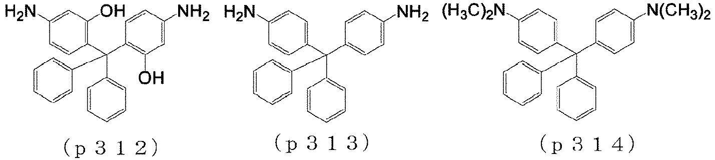

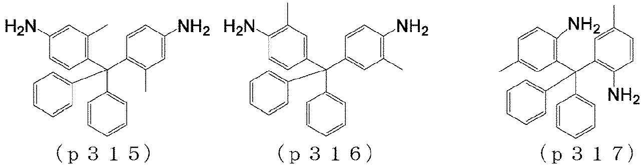

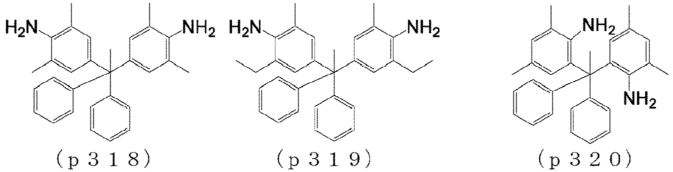

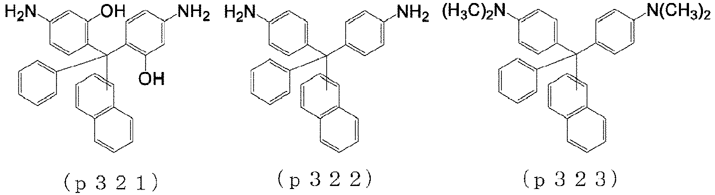

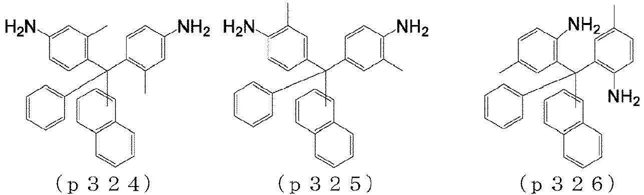

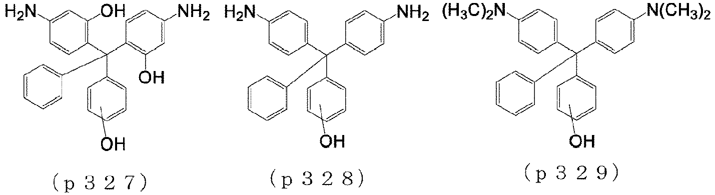

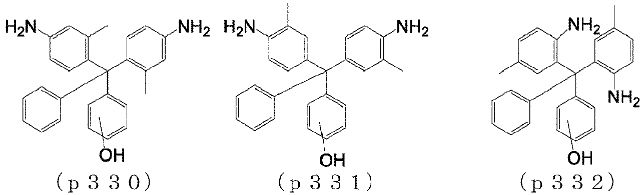

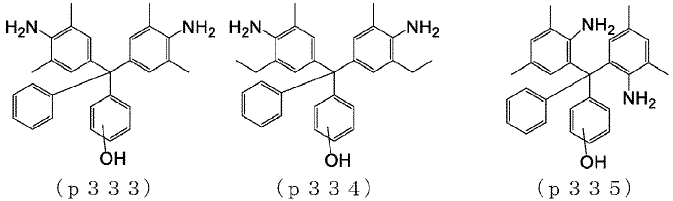

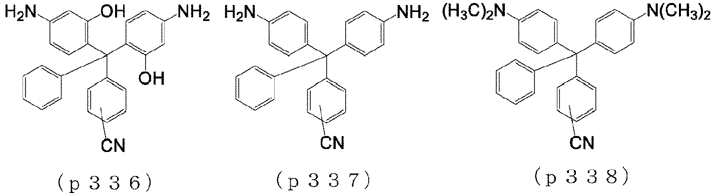

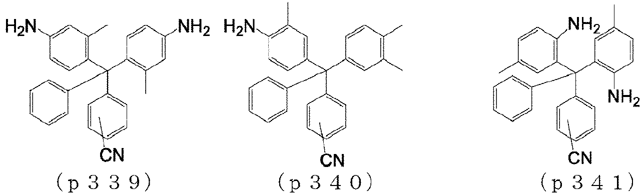

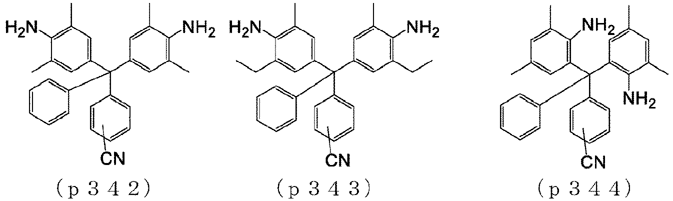

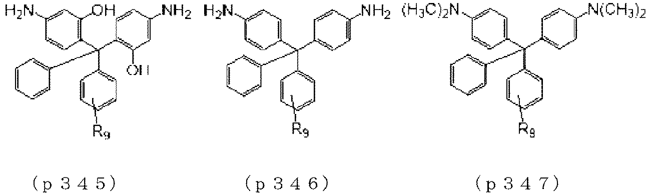

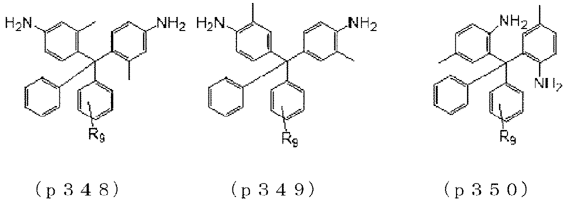

Examples of R₉ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₉ may be 1 or more. 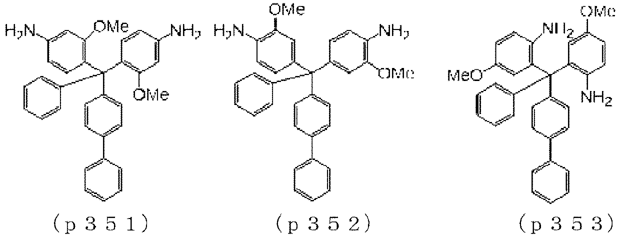 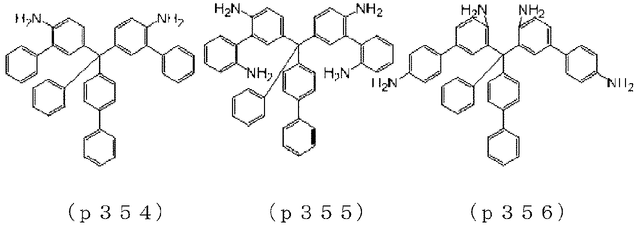 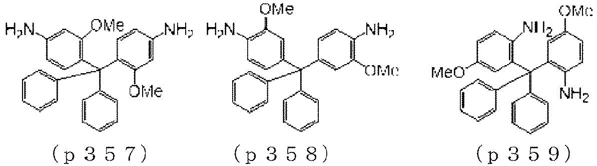 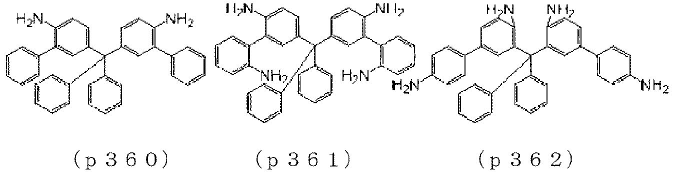 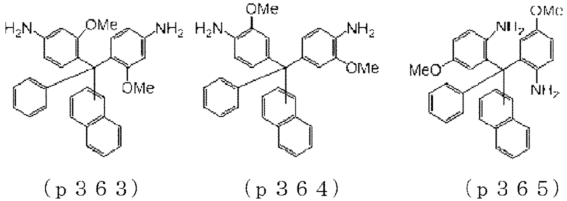 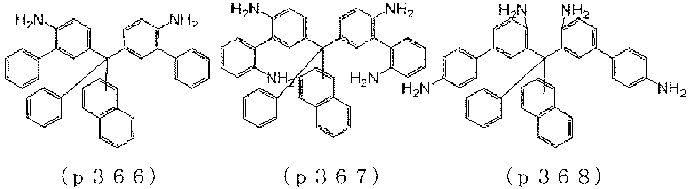 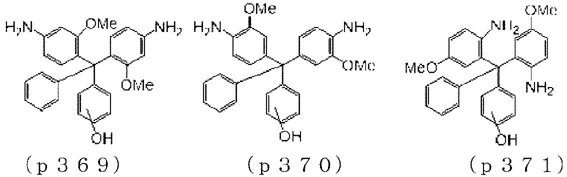 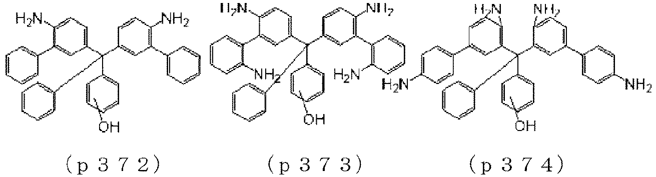 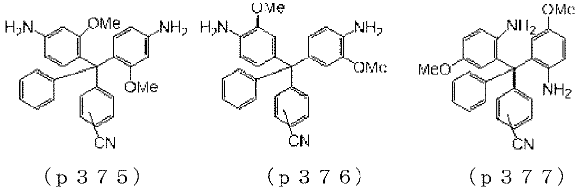 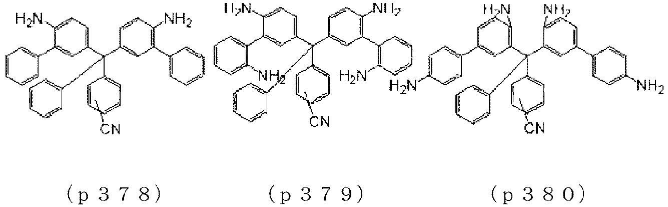 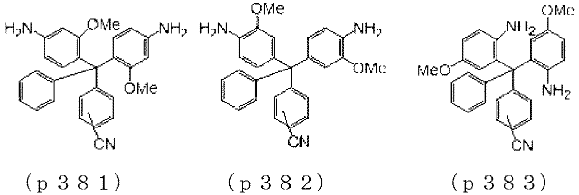 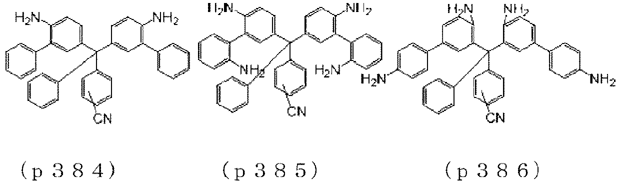 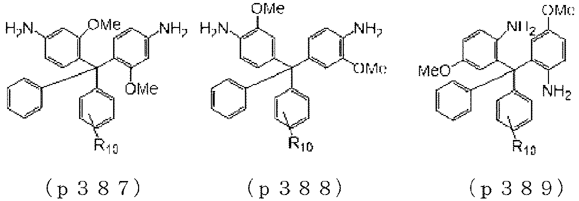 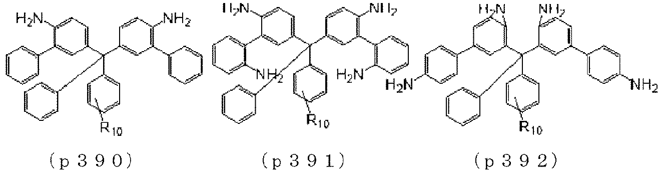 Examples of R₁₀ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₀ may be 1 or more.

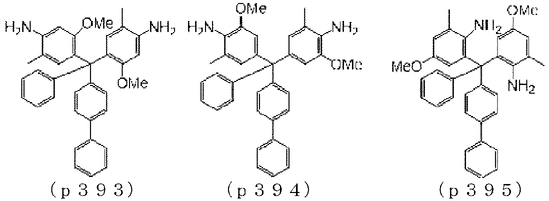

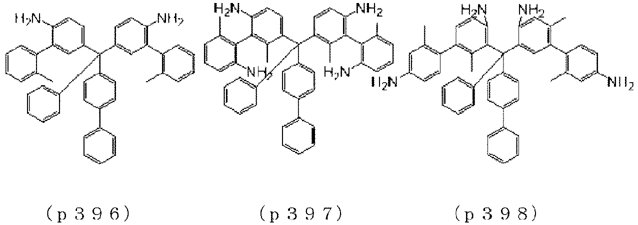

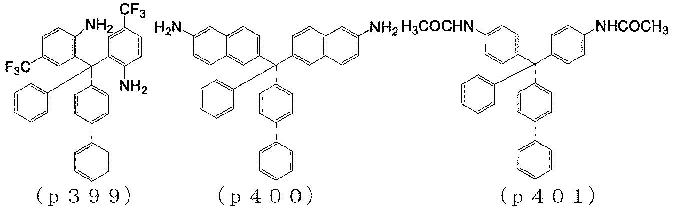

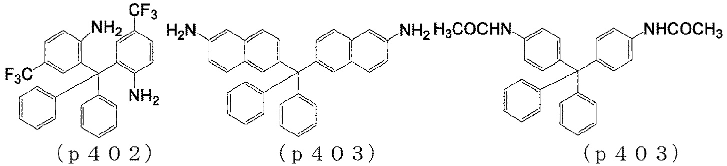

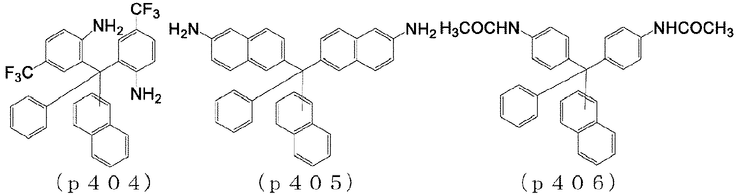

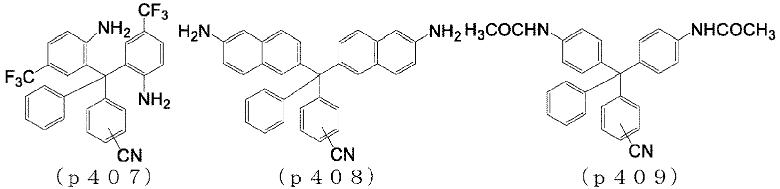

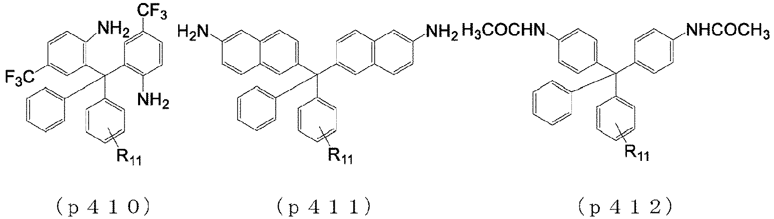

Examples of R₁₁ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₁ may be 1 or more.

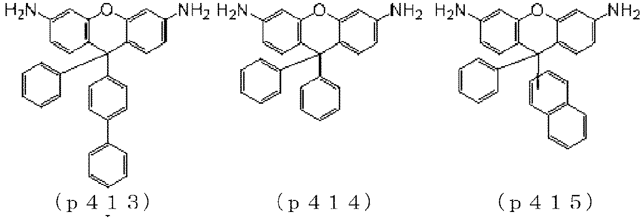

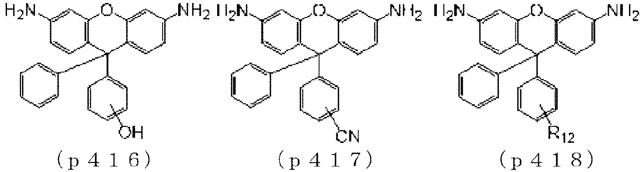

Examples of R₁₂ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₂ may be 1 or more.

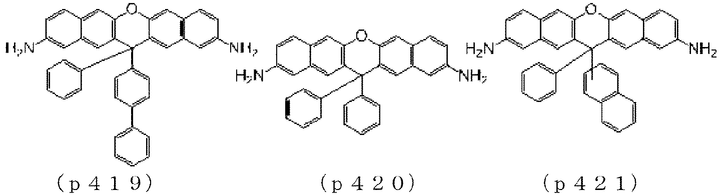

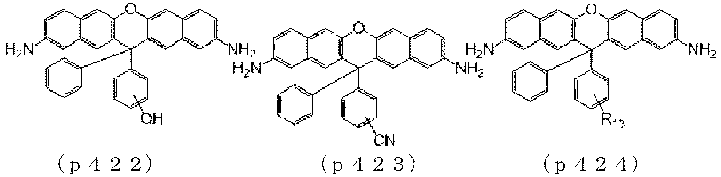

Examples of R₁₃ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a naphthyl group, an anthracene group, a heptacene group, a triacontenyl group, a methoxy group, an ethoxy group, and a triacontyloxy group, all of which include isomers. For example, a butyl group includes a n-butyl group, an isobutyl group, a sec-butyl group, and a tert-butyl group. The number of R₁₃ may be 1 or more.

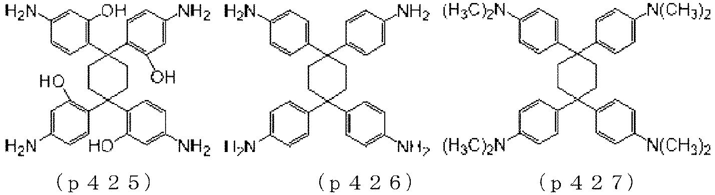

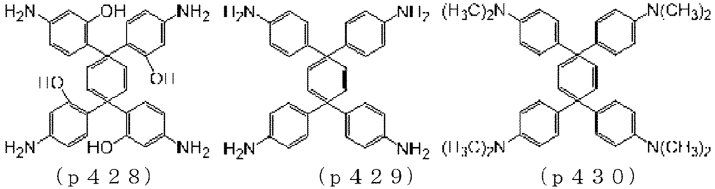

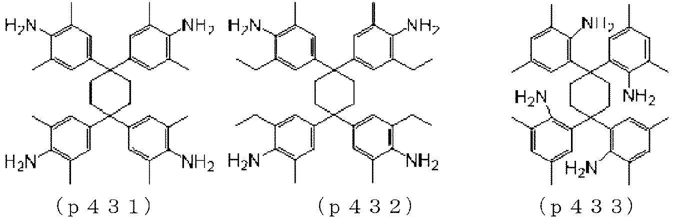

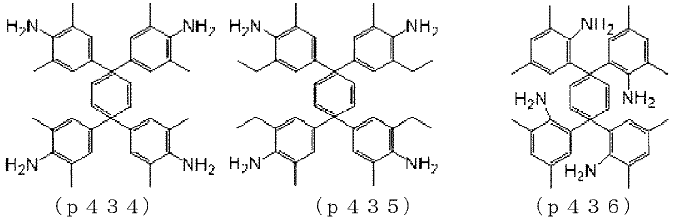

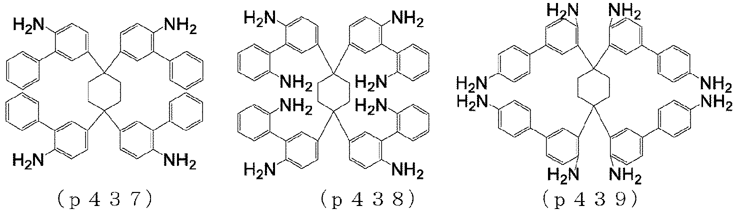

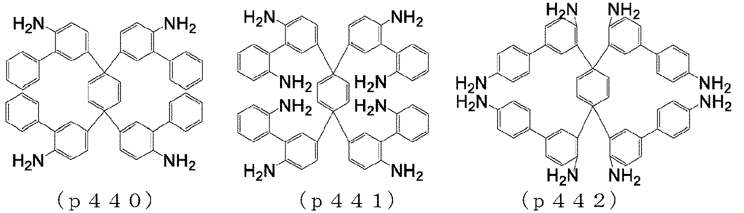

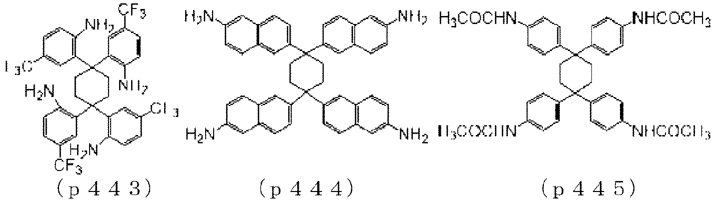

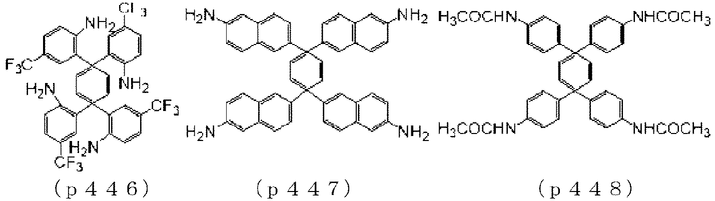

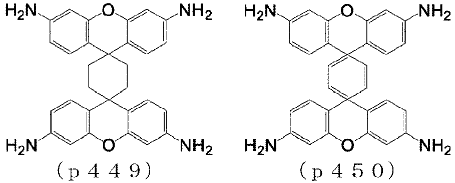

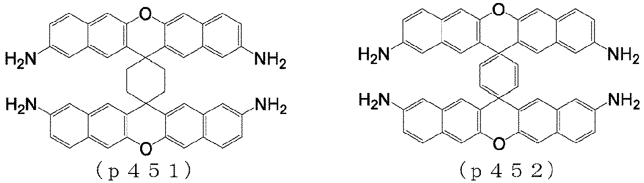

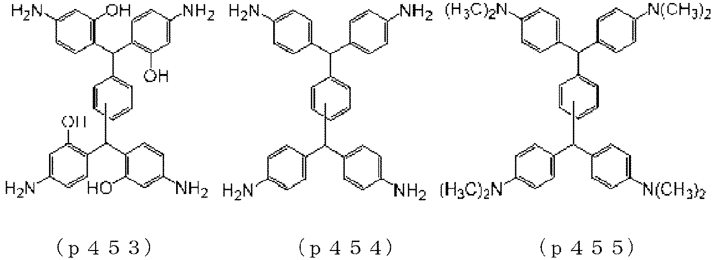

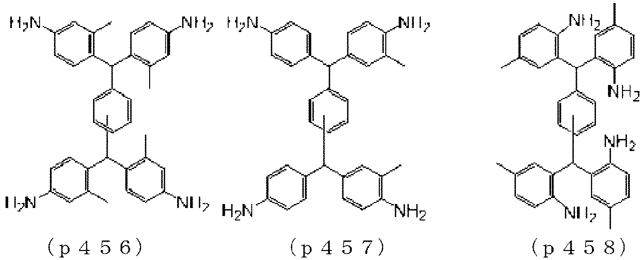

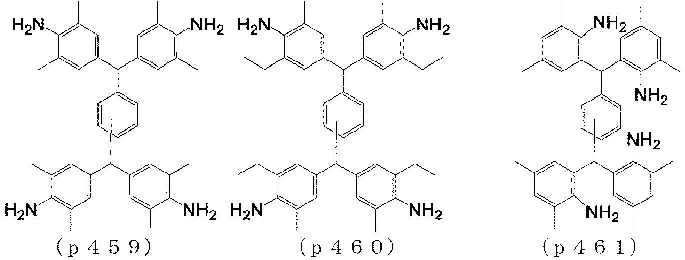

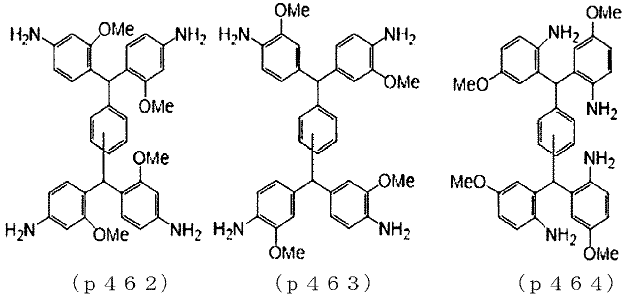

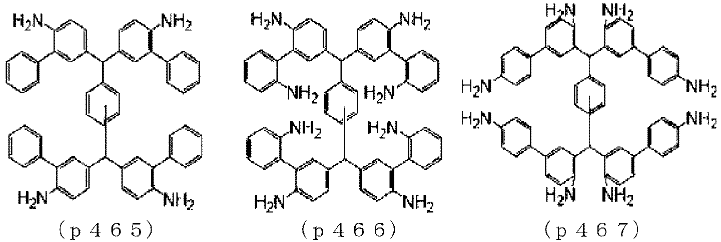

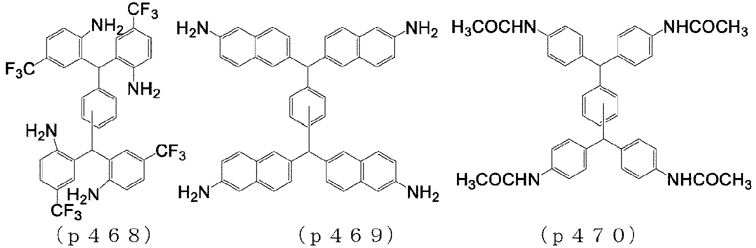

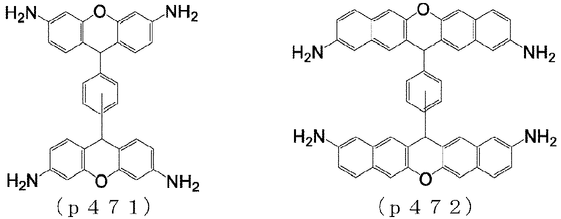

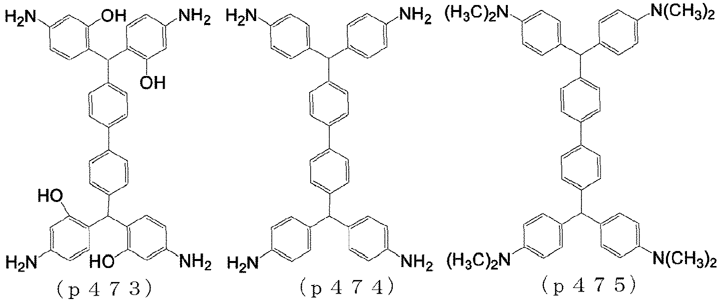

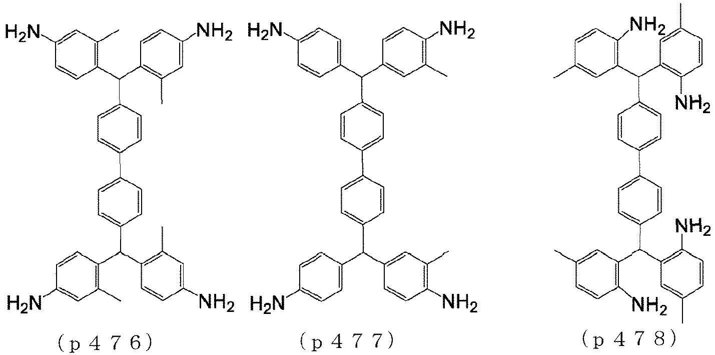

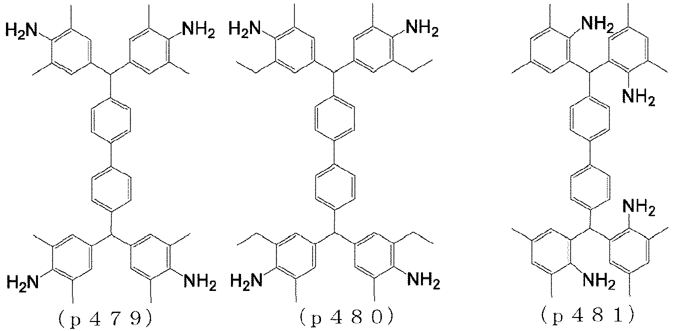

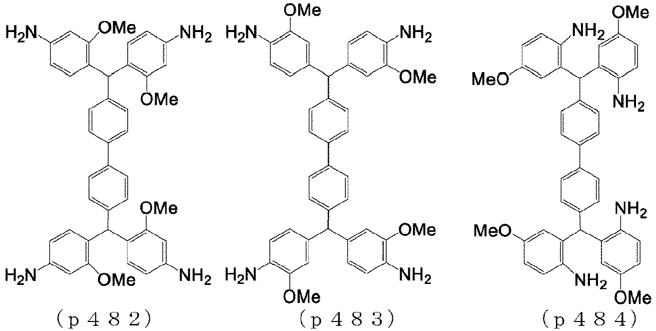

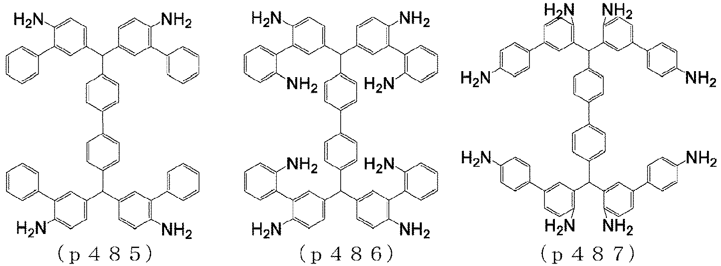

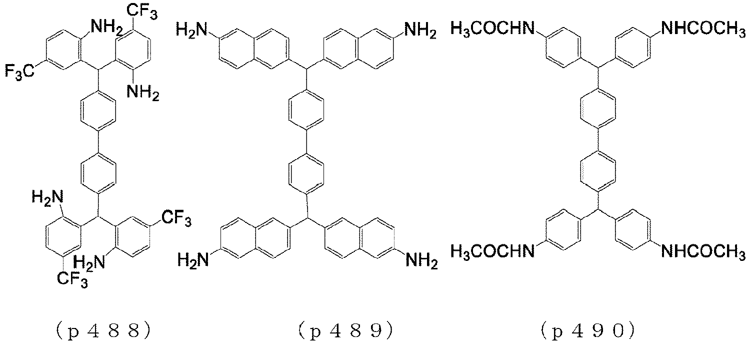

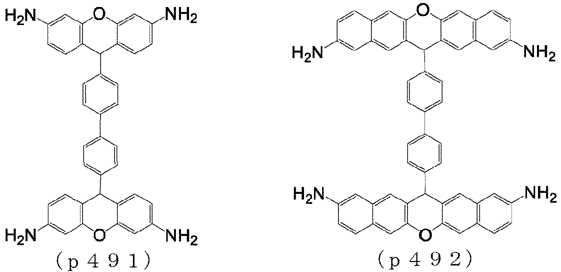

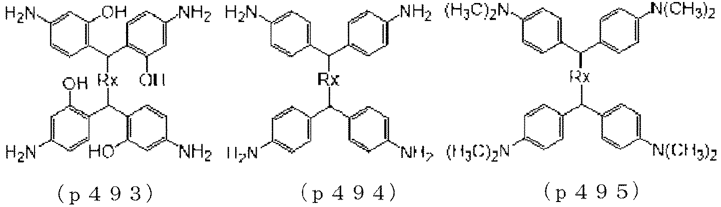

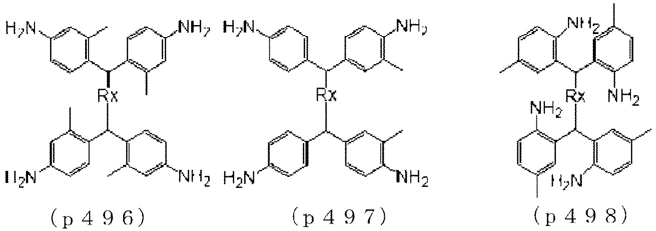

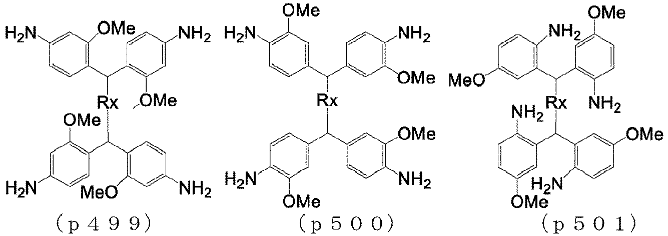

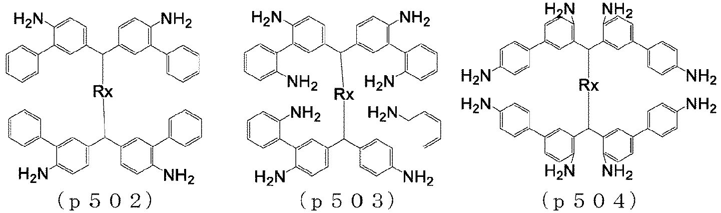

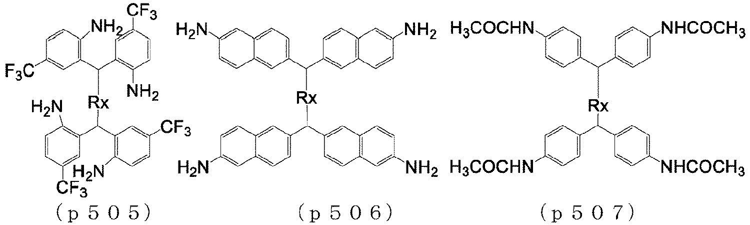

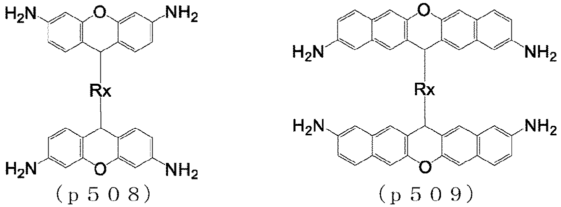

Rx represents divalent alkylene groups obtained by removing one hydrogen atom from monovalent alkyl groups such as a methyl group, an ethyl group, a propyl group, a butyl group, a pentyl group, a hexyl group, a heptyl group, an octyl group, a nonyl group, a decyl group, an undecyl group, a dodecyl group and a triacontyl group; and divalent groups obtained by removing one hydrogen atom from monovalent groups having an alicyclic structure such as cyclopropyl, cyclobutyl, cyclopentyl, cyclohexyl, cycloheptyl, cyclooctyl, cyclononyl, cyclodecyl, cycloundecyl, cyclododecyl, cyclotriacontyl, norbornyl and adamantyl.

### [Method for Producing (Poly)amine Compound]

A method for producing a (poly)amine compound according to the present embodiment represented by any of the formula (0), the formula (1), the formula (2), the formula (3), the formula (0-1), the formula (0-2) and the formula (0-3) (hereinafter, also simply referred to as the "(poly)amine compound according to the present embodiment") includes a step of subjecting an aniline and an aldehyde and/or a ketone as reaction raw materials to polycondensation reaction in the presence of an acid catalyst (a polycondensation step). For a more detailed method for polycondensation, see, for example, a method described in Tetrahedron Letters; Vol. 46 (2005); p.1119-1122. The polycondensation step may be carried out, for example, at normal pressure.

The production method according to the present embodiment may further include a step of substituting a hydrogen atom in at least one functional group (hereinafter, referred to as a "specific functional group") selected from the group consisting of an amino group, a carboxyl group, a thiol group and a hydroxy group of the (poly)amine compound obtained in the polycondensation step with an acid crosslinkable group (a substitution step). Examples of a method of substituting a hydrogen atom with an acid crosslinkable group include, for example, a method mentioned later. In the substitution step, pressure may be increased as needed.

### [Polycondensation Step]

Hereinafter, the polycondensation step will be described.

### (1. Aniline)

Examples of the aniline include an aniline represented by the following formula (0-a), and it is preferably an aniline represented by the following formula (1-a).

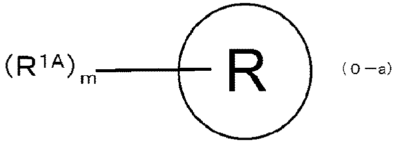

In the above formula, R^{1A}, m and R are all the same as R^{1A}, m and R in the formula (0).

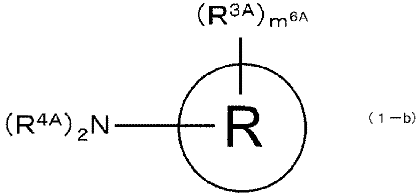

In the above formula, R^{3A}, m^{6A}, R^{4A} and R are all the same as R^{3A}, m^{6A}, R^{4A} and R in the formula (1) .

Examples of the aniline include aromatic amines optionally having a substituent and/or derivatives thereof. Specific examples of the aniline include aniline, toluidine, trimethylaniline, anisidine, hydroxyphenylamine, phenylaniline, biphenyldiamine, (trifluoromethyl)aniline, aminophenol, naphthylamine, dimethylaminobenzene and acetanilide. The site of the substituent is not particularly limited and may be any of position 2, position 3, position 4, position 5 and position 6, and the number of the substituent may be one or may be multiple. These anilines can be used alone as one kind or can be used in combination of two or more kinds. Among them, it is preferable to use any of aniline, dimethylaminobenzene and aminophenol from the viewpoint of the stable supply of raw materials.

### (2. Aldehyde)

Examples of the aldehyde include a compound represented by the following formula (0-b), and it is preferably a compound represented by the following formula (3-a).

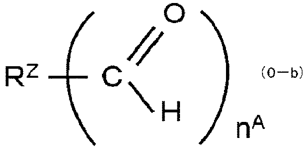

In the above formula, R^{z} and n^{A} are the same as R^{z} and n^{A} in the formula (0).

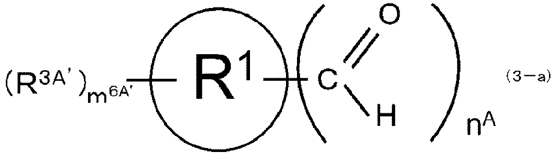

In the above formula, R^{3A'}, m^{6A'}, R¹ and n^{A} are the same as R^{3A'}, m^{6A'}, R¹ and n^{A} in the formula (3).

Specific examples of the formaldehyde include formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, dimethylbenzaldehyde, ethylbenzaldehyde, propylbenzaldehyde, butylbenzaldehyde, fluorobenzaldehyde, biphenylaldehyde, naphthaldehyde, anthracenecarbaldehyde, phenanthrenecarbaldehyde, pyrenecarbaldehyde and furfural. These formaldehydes can be used alone as one kind or can be used in combination of two or more kinds. Among them, it is preferable to use at least one selected from the group consisting of benzaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde and furfural from the viewpoint of being capable of providing a (poly)amine compound to be obtained with further higher heat resistance, and it is preferable to use at least one selected from the group consisting of benzaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde and furfural from the viewpoint of being capable of providing a (poly)amine compound to be obtained with further higher etching resistance. It is also preferable to use an aldehyde having an aromatic ring from the viewpoint of being capable of providing a (poly)amine compound to be obtained with further higher heat resistance and refractive index.

### (3. Ketone)

Examples of the ketone include a ketone represented by the following formula (0-c).

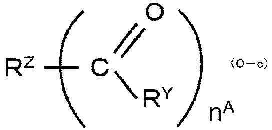

In the above formula, R^{Y}, R^{Z} and n^{A} are all the same as R^{Y}, R^{Z} and n^{A} in the formula (2).

The ketone may be, for example, a ketone represented by the following formulas (cy1-c), (cy2-c), (cy3-c) and (cy4-c).

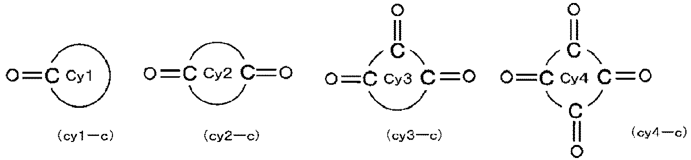

In the above formulas, cy1, cy2, cy3 and cy4 are the same as cy1, cy2, cy3 and cy4 in the above formulas (cy1), (cy2), (cy3) and (cy4).

Specific examples of the ketone include acetone, methyl ethyl ketone, cyclobutanone, cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl. These ketones can be used alone as one kind or can be used in combination of two or more kinds. Among them, it is preferable to use at least one selected from the group consisting of cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl from the viewpoint of being capable of providing a (poly)amine compound to be obtained with further higher heat resistance, and it is preferable to use at least one selected from the group consisting of acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl and diphenylcarbonylbiphenyl from the viewpoint of being capable of providing a (poly)amine compound to be obtained with further higher etching resistance. It is also preferable to use a ketone having an aromatic ring from the viewpoint of being capable of providing a (poly)amine compound to be obtained with further higher heat resistance and refractive index.

### (4. Acid Catalyst)

For the acid catalyst, those publicly known can be used, and examples thereof include inorganic acids and organic acids. Examples of the inorganic acid include hydrochloric acid, sulfuric acid, phosphoric acid, hydrobromic acid and hydrofluoric acid. Examples of the organic acid include oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid and naphthalenedisulfonic acid. The acid catalyst may be a Lewis acid such as zinc chloride, aluminum chloride, iron chloride and boron trifluoride, or a solid acid such as tungstosilicic acid, phosphotungstic acid, silico molybdic acid and phosphomolybdic acid. Among them, organic acids and solid acids are preferable from the viewpoint of production (from the viewpoint of easy availability and handleability), and hydrochloric acid or sulfuric acid is more preferable. These acid catalysts can be used alone as one kind or can be used in combination of two or more kinds. The amount of the acid catalyst used can be arbitrarily set according to, for example, the kind of the raw materials used and the catalyst used and moreover the reaction conditions, and it may be about 0.01 to 100 parts by mass based on 100 parts by mass of the entire reaction raw materials.

### (5. Reaction Solvent)

During the polycondensation step, reaction raw materials may be subjected to reaction in a solvent. The solvent may be any solvent as long as it allows sufficient progress of reaction between the aniline and the aldehyde and/or the ketone, which are used as the reaction raw materials. Examples of the solvent include water, methanol, ethanol, propanol, butanol, tetrahydrofuran, dioxane, ethylene glycol dimethyl ether, ethylene glycol diethyl ether and a mixed solvent thereof. These solvents can be used alone as one kind or can be used in combination of two or more kinds.

The amount of the solvent used can be arbitrarily set according to, for example, the kind of the raw materials used and the catalyst used and moreover the reaction conditions, and it may be about 0 to 2000 parts by mass based on 100 parts by mass of the entire reaction raw materials.

The reaction temperature during the polycondensation reaction can be arbitrarily selected according to the reactivity of reaction raw materials, but it may be normally within the range of about 10 to 200°C. In order to obtain the (poly)amine compound according to the present embodiment, a higher reaction temperature is more preferable. Specifically, the range of 60 to 200°C is preferable.

In the polycondensation step, the aniline, the aldehyde and/or the ketone, and the catalyst may be charged in one portion to allow reaction, or the aniline, the aldehyde and/or the ketone may be sequentially added dropwise in the presence of the catalyst to allow reaction. After the polycondensation reaction terminates, the obtained compound may be isolated by a publicly known method. For example, by elevating the temperature of the reaction vessel to 130 to 230°C in order to remove unreacted raw materials and catalyst present in the system, and by removing volatile portions at about 1 to 50 mmHg, the (poly)amine compound can be obtained.

It is preferable to add the aniline in an amount of 1.0 mole or more and to add the acid catalyst in the range of 0.001 to 1 mole, based on 1.0 mole of the aldehyde and/or the ketone. In addition, it is preferable to conduct the reaction at normal pressure at a reaction temperature of 50 to 150°C and for a reaction time of about 20 minutes to 100 hours.

After the reaction terminates, the (poly)amine compounds may be isolated by a publicly known method. In the isolation method, for example, the reaction solution is first concentrated, and pure water is added to precipitate the reaction product. Next, the precipitated reaction product is cooled to room temperature, and then separated by filtration. The solid matter obtained by the separation is further filtered and dried. The dried reaction product is separated and purified by column chromatography into the product and a byproduct. Furthermore, the reaction product separated and purified is subjected to solvent distillation, followed by filtration and drying, thereby obtaining the (poly)amine compound according to the present embodiment, which is the target compound.

After the polycondensation step, if the obtained (poly)amine compound has a substituent, the substituent may be eliminated by a publicly known method, thereby producing an unsubstituted (poly)amine compound.

### [Substitution Step]

Hereinafter, the substitution step according to the present embodiment will be described.

In the substitution step according to the present embodiment, for example, a compound for introducing a crosslinkable group is used to substitute a hydrogen atom in a specific functional group with an acid crosslinkable group. Examples of the compound for introducing a crosslinkable group include allyl halides, acrylic acid, methacrylic acid, acrylic acid halides, methacrylic acid halides, vinylbenzyl halides and epihalohydrins, and these compounds may be synthesized by a publicly known synthetic method, or commercial products may be used therefor.

Hereinafter, the substitution method will be described with reference to a specific example. First, the compound for introducing the crosslinkable group is dissolved or suspended in an aprotic solvent (for example, acetone, tetrahydrofuran (THF), and propylene glycol monomethyl ether acetate). Next, an epihalohydrin (for example, epichlorohydrin and epibromohydrin) is added to the aprotic solvent. In the presence of an acid catalyst such as hydrochloric acid, reaction is allowed at normal pressure at a reaction temperature of 0 to 60°C for a reaction time of 6 to 72 hours. The reaction solution is neutralized with a publicly known alkali compound, and distilled water is added to the reaction solution to precipitate a white solid. The separated white solid is washed with distilled water and dried, thereby obtaining a (poly)amine compound in which a hydrogen atom in the specific functional group has been substituted with the acid crosslinkable group.

Alternatively, a (poly)amine compound having a specific functional group is dissolved or suspended in an aprotic solvent. Next, an allyl halide (for example, allyl chloride and allyl bromide), acrylic acid, methacrylic acid, an acrylic acid halide (for example, acrylic acid chloride and acrylic acid bromide), a methacrylic acid halide (for example, methacrylic acid chloride and methacrylic acid bromide), or a vinylbenzyl halide (for example, vinylbenzyl chloride and vinylbenzyl bromide) are added to the solvent, and in the presence of an alkali catalyst (for example, sodium hydroxide, triethylamine and potassium carbonate), reaction is allowed at normal pressure at a reaction temperature of 0 to 110°C for a reaction time of 6 to 72 hours. The reaction solution is neutralized with an acid such as hydrochloric acid, and distilled water is added to the reaction solution to precipitate a white solid. Next, the separated white solid is washed with distilled water and dried, thereby obtaining a (poly)amine compound in which a hydrogen atom in the specific functional group has been substituted with the acid crosslinkable group.

The timing at which the acid crosslinkable group is introduced may be before the polycondensation reaction between the aniline and the aldehyde and/or the ketone, or after the polycondensation reaction. In addition, it may also be after producing a resin, which will be mentioned later.

When the (poly)amine compound according to the present embodiment includes an acid crosslinkable group, the acid crosslinkable group is allowed to react in the presence of or in the absence of a radical, or an acid or an alkali, thereby improving refractive index, heat resistance, mechanical strength, permeability, and solubility in a coating solvent, or an acid or an alkali, or an organic solvent used for a developing solution.

Examples of the acid crosslinkable group include an allyl group, a (meth)acryloyl group, a vinyl group, an epoxy group, an alkoxymethyl group and a cyanato group.

### [Cured Product]

The (poly)amine compound according to the present embodiment is used as a curing agent for curing a compound such as epoxy, urethane, urea and cyanate. As such, a cured product according to the present embodiment includes a cured product obtained by curing a curable compound with a curing agent, wherein the cured product has the (poly)amine compound according to the present embodiment. The curable compound normally refers to a compound that reacts with an amine-based compound, which is used as a curing agent, and is cured, and examples thereof include epoxy compounds, urethane compounds, urea compounds and cyanate compounds.

### [Resin]

A resin according to the present embodiment includes a monomer unit containing a unit derived from the (poly)amine compound according to the present embodiment. The resin according to the present embodiment has excellent structure-forming ability (for example, film-forming ability), heat resistance, transparency and refractive index due to the fact that it includes the monomer unit according to the present embodiment.

The resin according to the present embodiment may be composed of the monomer unit according to the present embodiment alone, may include another monomer unit that is copolymerizable with the (poly)amine compound, or may include a unit derived from a crosslinking compound.

Examples of the monomer unit constituting the resin according to the present embodiment include the following formulas (4) to (7), (0-5), (0-6), (0-7) and (0-8). Among these monomer units, the monomer unit represented by the following formula (5) is preferable from the viewpoint of further improving structure-forming ability. Furthermore, the monomer unit represented by the following formula (6) is preferable from the viewpoint of further improving solubility in an organic solvent. Furthermore, the monomer unit represented by the following formula (7) is preferable and the monomer unit represented by the following formula (8) is more preferable from the viewpoint of further improving solubility in an organic solvent and further improving heat resistance and refractive index. In addition, the monomer units represented by formulas (4), (5), (6) and (7) are preferably monomer units represented by (0-5), (0-6), (0-7) and (0-8), respectively, from the viewpoint of further improving structure-forming ability.

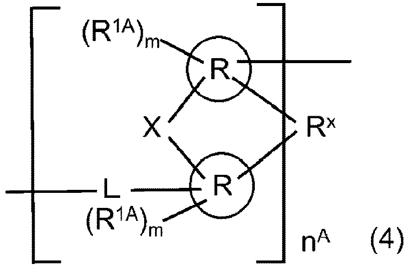

In the above formula (4), L is a linear or branched linking group having 1 to 30 carbon atoms, or a single bond.

R^{X}, R^{1A}, X, R, m and n^{A} are as defined above.

When L is a linking group, examples of the linking group include a group (residue) derived from a compound that is capable of oligomerizing or polymerizing the (poly)amine compound according to the present embodiment. The group derived from a compound that is capable of oligomerization or polymerization will be mentioned later.

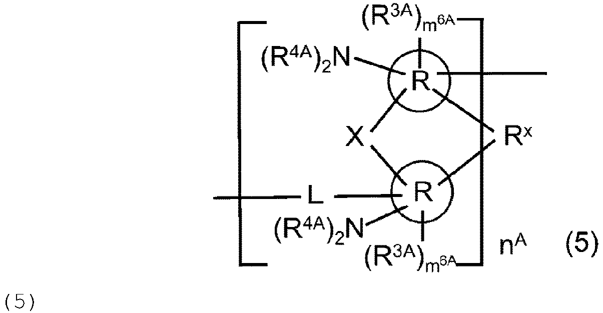

In the above formula (5), R, n^{A}, R^{3A}, R^{4A}, m^{6A} and L are as defined above.

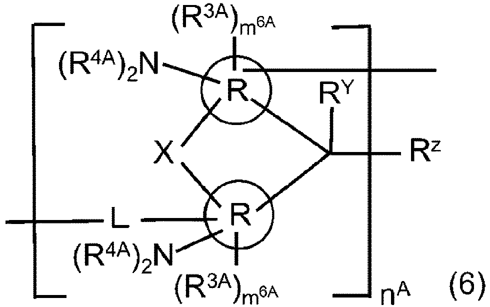

In the above formula (6), R, n^{A}, R^{3A}, R^{4A}, m^{6A}, R^{Y}, R^{Z} and L are as defined above.

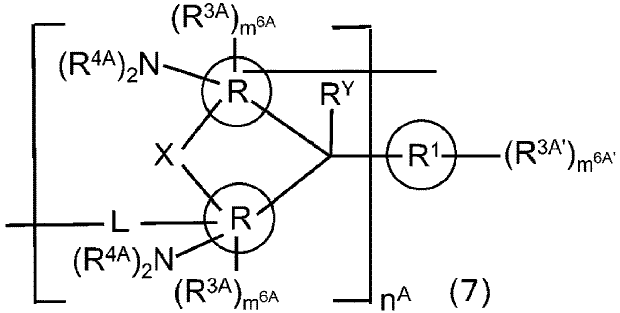

In the above formula (7), R, n^{A}, R^{3A}, R^{4A}, m^{6A}, R^{Y}, R^{3A'}, R¹, m^{6A'} and L are as defined above.

### [Monomer Unit Represented by Formula (7a) or (7b)]

The monomer unit constituting the (poly)amine resin, represented by the above formula (7), is preferably a monomer unit represented by the following formula (7a), and is more preferably a monomer unit represented by the following formula (7b) from the viewpoint of being capable of further improving solubility and heat resistance.

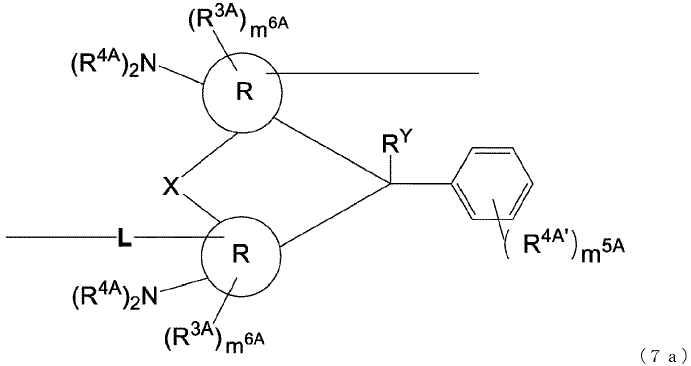

In the above formula (7a), X, R, R^{3A}, R^{4A}, m^{6A}, R^{Y} and L are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom). m^{5A} represents an integer of 1 to 5.

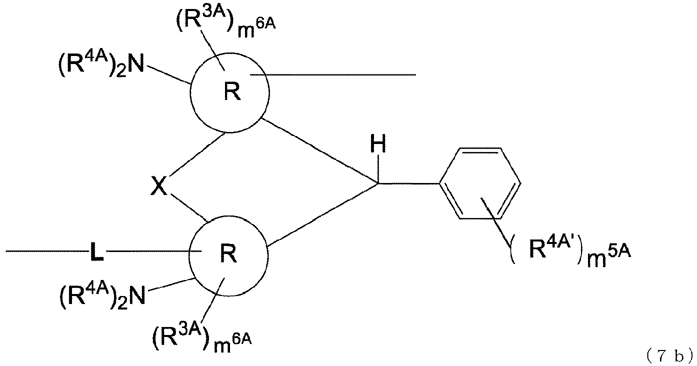

In the above formula (0a), X, R, R^{3A}, R^{4A}, m^{6A}, R^{Y} and L are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom). m^{5A} represents an integer of 1 to 5.

### [Monomer Unit Represented by Formula (7a-1) or (7b-1)]

The monomer unit constituting the (poly)amine resin, represented by the formula (7), is preferably a monomer unit represented by the following formula (7a-1), and is more preferably a monomer unit represented by the following formula (7b-1) from the viewpoint of being capable of further improving solubility.

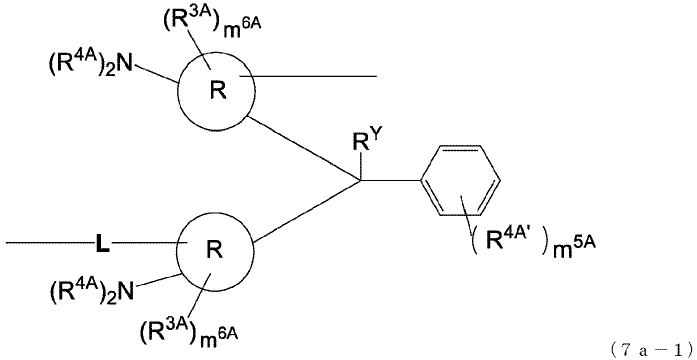

In the above formula (7a-1), R, R^{3A}, R^{4A}, m^{6A}, R^{4A'}, m^{5A}, R^{Y} and L are as defined above.

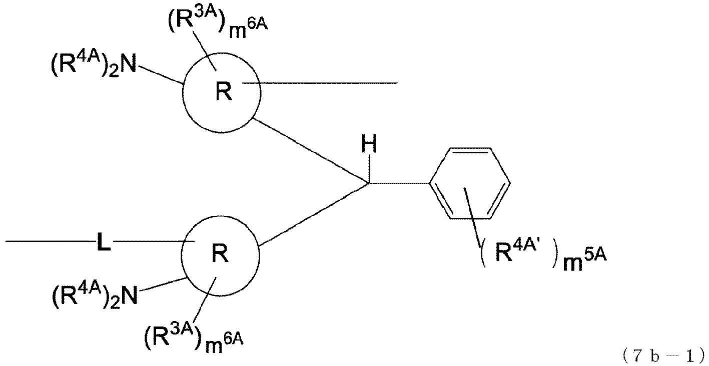

In the above formula (7b-1), R, R^{3A}, R^{4A}, m^{6A}, m^{5A}, R^{Y} and L are as defined above.

### [Monomer Unit Represented by Formula (7b-2)]

The monomer unit constituting the (poly)amine resin, represented by the formula (7b-1), is further preferably a monomer unit represented by the following formula (7b-2) from the viewpoint of being capable of further improving flowability upon formation of a film.

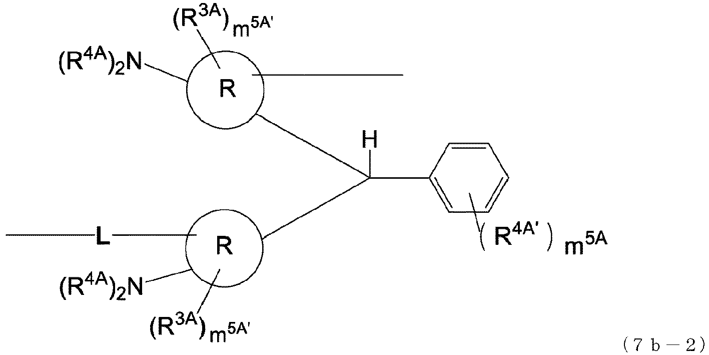

In the above formula (7b-2), R, R^{3A}, R^{4A}, R^{4A'}, m^{5A}, R^{Y} and L are as defined above, and each m^{5A'} is independently an integer of 1 to 5.

### [Monomer Unit Represented by Formula (7a-2)]

The monomer unit constituting the (poly)amine resin, represented by the formula (7a-1), is further preferably a monomer unit represented by the following formula (7a-2) from the viewpoint of being capable of further improving solubility.

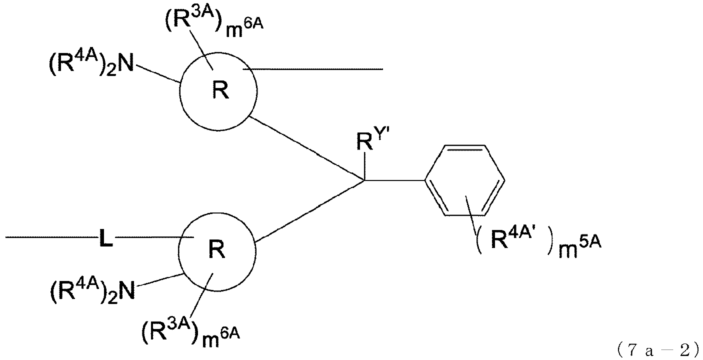

In the above formula (7a-2), R, R^{3A}, R^{4A}, m^{6A}, m^{5A} and L are as defined above, and R^{Y'} is obtained by removing a hydrogen atom from R^{Y}.

### [Monomer Unit Represented by Formula (7b-3)]

The monomer unit constituting the (poly)amine resin, represented by the formula (7b-1), is further preferably a compound represented by the following formula (7b-3) from the viewpoint of being capable of further improving flowability upon formation of a film.

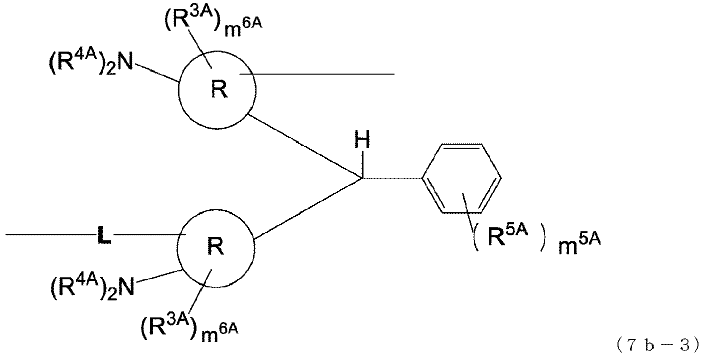

In the above formula (7b-3), R, R^{3A}, R^{4A}, R^{4A'}, m^{5A} and L are as defined above, and R^{5A} represents a linear or branched, or cyclic alkyl group having 5 or more carbon atoms and optionally having a substituent.

### [Compound Represented by Formula (7a-3) or (7b-4)]

The (poly)amine compound represented by the formula (7) is preferably a compound represented by the following formula (7a-3), and is more preferably a compound represented by the following formula (7b-4) from the viewpoint of further excellent supply of raw materials and being capable of further improving solubility and heat resistance.

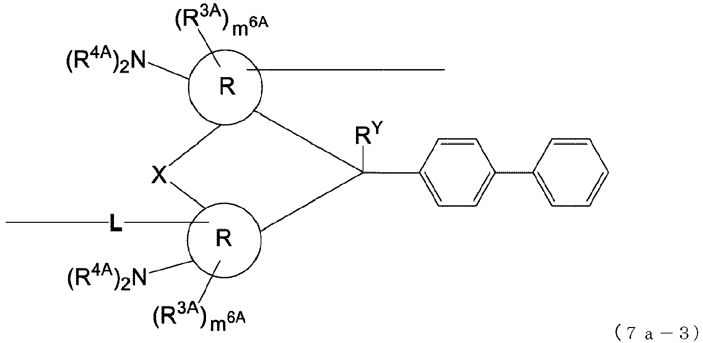

In the above formula (7a-3), X, R, R^{3A}, R^{4A}, m^{6A}, R^{Y} and L are as defined above.

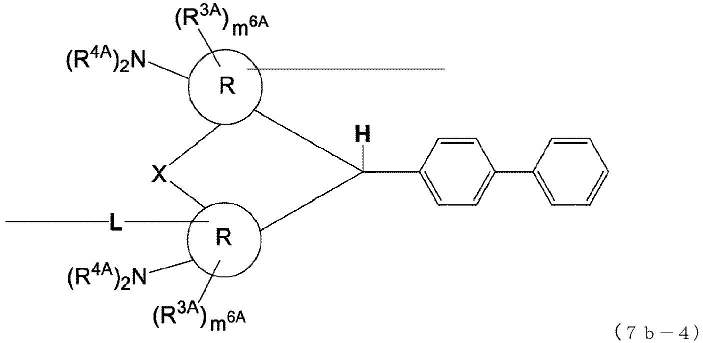

In the above formula (7b-4), X, R, R^{3A}, R^{4A}, m^{6A} and L are as defined above.

### [Monomer Unit Represented by Formula (7a') or (7b')]

The monomer unit constituting the (poly)amine resin, represented by the formula (7), is preferably a compound represented by the following formula (7a'), and is more preferably a compound represented by the following formula (7b') from the viewpoint of being capable of further improving heat resistance. 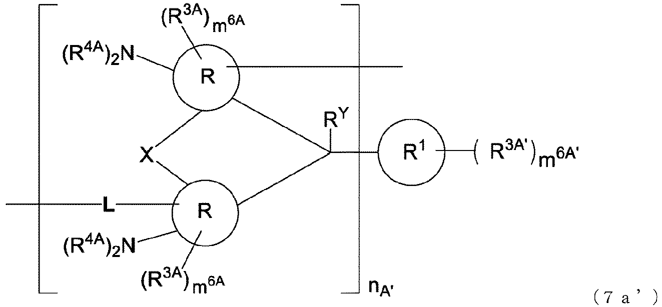 In the above formula (7a'), R, X, R^{3A}, R^{4A}, m^{6A}, R^{Y}, R1, m^{6A'} and L are as defined above, and n^{A'} is an integer of 2 to 4. 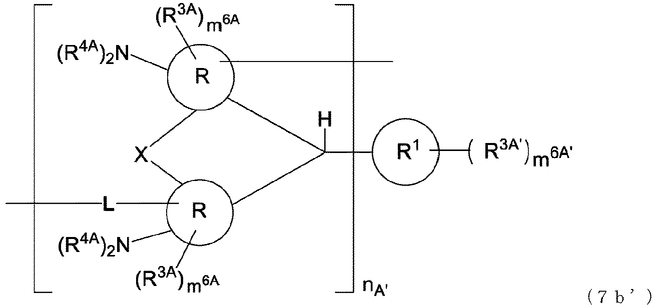 In the above formula (7b'), R, X, R^{3A}, R^{4A}, m^{6A}, R1 and m^{6A'} are as defined above, and n^{A'} is an integer of 2 to 4.

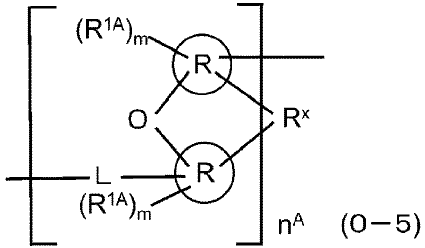

In the above formula (0-5), R^{X}, R^{1A}, R, m, n^{A} and L are as defined above.

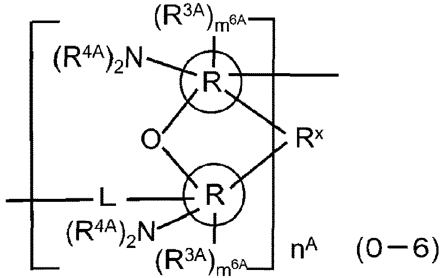

In the above formula (0-6), R^{X}, R, n^{A}, R^{3A}, R^{4A}, m^{6A} and L are as defined above.

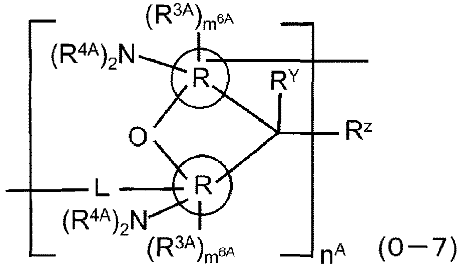

In the above formula (0-7), R, n^{A}, R^{3A}, R^{4A}, m^{6A}, R^{Y}, R^{Z} and L are as defined above.

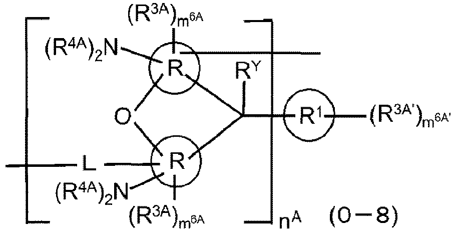

In the above formula (0-8), R, n^{A}, R^{3A}, R^{4A}, m^{6A}, R^{Y}, R^{3A'}, m^{6A'} and L are as defined above.

### [Method for Producing Resin According to Present Embodiment]

The resin according to the present embodiment is obtained by allowing the compound according to the present embodiment to react with a crosslinking compound as needed.

The crosslinking compound is not particularly limited as long as it is a compound that is capable of oligomerizing or polymerizing the (poly)amine compound according to the present embodiment. Specific examples thereof include aldehydes, ketones, carboxylic acids, carboxylic acid anhydrides, carboxylic acid halides, halogen-containing compounds, isocyanates and unsaturated hydrocarbon group-containing compounds. When L is a linking group in the formula (4), that linking group corresponds to a group derived from these compounds.

Specific examples of the resin according to the present embodiment include resins that have been made into novolac by, for example, a condensation reaction between the (poly)amine compound according to the present embodiment and an aldehyde and/or a ketone, which is a crosslinking compound.

Herein, examples of the aldehyde for making a resin into novolac include formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde and furfural. Examples of the ketone for making a resin into novolac include ketones exemplified as the reaction raw material. Among them, formaldehydes are preferable. These aldehydes and/or ketones can be used alone as one kind or can be used in combination of two or more kinds. The amount of the above aldehyde and/or ketone used is not particularly limited, but it is preferably 0.2 to 5 moles and more preferably 0.5 to 2 moles based on 1 mole of the (poly)amine compound according to the present embodiment.

Also, a catalyst can be used in the condensation reaction between the (poly)amine compound according to the present embodiment and the aldehyde and/or the ketone. Examples of the acid catalyst used herein include acid catalysts exemplified in the section of the condensation step.

Also, the amount of the acid catalyst used can be arbitrarily set according to, for example, the kind of the raw materials used and the catalyst used and moreover the reaction conditions. The amount of the acid catalyst used is preferably 0.01 to 100 parts by mass based on 100 parts by mass of the reaction raw materials.

The aldehyde and/or the ketone is not necessarily needed in the case of subjecting the (poly)amine compound according to the present embodiment to a copolymerization reaction with a compound having a non-conjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborn-2-ene, α-pinene, β-pinene and limonene.

Also, a reaction solvent can be used in the condensation reaction between the (poly)amine compound according to the present embodiment and the aldehyde and/or the ketone. Examples of the reaction solvent may include reaction solvents exemplified in the section of the polycondensation step.

In addition, the amount of the solvent used, reaction temperature, reaction time, isolation method after the reaction and the like can also be exemplified by the amount of the solvent used, reaction temperature, reaction time and isolation method after the reaction, all of which are exemplified in the section of the polycondensation step.

The resin according to the present embodiment may be a homopolymer of the (poly)amine compound according to the present embodiment, that is, a polymer composed of the formula (4) alone, but it may also be a copolymer between the (poly)amine compound and a copolymerizable compound (for example, a phenol). Herein, examples of the copolymerizable phenol include phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol and thymol.

The resin according to the present embodiment may be copolymerized with a polymerizable monomer other than the above-described further phenol. Examples of the copolymerization monomer include naphthol, methylnaphthol, methoxynaphthol, dihydroxynaphthalene, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornene, pinene and limonene. The resin according to the present embodiment may be a binary or higher (for example, binary to quaternary) copolymer of the (poly)amine compound according to the present embodiment and the above-mentioned phenol. It may be a binary or higher (for example, binary to quaternary) copolymer of the (poly)amine compound according to the present embodiment and the above-mentioned copolymerization monomer. It may be a ternary or higher (for example, ternary to quaternary) copolymer of the (poly)amine compound according to the present embodiment, the above-mentioned phenol, and the above-mentioned copolymerization monomer.

The weight average molecular weight of the resin according to the present embodiment is not particularly limited, but it is preferably 500 to 300,000 and is more preferably 750 to 200,000 in terms of polystyrene. The resin according to the present embodiment preferably has dispersibility (weight average molecular weight Mw/number average molecular weight Mn) within the range of 1.2 to 7 from the viewpoint of enhancing crosslinking efficiency while suppressing volatile components during baking.

The resin according to the present embodiment preferably has high solubility in a solvent from the viewpoint of easier application to a wet process, etc. More specifically, in the case of using 1-methoxy-2-propanol (PGME) and/or propylene glycol monomethyl ether acetate (PGMEA) as a solvent, these (poly)amine compounds and/or resins preferably has a solubility of 10% by mass or more in the solvent. Herein, the solubility in PGME and/or PGMEA is defined as "mass of the resin / (mass of the resin + mass of the solvent) × 100 (% by mass)". For example, when 10 g of the resin is dissolved in 90 g of PGMEA, the solubility of the resin in PGMEA is "10% by mass or more"; and when 10 g of the resin is not dissolved in 90 g of PGMEA, the solubility is "less than 10% by mass".

### Examples

Hereinafter, the present embodiment will be described with reference to Examples, but the present embodiment is not limited to Examples.

### (Example 1) Synthesis of BiA-1

To a container (internal capacity: 100 mL) equipped with a stirrer, a condenser tube and a burette, 2.2 g (20 mmol) of m-aminophenol (a reagent manufactured by Sigma-Aldrich) and 20.0 ml of a 1,4-dioxane solution of hydrogen chloride were added, and 1.8 g (10 mmol) of 4-biphenylaldehyde (a reagent manufactured by Sigma-Aldrich) was added, and the contents were stirred at a reaction temperature of 100°C for a reaction time of 6 hours to allow reaction. To this reaction solution, 1000 mL of pure water was added, and the crude compound was then filtered off. The obtained crude compound was purified by column chromatography to obtain 0.3 g of a (poly)amino compound (BiA-1) represented by the following formula (BiA-1). As a result of measuring the molecular weight of the obtained compound (BiA-1), it was 382. In addition, the following peaks were found by ¹H-NMR measurement performed on the obtained compound (BiA-1), and the compound was confirmed to have a chemical structure of the following formula (BiA-1).
δ (ppm) 9.5 (2H, O-H), 6.9-7.6 (15H, Ph-H), 6.5 (1H, C-H), 4.3 (4H, NH2)

### [Molecular Weight]

The molecular weight of a compound was measured by LC-MS analysis using Acquity UPLC/MALDI-Synapt HDMS manufactured by Waters Corp.
Measurement conditions for NMR are shown below.

### [Structure of Compound]

The structure of the compound was verified by carrying out ¹H-NMR measurement under the following conditions using "Advance 600 II spectrometer" manufactured by Bruker.
Frequency: 400 mhz
Solvent: d6-DMSO
Internal standard: TMS
Measurement temperature: 23°C

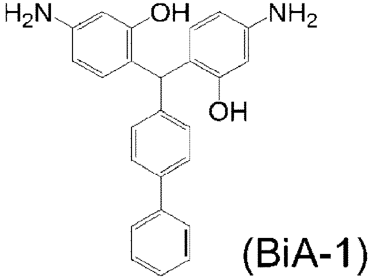

### (Examples 2 to 12) Synthesis of BiA-2 to BiA-12

(Poly)amine compounds represented by the following formulas (BiA-2) to (BiA-12) were obtained in the same manner as Example 1 except that, instead of m-aminophenol and 4-biphenylaldehyde in Example 1, anilines and aldehydes listed in the following Table 1 were used.

**[Table 1]**

| Example | Anilines | Aldehydes | Molecular weight |
|---|---|---|---|
| 2 | o-Methylethylaniline | 4-Biphenylaldehyde | 434 |
| 3 | o-Methoxyaniline | 4-Biphenylaldehyde | 410 |
| 4 | o-Phenylaniline | 4-Biphenylaldehyde | 502 |
| 5 | 2,2'-Diaminobiphenyl | 4-Biphenylaldehyde | 532 |
| 6 | Naphthylamine | 4-Biphenylaldehyde | 450 |
| 7 | Aniline | 4-Cyanobenzaldehyde | 299 |
| 8 | Aniline | Imidazole-4-carboxyaldehyde | 280 |
| 9 | Aniline | 3,4-Dimethylbenzaldehyde | 302 |
| 10 | Aniline | 2,4-Dimethylbenzaldehyde | 302 |
| 11 | Aniline | 3,4,6-Trimethylbenzaldehyde | 316 |
| 12 | Aniline | 2,4,6-Trimethylbenzaldehyde | 333 |
| 13 | Aniline | 4-Propylbenzaldehyde | 316 |
| 14 | Aniline | 4-Pentylbenzaldehyde | 330 |
| 15 | Aniline | 4-Cumylaldehyde | 330 |
| 16 | Aniline | 4-Cyclohexylbenzaldehyde | 350 |
| 17 | Aniline | 2,6-Dimethyl-4-cyclohexylbenzaldehyde | 384 |

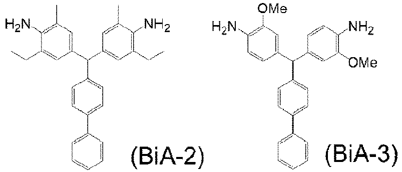

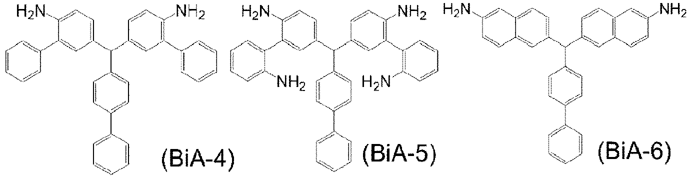

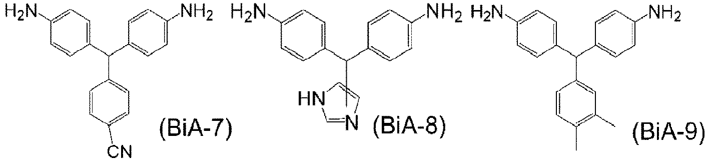

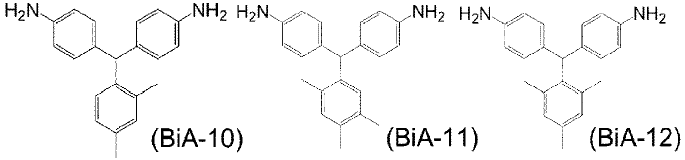

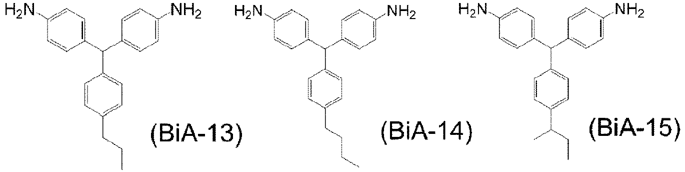

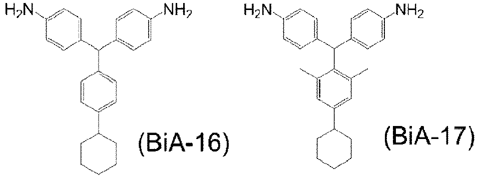

### (Example 18) Synthesis of BiA-18

To a container (internal capacity: 100 mL) equipped with a stirrer, a condenser tube and a burette, 4.4 g (40 mmol) of aniline (a reagent manufactured by Sigma-Aldrich) and 20.0 ml of a 1,4-dioxane solution of hydrogen chloride were added, and 1.8 g (10 mmol) of 4-biphenyldialdehyde (a reagent manufactured by Sigma-Aldrich) was added, and the contents were stirred at a reaction temperature of 110°C for a reaction time of 8 hours to allow reaction. To this reaction solution, 1000 mL of pure water was added, and the crude compound was then filtered off. The obtained crude compound was purified by column chromatography to obtain 0.7 g of a (poly)amino compound (BiA-18) represented by the following formula (BiA-18). As a result of measuring the molecular weight of the obtained compound (BiA-18), it was 546. In addition, the following peaks were found by ¹H-NMR measurement performed on the obtained compound (BiA-18), and the compound was confirmed to have a chemical structure of the following formula (BiA-18). δ (ppm) 7.0-7.8 (24H, Ph-H), 6.6 (2H, C-H), 4.2 (8H, NH2)

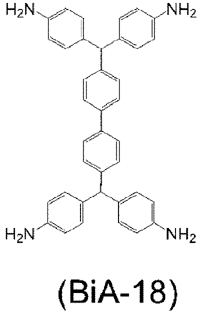

### (Examples 19 to 26) Synthesis of BiA-19 to BiA-26

(Poly)amine compounds represented by the following formulas (BiA-19) to (BiA-26) were obtained in the same manner as Example 1 except that, instead of aniline and 4-biphenyldialdehyde in Example 18, anilines and aldehydes listed in the following Table 2 were used.

**[Table 2]**

| Example | Anilines | Aldehydes | Molecular weight |
|---|---|---|---|
| 19 | Dimethylaminobenzene | 4,4'-Biphenyldialdehyde | 658 |
| 20 | o-Methylethylaniline | 4,4'-Biphenyldialdehyde | 714 |
| 21 | o-Methoxyaniline | 4,4'-Biphenyldialdehyde | 666 |
| 22 | o-Phenylaniline | 4,4'-Biphenyldialdehyde | 850 |
| 23 | 2,2'-Diaminobiphenyl | 4,4'-Biphenyldialdehyde | 910 |
| 24 | Naphthylamine | 4,4'-Biphenyldialdehyde | 746 |
| 25 | Aniline | Terephthalaldehyde | 470 |
| 26 | Aniline | Isophthalaldehyde | 470 |

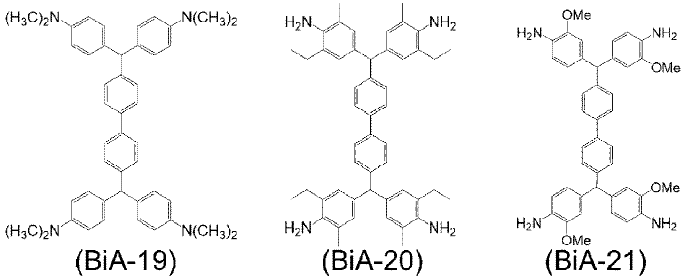

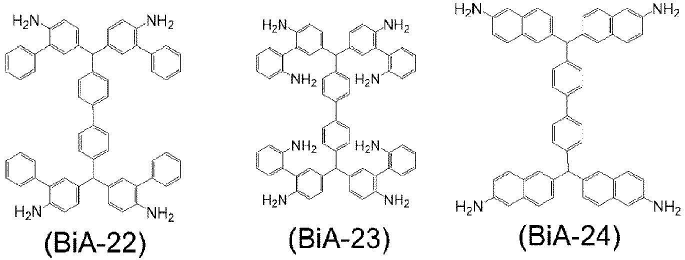

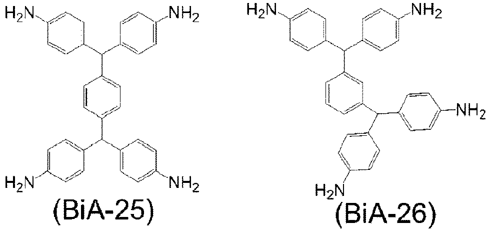

### (Example 27) Synthesis of BiA-27

To a container (internal capacity: 100 mL) equipped with a stirrer, a condenser tube and a burette, 2.2 g (20 mmol) of aniline (a reagent manufactured by Sigma-Aldrich) and 20.0 ml of a 1,4-dioxane solution of hydrogen chloride were added, and 1.3 g (10 mmol) of 4-acetylbiphenyl (a reagent manufactured by Sigma-Aldrich) was added, and the contents were stirred at a reaction temperature of 80°C for a reaction time of 18 hours to allow reaction. To this reaction solution, 1000 mL of pure water was added, and the crude compound was then filtered off. The obtained crude compound was purified by column chromatography to obtain 0.9 g of a (poly)amino compound (BiA-27) represented by the following formula (BiA-27). As a result of measuring the molecular weight of the obtained compound (BiA-27), it was 364. In addition, the following peaks were found by ¹H-NMR measurement performed on the obtained compound (BiA-27), and the compound was confirmed to have a chemical structure of the following formula (BiA-27).
δ (ppm) 7.0-7.8 (17H, Ph-H), 4.1 (4H, NH2), 2.3 (3H, CH3)

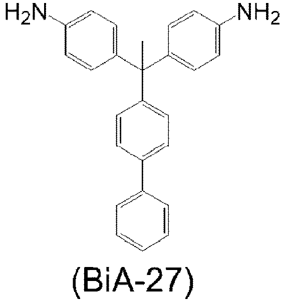

### (Example 28) Synthesis of BiA-28 to BiA-35

(Poly)amine compounds represented by the following formulas (BiA-28) to (BiA-35) were obtained in the same manner as Example 27 except that, instead of aniline and 4-acetylbiphenyl in Example 20, anilines and ketones listed in the following Table 3 were used.

**[Table 3]**

| Example | Anilines | Aldehydes | Molecular weight |
|---|---|---|---|
| 29 | Dimethylaminobenzene | 4-Acetylbiphenyl | 420 |
| 30 | o-Methylethylaniline | 4-Acetylbiphenyl | 448 |
| 31 | o-Methoxyaniline | 4-Acetylbiphenyl | 424 |
| 32 | o-Phenylaniline | 4-Acetylbiphenyl | 516 |
| 33 | 2,2'-Diaminobiphenyl | 4-Acetylbiphenyl | 546 |
| 34 | Naphthylamine | 4-Acetylbiphenyl | 464 |
| 35 | Aniline | 4-Acetophenone | 288 |

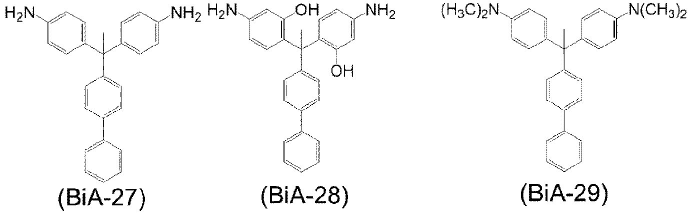

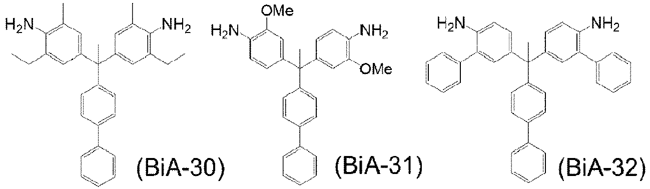

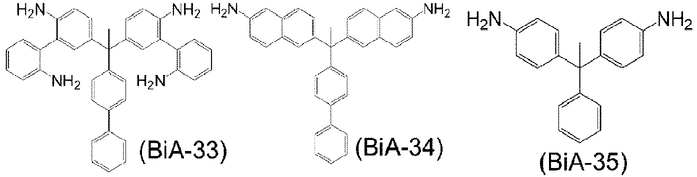

### (Example 1-1) Synthesis of CBiA-1

The crude compound obtained in Example 1 was dried as it was in a vacuum dryer without isolation to obtain 3.0 g of a reaction product (CBiA-1) mainly composed of the compound represented by the formula (BiA-1).

### (Examples 2-1 to 2-35) Synthesis of CBiA-2 to CBiA-35

In the same manner as Example 1-1, the crude compounds obtained in Example 2 to Example 35 were dried as they were in a vacuum dryer without isolation to obtain reaction products (CBiA-2 to CBiA-35) mainly composed of the compounds represented by the formulas (BiA-2 to BiA-35), respectively.

### (Example 36) Synthesis of RBiA-1

To a container (internal capacity: 100 mL) equipped with a stirrer, a condenser tube and a burette, 1.8 g of BiA-1 and 20.0 ml of a 1,4-dioxane solution of hydrogen chloride were added, and 1.8 g (10 mmol) of 4-biphenylaldehyde (a reagent manufactured by Sigma-Aldrich) was further added, and the contents were stirred at a reaction temperature of 100°C for a reaction time of 6 hours to allow reaction. To this reaction solution, 1000 mL of pure water was added, and the crude resin was then filtered off. The obtained crude resin was purified by column chromatography to obtain 0.2 g of a resin (RBiA-1) represented by the following formula (RBiA-1).

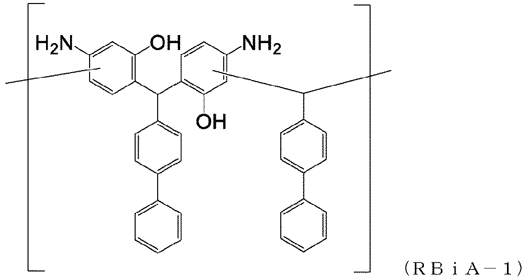

This application is based on Japanese Patent Application No. 2017-147097 filed on July 28, 2017, the contents of which are incorporated herein by reference.

### Industrial Applicability

The (poly)amine compound and resin according to the present invention has excellent structure-forming ability (for example, film-forming ability), heat resistance, transparency and refractive index. As such, it can be used advantageously as a raw material for functional resins such as polyurea, polyamide, polyimide, polyamideimide, benzoxazine and maleimide, and as a curing agent for functional cured products such as epoxy, urethane, urea and cyanate.

## Claims

1. A (poly)amine compound represented by the following formula (0): 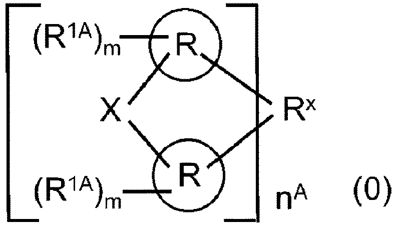 wherein
R^{X} is a 2n^{A}-valent group having 1 to 70 carbon atoms or a single bond;
each R^{1A} is independently any of an alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, a halogen atom, a nitro group, an amino group having 0 to 30 carbon atoms and optionally having a substituent, a carboxyl group, a thiol group and a hydroxy group, wherein when R^{1A} is any of the alkyl group, the aryl group, the crosslinking group and the alkoxy group, R^{1A} optionally contains at least one bond selected from the group consisting of an ether bond, a ketone bond and an ester bond, and at least one R^{1A} is an amino group having 0 to 30 carbon atoms and optionally having a substituent;
X is an oxygen atom or a sulfur atom, or X is optionally absent;
each R independently represents any of a benzene ring, a biphenyl ring, a naphthalene ring, an anthracene ring and a pyrene ring;
each m is independently an integer of 0 to 9, wherein at least one m is an integer of 1 to 9; and
n^{A} is an integer of 1 to 4.

2. The (poly)amine compound according to claim 1,
wherein the formula (0) is represented by the following formula (1): 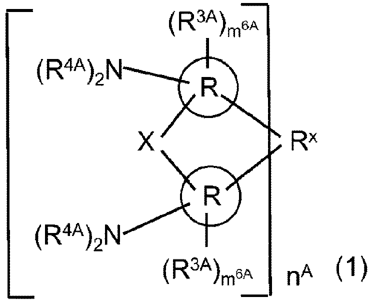 wherein
each R^{3A} is independently any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, an alkoxy group having 1 to 30 carbon atoms and optionally having a substituent, and a halogen atom;
each R^{4A} is independently any of a hydrogen atom, a linear or branched, or cyclic alkyl group or acyl group having 1 to 30 carbon atoms and optionally having a substituent, an aryl group having 6 to 30 carbon atoms and optionally having a substituent, and a crosslinking group having 2 to 30 carbon atoms and optionally having a substituent, wherein when R^{4A} is any of the alkyl group or acyl group, the aryl group and the crosslinking group, R^{4A} optionally contains at least one bond selected from the group consisting of an ether bond, a ketone bond and an ester bond;
each m^{6A} is independently an integer of 0 to 5; and
R^{X}, X, R and n^{A} are as defined above.

3. The (poly)amine compound according to claim 2,
wherein the formula (1) is represented by the following formula (2): 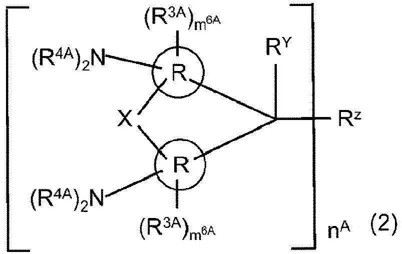 wherein
R^{Y} is any of a hydrogen atom, a linear or branched, or cyclic alkyl group having 1 to 30 carbon atoms and optionally having a substituent and an aryl group having 6 to 30 carbon atoms and optionally having a substituent;
R^{Z} is an n^{A}-valent group having 1 to 60 carbon atoms or a single bond, and the total number of carbon atoms in R^{Y} and R^{Z} is 69 or less; and
X, R, n^{A}, R^{3A}, R^{4A} and m^{6A} are as defined above.

4. The (poly)amine compound according to claim 3,
wherein the formula (2) is represented by the following formula (3): 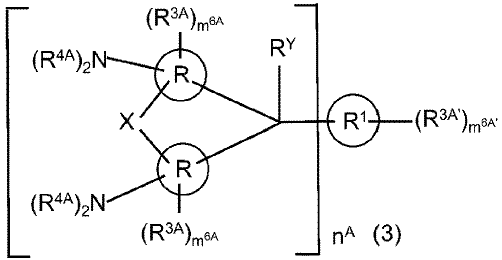
R^{3A'} is any of a linear or branched, or cyclic alkyl group or alkoxy group having 1 to 30 carbon atoms and optionally having a substituent, an amino group optionally having a substituent, an aryl group having 6 to 30 carbon atoms and optionally having a substituent, a crosslinking group having 2 to 30 carbon atoms and optionally having a substituent, a hydroxy group and a cyano group;
each R¹ independently represents any of a benzene ring, a biphenyl ring, a naphthalene ring, an anthracene ring, and a heterocyclic ring containing any of an oxygen atom, a sulfur atom and a nitrogen atom as a heteroatom;
m^{6A'} is an integer of 0 to 5;
X, R, n^{A}, R^{3A}, R^{4A}, m^{6A} and R^{Y} are as defined above; and
when R¹ is a benzene ring, R^{3A'} is not a phenyl group.

5. The (poly)amine compound according to claim 4,
wherein
R^{3A'} is any of a linear or branched, or cyclic alkyl group or alkoxy group having 1 to 30 carbon atoms and
optionally having a substituent, an aryl group having 6 to 30 carbon atoms and optionally having a substituent, a crosslinking group having 2 to 30 carbon atoms and
optionally having a substituent, a hydroxy group and a cyano group; and
when R¹ is a benzene ring or a naphthalene ring, R^{3A'} is any of a linear or branched, or cyclic alkyl group or alkoxy group having 1 to 30 carbon atoms and optionally having a substituent, an aryl group having 6 to 30 carbon atoms and optionally having a substituent, a crosslinking group having 2 to 30 carbon atoms and optionally having a substituent, a hydroxy group and a cyano group.

6. The (poly)amine compound according to claim 4 or 5, wherein the (poly)amine compound represented by the formula (3) is a compound represented by the following formula (0a): 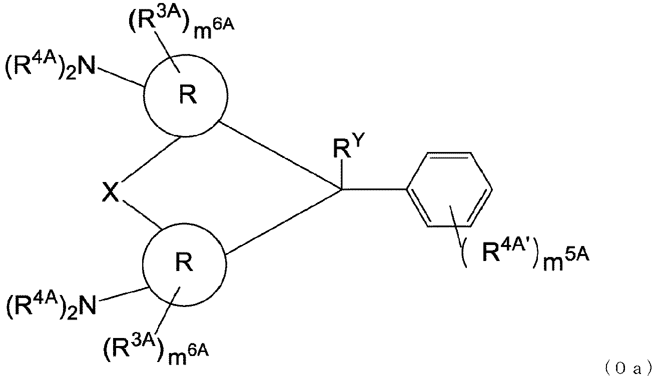
wherein X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom); and m^{5A} represents an integer of 1 to 5, or
a compound represented by the following formula (0b) : 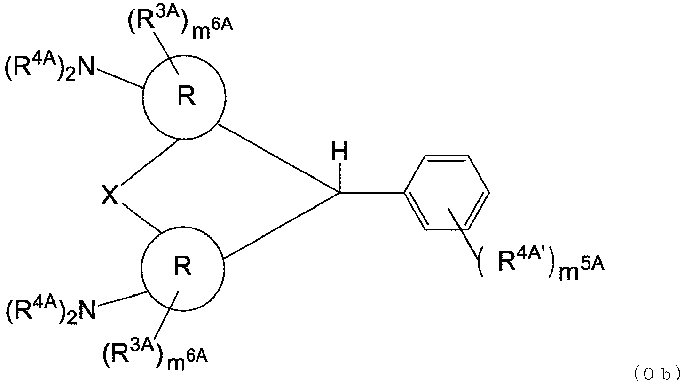
wherein X, R, R^{3A}, R^{4A}, m^{6A} and R^{Y} are as defined above, and R^{4A'} represents a hydrogen atom, or any of a linear or branched, or cyclic alkyl group having 1 to 30 carbon atoms and optionally having a substituent, an aryl group having 6 to 30 carbon atoms and optionally having a substituent, a crosslinking group having 2 to 30 carbon atoms and optionally having a substituent, and a halogen atom (such as a fluorine atom, a chlorine atom and a bromine atom); and m^{5A} represents an integer of 1 to 5.

7. The (poly)amine compound according to any one of claims 4 to 6, wherein the (poly)amine compound represented by the formula (3) is a compound represented by the following formula (0a-1) : 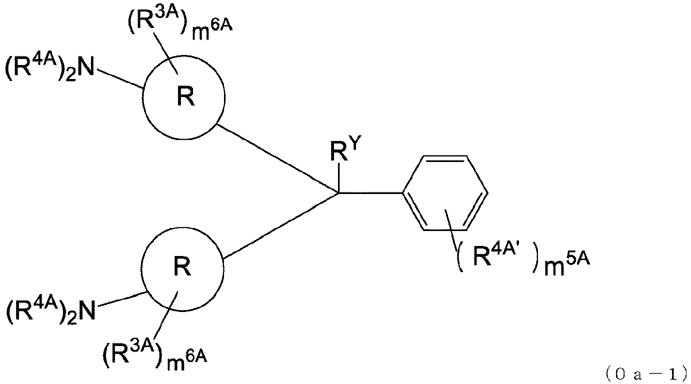
wherein R, R^{3A}, R^{4A}, m^{6A}, R^{4A'}, m^{5A} and R^{Y} are as defined above, or
a compound represented by the following formula (0b-1) : 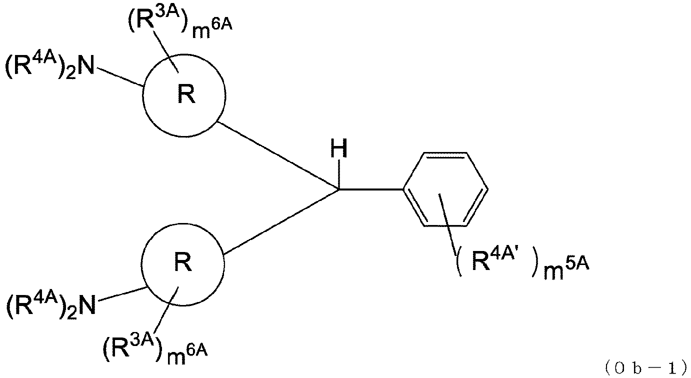
wherein R, R^{3A}, R^{4A}, m^{6A}, m^{5A} and R^{Y} are as defined above.

8. The (poly)amine compound according to claim 7,
wherein the (poly)amine compound represented by the formula (0b-1) is a compound represented by the following formula (0b-2) : 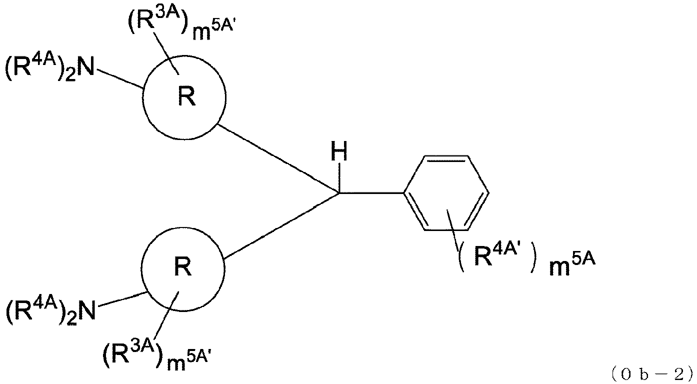 wherein R, R^{3A}, R^{4A}, R^{4A'}, m^{5A} and R^{Y} are as defined above, and each m^{5A'} is independently an integer of 1 to 5.

9. The (poly)amine compound according to claim 8,
wherein the (poly)amine compound represented by the formula (0a-1) is a compound represented by the following formula (0a-2): 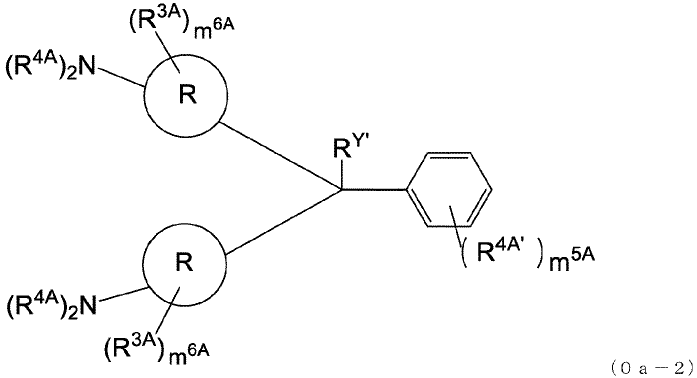 wherein R, R^{3A}, R^{4A}, m^{6A}, m^{5A} and R^{Y} are as defined above, and R^{Y'} is any of a linear or branched, or cyclic alkyl group having 1 to 30 carbon atoms and optionally having a substituent and an aryl group having 6 to 30 carbon atoms and optionally having a substituent.

10. The (poly)amine compound according to claim 8,
wherein the (poly)amine compound represented by the formula (0b-1) is a compound represented by the following formula (0b-3): 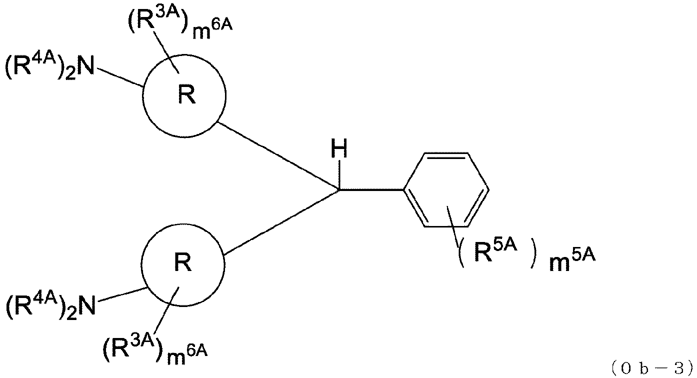 wherein R, R^{3A}, R^{4A}, R^{4A'} and m^{5A} are as defined above, and R^{5A} represents a linear or branched, or cyclic alkyl group having 5 or more carbon atoms and optionally having a substituent.

11. The (poly)amine compound according to any one of claims 4 to 6, wherein the (poly)amine compound represented by the formula (3) is a compound represented by the following formula (3a): 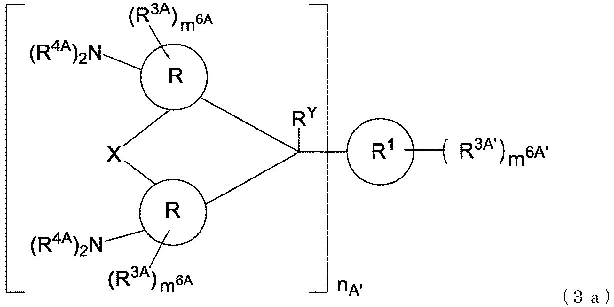
wherein R, X, R^{3A}, R^{4A}, m^{6A}, R^{Y}, R¹ and m^{6A'} are as defined above, and n^{A'} is an integer of 2 to 4, or
a compound represented by the following formula (3b): 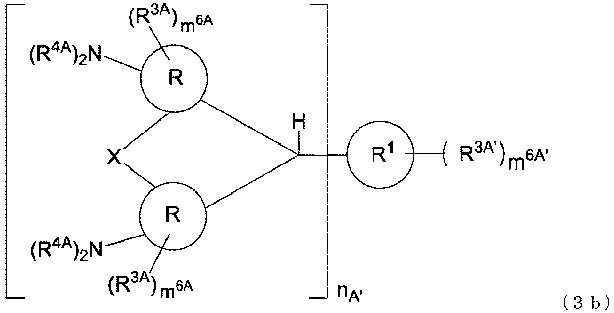
wherein R, X, R^{3A}, R^{4A}, m^{6A}, R1 and m^{6A'} are as defined above, and n^{A'} is an integer of 2 to 4.

12. The (poly)amine compound according to any one of claims 1 to 11, wherein X is an oxygen atom in the formulas (0), (1), (2) and (3).

13. The (poly)amine compound according to any one of claims 1 to 12, wherein the (poly)amine compound is any of compounds represented by the following formulas (BiA-1) to (BiA-35):

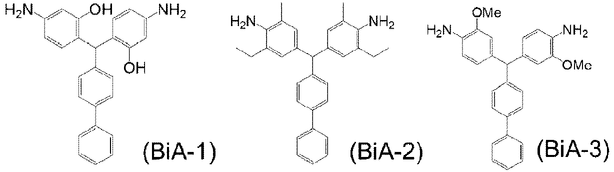

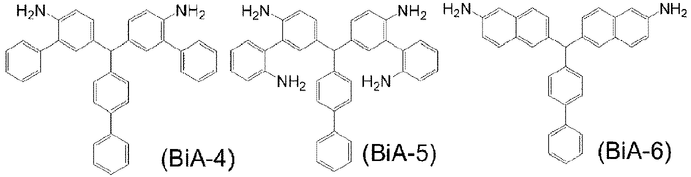

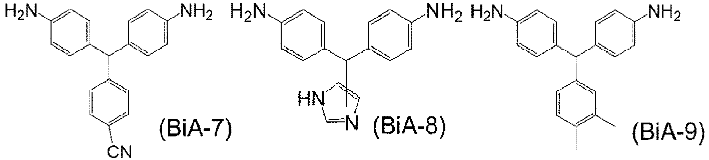

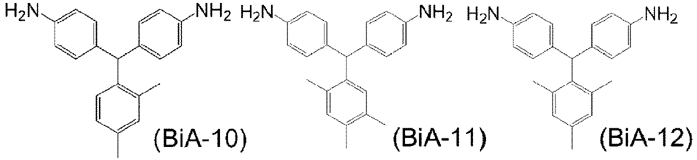

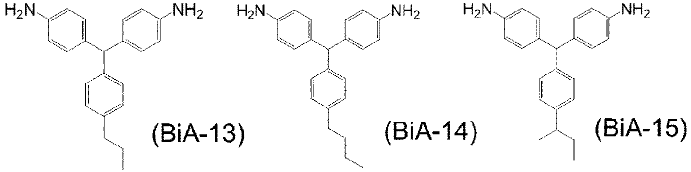

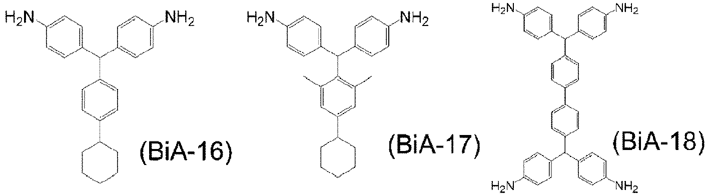

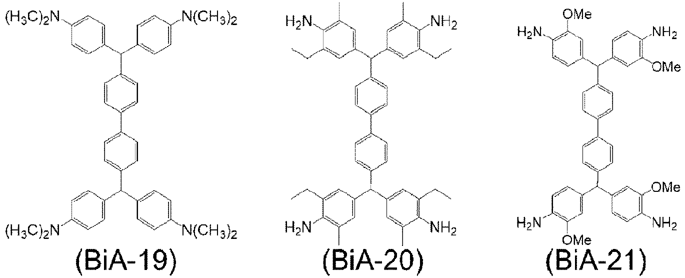

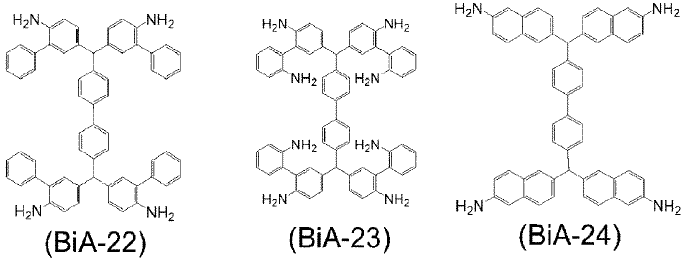

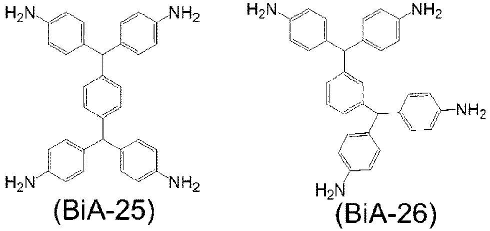

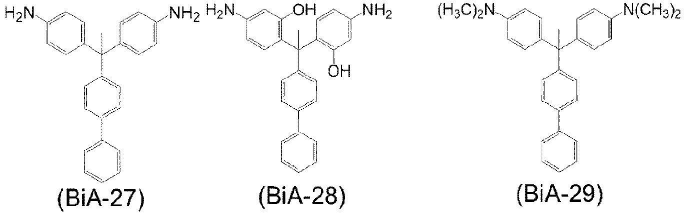

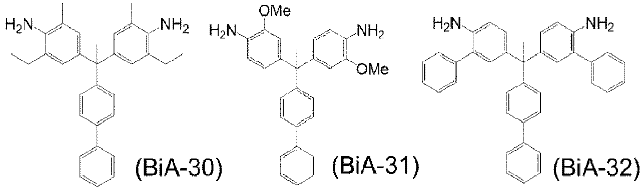

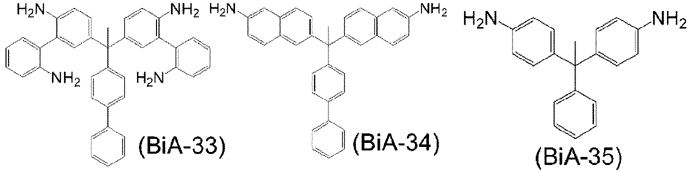

14. A resin comprising a monomer unit containing a unit derived from the (poly)amine compound according to any one of claims 1 to 13.

15. The resin according to claim 14, wherein the monomer unit containing a unit derived from the (poly)amine compound is a unit represented by the following formula (4) : 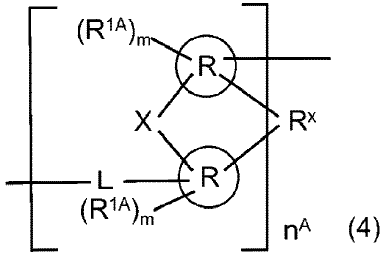 wherein
L is a linear or branched linking group having 1 to 30 carbon atoms, or a single bond; and
R^{X}, R^{1A}, X, R, m and n^{A} are as defined above.

16. A cured product obtained by curing a curable compound with a curing agent,
wherein the curing agent comprises the (poly)amine compound according to any one of claims 1 to 13.
